(19) Europäisches Patentamt
European Patent Office
Office européen des brevets

(11) Numéro de publication : **0 519 831 A1**

(12) **DEMANDE DE BREVET EUROPEEN**

(21) Numéro de dépôt : **92401715.5**

(22) Date de dépôt : **19.06.92**

(51) Int. Cl.[5] : **C07D 235/02,** C07D 233/70, C07D 403/12, A61K 31/415, C07D 403/10, C07D 413/12

(30) Priorité : **21.06.91 FR 9107685**

(43) Date de publication de la demande : **23.12.92 Bulletin 92/52**

(84) Etats contractants désignés : **AT BE CH DE DK ES FR GB GR IT LI LU NL PT SE**

(71) Demandeur : **ELF SANOFI**
**32-34, rue Marbeuf**
**F-75008 Paris (FR)**

(72) Inventeur : **Arnaud, Joelle**
**24, Rue de la Croix**
**F-34080 Montpellier (FR)**
Inventeur : **Assens, Jean-Louis**
**13, Rue des Serpolets**
**F-34790 Grabels (FR)**
Inventeur : **Bernhart, Claude**
**21, Rue de la Frigoule**
**F-34980 Saint Gely du Fesc (FR)**
Inventeur : **Ferrari, Bernard**
**Chemin des Perayrols**
**F-34270 Les Matelles (FR)**
Inventeur : **Haudricourt, Frédérique**
**679, Rue de Las Sorbés**
**F-34090 Montpellier (FR)**
Inventeur : **Perreaut, Pierre**
**146, Rue des Vignes Blanches**
**F-34980 Saint Gely du Fesc (FR)**

(74) Mandataire : **Gillard, Marie-Louise et al**
**Cabinet Beau de Loménie 55, Rue d'Amsterdam**
**F-75008 Paris (FR)**

(54) **Dérivés hétérocycliques N-substitués, leur préparation, les compositions pharmaceutiques en contenant.**

(57) L'invention a pour objet des composés de formule

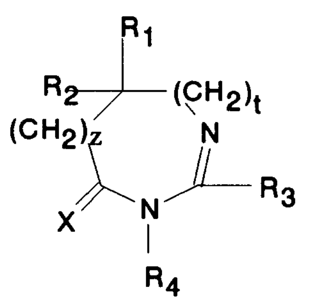

(I)

dans laquelle :

— $R_1$ et $R_2$ représentent chacun indépendamment un alkyle en $C_1$-$C_6$, un cycloalkyle en $C_3$-$C_7$ un phényle, un phénylalkyle, lesdits groupes alkyle, phényle et phénylalkyle étant éventuellement substitués par un ou plusieurs atomes d'halogène ou par un groupe choisi parmi un perfluoroalkyle en $C_1$-$C_4$, un hydroxyle, un alcoxy en $C_1$-$C_4$ ;

— ou $R_1$ et $R_2$ ensemble forment un groupe de formule $=CR'_1R'_2$, dans laquelle $R'_1$ représente l'hydrogène, un alkyle en $C_1$-$C_4$ ou un phényle, et $R'_2$ représente un alkyle en $C_1$-$C_4$ ou un phényle ;

— ou encore $R_1$ et $R_2$ liés ensemble représentent, soit un groupe de formule $-(CH_2)_n-$, soit un groupe de formule $-(CH_2)_pY(CH_2)_q-$, dans lequel Y est, soit un atome d'oxygène, soit un atome de soufre, soit un groupe CH substitué par un groupe alkyle en $C_1$-$C_4$ un phényle ou un phénylalkyle, soit un groupe N-$R_5$ dans lequel $R_5$ représente un hydrogène, un alkyle, un phénylalkyle, un alkylcarbonyle en $C_1$-$C_4$, un halogénoalkylcarbonyle en $C_1$-$C_4$, un polyhalogénoalkylcarbonyle en $C_1$-$C_4$, un benzoyle, un alpha aminoacyle ou un groupe N-protecteur, ou $R_1$ et $R_2$ liés ensemble avec l'atome de carbone auquel ils sont liés constituent un indane ou un adamantane ;

— $R_3$ représente un hydrogène, un alkyle en $C_1$-$C_6$, éventuellement substitué par un ou plusieurs atomes d'halogène ; un alcényle en $C_2$-$C_6$ ; un cycloalkyle en $C_3$-$C_7$ ; un phényle ; un phénylalkyle dans

EP 0 519 831 A1

lequel l'alkyle est en $C_1$-$C_3$ ; un phénylalcényle dans lequel l'alcényle est en $C_2$-$C_3$, lesdits groupes phényles étant non substitués ou substitués une ou plusieurs fois par un atome d'halogène, un alkyle en $C_1$-$C_4$, un halogénoalkyle en $C_1$-$C_4$, un polyhalogénoalkyle en $C_1$-$C_4$, un hydroxyle ou un alcoxy en $C_1$-$C_4$ ;

— $R_4$ représente un groupement aromatique ;

— $p + q = m$ ;

— n est un nombre entier compris entre 2 et 11 ;

— m est un nombre entier compris entre 2 et 5 ;

— X représente un atome d'oxygène ou atome de soufre ;

— z et t sont nuls ou l'un est nul et l'autre représente 1 ; et leurs sels.

Application : Médicaments utiles pour le traitement d'affections cardiovasculaires, d'affections du système nerveux central, du glaucome et de la rétinopathie diabétique.

La présente invention concerne des dérivés hétérocycliques N-substitués, leur préparation et les compositions pharmaceutiques en contenant.

Les composés selon l'invention antagonisent l'action de l'angiotensine II qui est une hormone de formule :

H-Asp-Arg-Val-Tyr-Ile-His-Pro-Phe-OH

L'angiotensine II est un puissant agent vasopresseur qui est le produit biologiquement actif du système rénine-angiotensine : la rénine agit sur l'angiotensinogène du plasma pour produire l'angiotensine I, celle-ci est convertie en angiotensine II par action de l'enzyme de conversion de l'angiotensine I.

Les composés de la présente invention sont des composés non peptidiques, antagonistes de l'angiotensine II. En inhibant l'action de l'angiotensine II sur ses récepteurs, les composés selon l'invention empêchent notamment l'augmentation de la pression sanguine produite par l'interaction hormone-récepteur; ils ont également d'autres actions physiologiques au niveau du système nerveux central.

Ainsi les composés selon l'invention sont utiles dans le traitement d'affections cardiovasculaires comme l'hypertension, la défaillance cardiaque ainsi que dans le traitement d'affections du système nerveux central et dans le traitement du glaucome et de la rétinopathie diabétique.

La présente invention a pour objet des composés de formule :

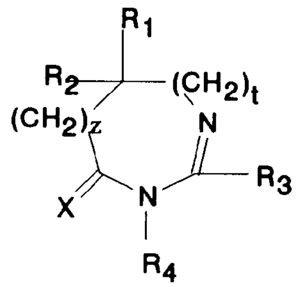

(I)

dans laquelle

- $R_1$ et $R_2$ représentent chacun indépendamment un alkyle en $C_1$-$C_6$, un cycloalkyle en $C_3$-$C_7$ un phényle, un phénylalkyle dans lequel l'alkyle est en $C_1$-$C_3$, lesdits groupes alkyle, phényle et phénylalkyle étant non substitués ou substitués par un ou plusieurs atomes d'halogène ou par un groupe choisi parmi un perfluoroalkyle en $C_1$-$C_4$, un hydroxyle, un alcoxy en $C_1$-$C_4$ ;
- ou $R_1$ et $R_2$ ensemble forment un groupe de formule $=CR'_1R'_2$, dans laquelle $R'_1$ représente l'hydrogène, un alkyle en $C_1$-$C_4$ ou un phényle, et $R'_2$ représente un alkyle en $C_1$-$C_4$ ou un phényle;
- ou encore $R_1$ et $R_2$ liés ensemble représentent, soit un groupe de formule $-(CH_2)_n-$, soit un groupe de formule $-(CH_2)_pY(CH_2)_q-$, dans lequel Y est, soit un atome d'oxygène, soit un atome de soufre, soit un groupe CH substitué par un groupe alkyle en $C_1$-$C_4$ un phényle ou un phénylalkyle dans lequel l'alkyle est en $C_1$-$C_3$ soit un groupe N-$R_5$ dans lequel $R_5$ représente un hydrogène, un alkyle en $C_1$-$C_4$, un phénylalkyle dans lequel l'alkyle est en $C_{1-3}$, un alkylcarbonyle en $C_1$-$C_4$, un halogénoalkylcarbonyle en $C_1$-$C_4$, un polyhalogénalkylcarbonyle en $C_1$-$C_4$, un benzoyle, un alpha aminoacyle ou un groupe N-protecteur, ou $R_1$ et $R_2$ liés ensemble avec l'atome de carbone auquel ils sont liés constituent un indane ou un adamantane ;
- $R_3$ représente un hydrogène, un alkyle en $C_1$-$C_6$, non substitué ou substitué par un ou plusieurs atomes d'halogène ; un alcényle en $C_2$-$C_6$ ; un cycloalkyle en $C_3$-$C_7$ ; un phényle ; un phénylalkyle dans lequel l'alkyle est en $C_1$-$C_3$ ; un phénylalcényle dans lequel l'alcényle est en $C_2$-$C_3$, lesdits groupes phényles étant non substitués ou substitués une ou plusieurs fois par un atome d'halogène, un alkyle en $C_1$-$C_4$, un halogénoalkyle en $C_1$-$C_4$, un polyhalogénoalkyle en $C_1$-$C_4$, un hydroxyle ou un alcoxy en $C_1$-$C_4$ ;
- $R_4$ représente un groupement choisi parmi :

a) 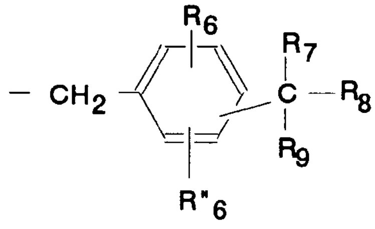 ,

b) 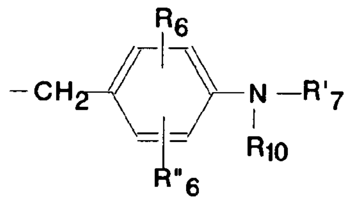

c) 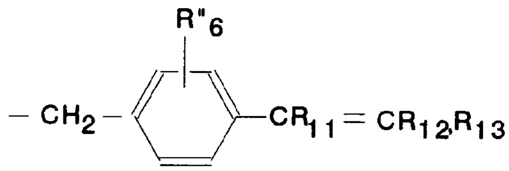 ,

d) 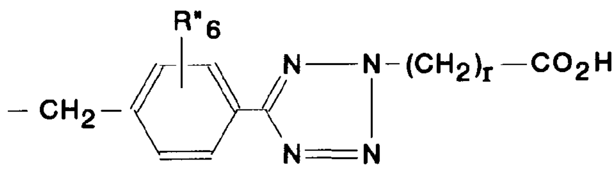 ,

e) 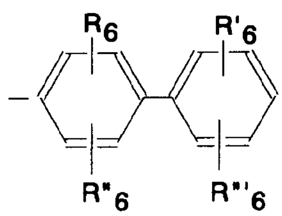 ,

f) 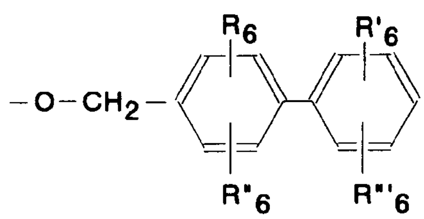 ,

g) 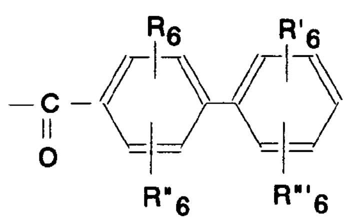 ,

h) 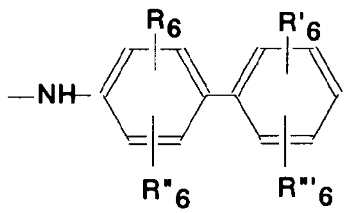 ,

j) 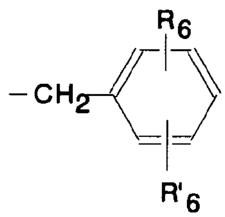

k) 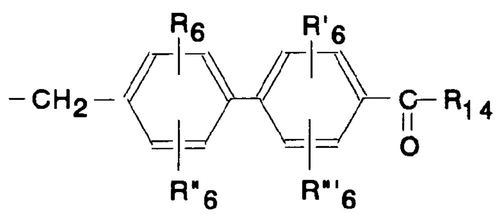

l) 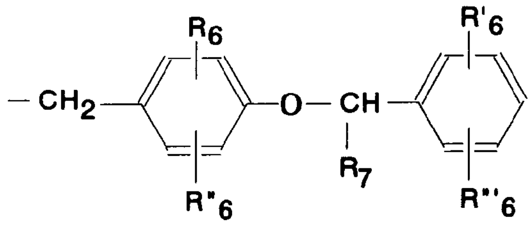 ,

- $R''_6$ et $R'''_6$ sont semblables ou différents et représentent chacun indépendamment l'hydrogène, un atome de chlore ou un groupe choisi parmi un alkyle en $C_1$-$C_6$, un alcoxy en $C_1$-$C_4$, un amino, un aminométhyle ;
- $R_6$ et $R'_6$ sont semblables ou différents et représentent chacun indépendamment un atome d'hydrogène, un carboxy, un alcoxycarbonyle dans lequel l'alcoxy est en $C_1$-$C_4$, un cyano, un tétrazolyle -5, un méthyltétrazolyle-5 ou un 4H-oxo-5 oxadiazol-1,2,4 yle-3, ou un 4H-dioxo-3,5 oxadiazole-1, 2, 4 yle-2 à la condition que pour les groupes e, f, g, h, j, au moins un des substituants $R_6$ ou $R'_6$ soit différent de l'hydrogène ;
- $R_7$ représente un alkyle en $C_1$-$C_4$ ou un groupe choisi parmi :

i) $-(CH_2)_r-CO_2H$ ,

ii) 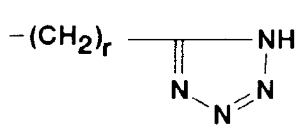 ,

iii) 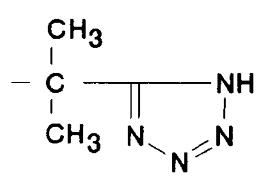 ,

iv) 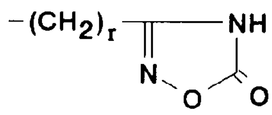 ,

v) 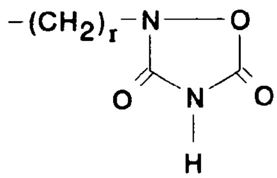

vi) 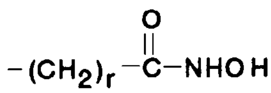

vii) - $(CH_2)_r$- CN

viii) -$OCOCH_3$

- R'$_7$ représente un alkyle en $C_1$-$C_4$ ou un groupe choisi parmi :

-$(CH_2)_s$- $CO_2H$ ,

-$(CH_2)_r$- CN ,

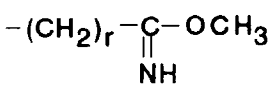

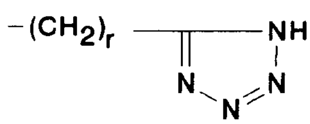 ,

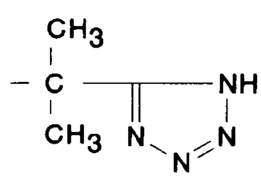 ,

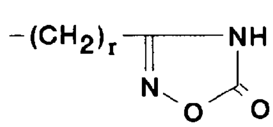

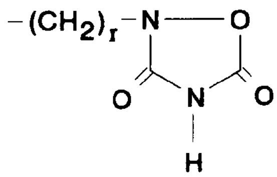

- $R_8$ est identique à $R_7$, ou bien $R_8$ représente l'hydrogène, un atome d'halogène, un alkyle en $C_1$-$C_4$, un cycloalkyle en $C_3$-$C_7$, un phényle substitué par $R'_6$, un benzyle dans lequel le phényle est substitué par $R'_6$, un hydroxyle, un alcoxy en $C_1$-$C_4$, un cyano, un groupe $OCOR_{15}$ dans lequel $R_{15}$ représente un alkyle en $C_1$-$C_4$, un halogénoalkyle en $C_1$-$C_4$ ou un phényle substitué par $R'_6$ ;
- $R_9$ est l'hydrogène ou $R_9$ est identique à $R_8$, avec la limitation que $R_9$ ne peut pas être identique à $R_7$ ;
- ou $R_8$ et $R_9$ ensemble avec l'atome de carbone auquel ils sont liés constituent un cycloalkyle en $C_3$-$C_7$ ou un groupe carbonyle ;
- $R_{10}$ représente l'hydrogène, un alkyle en $C_1$-$C_4$, un cycloalkyle en $C_3$-$C_7$, un phényle substitué par $R'_6$ ou un benzyle dont le phényle est substitué par $R'_6$ ;
- $R_{11}$ représente l'hydrogène, un alkyle en $C_1$-$C_6$, un phényle, un carboxy, un alcoxycarbonyle dans lequel l'alcoxy est en $C_1$-$C_4$, un tétrazolyle-5 ou un 4H-oxo-5 oxadiazol-1, 2, 4 yle-3 ou un 4H-dioxo-3,5 oxadiazole-1, 2, 4 yle-2;
- $R_{12}$ représente l'hydrogène, un alkyle en $C_1$-$C_6$, un cyano, un carboxy, un alcoxycarbonyle dans lequel l'alcoxy est en $C_1$-$C_4$, un tétrazolyle-5, un phényle substitué par $R'_6$ ou un benzyle dont le phényle est substitué par $R'_6$ ;
- $R_{13}$ représente l'hydrogène, un alkyle en $C_1$-$C_6$, un alcoxycarbonyle dans lequel l'alcoxy est en $C_1$-$C_4$, un phényle substitué par $R'_6$, un benzyle dont le phényle est substitué par $R'_6$, un cyano, un carboxy, un tétrazolyle-5, un 4H-oxo-5 oxadiazol-1, 2, 4 yle-3, un 4H-dioxo-3,5-oxadiazole-1,2,4-yle-2 à la condition que $R_{11}$ et $R_{13}$ ne représentent pas simultanément l'un des groupes suivants : un carboxy, un tétrazolyle-5, un 4H-oxo-5 oxadiazol-1, 2, 4 yle-3 ou un 4H-dioxo-3,5 oxadiazole-1, 2, 4 yle-2 ;
- $R_{14}$ représente un carboxyalkyle en $C_1$-$C_4$, un phényle, un carboxyphényle ;
- $p + q = m$ ;
- n est un nombre entier compris entre 2 et 11 ;
- m est un nombre entier compris entre 2 et 5 ;
- r représente 0, 1 ou 2;
- s représente 1 ou 2;
- X représente un atome d'oxygène ou atome de soufre ;
- z et t sont nuls ou l'un est nul et l'autre représente 1 ;

avec la limitation que pour les valeurs a), b), k) et l) de $R_4$, $R_6$ représente l'hydrogène lorsque $R_7$, $R'_7$ ou $R_{14}$ contient soit un groupement carboxy, soit un groupement tétrazolyle-5, soit un groupement 4H-oxo-5 oxadiazol-1,2,4 yle-3 soit un groupement 4H-dioxo-3,5 oxadiazole-1, 2, 4 yle-2 ;

et leurs sels.

Lorsqu'un composé selon l'invention présente un carbone asymétrique, l'invention comprend les 2 isomères optiques de ce composé. La séparation des isomères optiques peut être effectuée selon les méthodes décrites dans "Synthesis of Optically Active alpha-aminoacids", R.M. Williams, Pergamon Press, 1989.

Les sels des composés de formule (I) selon la présente invention comprennent ceux avec des acides minéraux ou organiques qui permettent une séparation ou une cristallisation convenable des composés de formule (I), tels que l'acide trifluoroacétique, l'acide picrique, l'acide oxalique ou un acide optiquement actif, par exemple un acide mandélique ou un acide camphosulfonique, et ceux qui forment des sels pharmaceutiquement acceptables tels que le chlorhydrate, le bromhydrate, le sulfate, l'hydrogénosulfate, le dihydrogénophosphate, le méthanesulfonate, le maléate, le fumarate, le 2-naphtalènesulfonate.

Les sels des composés de formule (I) comprennent également les sels avec des bases organiques ou minérales, par exemple les sels minéraux alcalins ou alcalino-terreux comme les sels de sodium, de potassium, de calcium, les sels de sodium et de potassium étant préférés, ou avec une amine tertiaire, telle que le trométamol, ou bien les sels d'arginine, de lysine, ou de toute amine pharmaceutiquement acceptable.

Selon la présente description et dans les revendications qui vont suivre, par atome d'halogène on entend un atome de brome, de chlore ou de fluor ; par alkyle on entend un radical d'hydrocarbure linéaire ou ramifié ; par groupe N-protecteur (également désigné par Pr) on entend un groupe utilisé classiquement dans la chimie des peptides pour permettre une protection temporaire de la fonction amine, par exemple un groupe Boc, Z,

Fmoc ou un groupe benzyle ; par groupe carboxy estérifié on entend un ester labile dans les conditions appropriées, comme par exemple un ester méthylique, éthylique, benzylique ou tertiobutylique ; par groupe α-aminoacide on entend le résidu d'un α-aminoacide naturel ne portant pas d'autres groupes fonctionnels que les fonctions amino et carboxy.

Les composés de formule (I) dans lesquels $R_1$ et $R_2$ liés ensemble représentent un groupe $-(CH_2)_n-$ dans lequel n est 4 ou 5, sont des composés préférés; les composés de formule (I) dans lesquels $R_1$ et $R_2$ représentent, l'un un méthyle, l'autre un cyclohexyle, sont également préférés.

De même, les composés de formule (I) dans lesquels $R_3$ représente un groupe alkyle linéaire en $C_1$-$C_6$ sont des composés préférés.

Les composés de formule (I) dans lesquels X représente un atome d'oxygène sont également des composés préférés.

Les composés de formule (I) dans lesquels $R_4$ représente le groupe a) sont préférés : tout particulièrement préférés sont les composés (I) dans lesquels $R_4$ représente un groupe de formule :

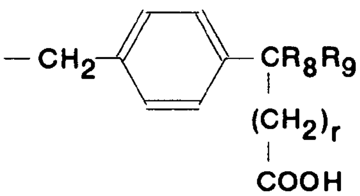

dans laquelle r, $R_8$ et $R_9$ ont les significations indiquées ci-dessus pour (I).

Enfin les composés de formule (I) dans lesquels z = t = 0, sont des composés préférés.

Les abréviations suivantes sont utilisés dans la description et dans les exemples :

| | |
|---|---|
| Et | : Ethyl |
| nBu, tBu | : n-butyl, *tert*-butyl |
| DMF | : diméthylformamide |
| THF | : tétrahydrofuranne |
| DCM | : dichlorométhane |
| NBS | : N-bromosuccinimide |
| DCC | : dicyclohexylcarbodiimide |
| DIPEA | : diisopropyléthylamine |
| Ether | : éther éthylique |
| TFA | : acide trifluoroacétique |
| Z | : benzyloxycarbonyle |
| Boc | : *ter*-butoxycarbonyle |
| BOP | : hexafluorophosphate de benzotriazolyloxy trisdiméthylamino phosphonium |
| Fmoc | : fluorenylméthyloxycarbonyle |
| AcOH | : acide acétique |
| AcOEt | : acétate d'éthyle |
| MeOH | : méthanol |
| DMAP | : dimethylamino-4 pyridine |
| LDA | : lithium diisopropylamide |
| tetr | : tétrazolyle-5 |

La présente invention a également pour objet le procédé de préparation des composés (I). Les composés (I) selon l'invention dans lesquels $R_4$ a l'une des valeurs a), b), c), d), j), k) ou l) sont préparés selon le procédé décrit ci-après et appelé procédé 1. Ledit procédé est caractérisé en ce que :

a1) on fait réagir un dérivé hétérocyclique de formule :

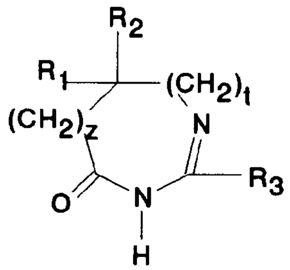

(2)

dans laquelle z, t, $R_1$ , $R_2$ , et $R_3$ ont les significations indiquées ci-dessus pour (I), sur un halogènure de formule :

Hal-R'$_4$ (3)

dans laquelle Hal représente un atome d'halogène, de préférence le brome, et R'$_4$ représente soit $R_4$, soit un groupement précurseur de $R_4$ ;

b1) éventuellement, le composé ainsi obtenu, de formule :

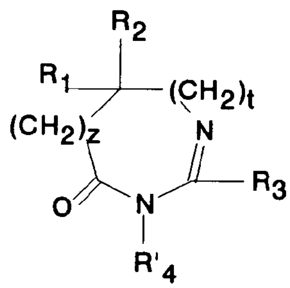

(4)

est traité par le réactif de Lawesson [bis(méthoxy-4 phényl)-2,4 dithia-1,3 diphosphétane-2,4 disulfure 2,4] ;

c1) le composé obtenu en a1) ou en b1), de formule :

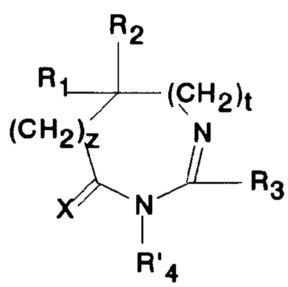

(5)

dans laquelle X représente un atome d'oxygène ou un atome de soufre, est éventuellement traité pour préparer le composé (I) par transformation du groupe R'$_4$ en $R_4$.

L'étape a1) du procédé est réalisée dans un solvant inerte tel que le DMF, le DMSO ou le THF, en milieu basique, par exemple en présence de potasse, de soude, d'un alcoolate métallique ou d'un hydrure métallique.

L'étape b1) est réalisée pas chauffage sous azote dans un solvant tel que le toluène, selon la méthode décrite par M.P. Cava et al. (Tetrahedron, 1985, 41, 22, 5061).

Les composés (2) sont préparés par des méthodes connues. Par exemple, on peut utiliser la méthode décrite par Jacquier et al. (Bull. Soc. Chim. France, 1971, (3), 1040-1051) et par Brunken et Bach (Chem. Ber., 1956, 89, 1363-1373) et faire agir un imidate d'alkyle sur un acide aminé ou son ester, selon le schéma réactionnel suivant :

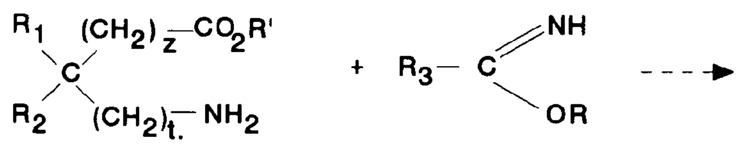

(5')  (6)

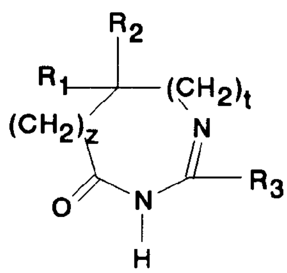

(2)

dans lequel R représente un alkyle en $C_1$-$C_4$ , R′ représente l'hydrogène ou un alkyle en $C_1$-$C_4$ et $R_1$, $R_2$, $R_3$, z et t sont tels que définis précédemment pour (I).

Cette réaction est effectuée en milieu acide, par chauffage dans un solvant inerte tel que le xylène ou le toluène.

Selon un autre mode opératoire, un composé (2) peut être préparé par action en milieu acide d'un aminoalkylamide (5″) sur un ortho-ester d'alkyle (10) selon le schéma réactionnel suivant :

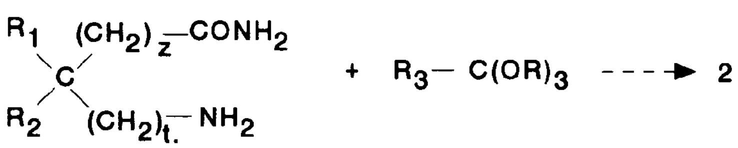

(5")  (10)

dans lequel R représente un alkyle en $C_1$-$C_4$.

En utilisant un mode opératoire décrit par H. Takenaka et al. (Heterocycles, 1989, 29, (6), 1185-89), on peut également préparer le composé (2), en faisant agir dans un premier temps sur le dérivé (5″) un halogénure d'acide de formule :

$R_3$ - CO - Hal (12)

dans laquelle Hal représente un halogène, de préférence le chlore ; la cyclisation est réalisée en milieu basique.

Les composés halogénés $R'_4$ Hal (3) sont connus ou préparés par des méthodes connues, par exemple les méthodes décrites dans les demandes de brevet EP 324377 et WO 91-12001.

Lorsque le composé (I) à préparer possède un groupe carboxy au sein du substituant $R_4$, $R'_4$ contient un groupe carboxy estérifié. Lorsque le composé (I) à préparer possède un groupe tétrazolyle au sein du substituant $R_4$, $R'_4$ contient, soit un tétrazolyle protégé, par exemple par un groupe trityle, soit un groupe cyano qui sera ensuite remplacé par un groupe tétrazolyle protégé par un trityle.La transformation de $R'_4$ en $R_4$ est alors effectuée à l'étape c1) du procédé.

Dans le cas particulier de la préparation d'un groupement $R_4$ comprenant un tétrazolyle, l'étape a1) peut être effectuée, soit avec un composé $R_4$Hal comprenant un groupe tétrazolyle protégé par un trityle, soit avec un composé $R'_4$Hal comprenant un cyano, précurseur du tétrazole.

Lorsque le composé (I) à préparer possède un groupe 4H-oxo-5 oxadiazol-1,2,4-yle-3, celui-ci est préparé par une des méthodes de la littérature citée ci-dessous :

. M.A. Perez et al., Synthesis, 1983, 483.

. A.R. Katritzky et al., Tetrahedron, 1965, 21, 1681-1692.

. K.R. Rao et al., Heterocycles, 1988, 27, (3), 683-685.

. G. Babu et al., J. Org. Chem., 1976, 41 (20), 3223-3237.

Ainsi, le substituant est obtenu à partir du groupe $C(OC_2H_5) = NCO_2CH_3$ qui lui-même est obtenu à partir d'un groupe carboxy transformé en groupe carboxamido [R.J. Bergeron et al., J. Org. Chem., 1985, 50 (15), 2781].

Lorsque le composé (I) à préparer possède un groupement 4H-dioxo-3,5 oxadiazol-1,2,4 yle-2, celui-ci est préparé à partir d'un groupement nitro selon la méthode décrite par J.L. Kraus et al. dans J. Het. Chem., 1982, 19, 971 ou selon la méthode décrite par N.B. Vsevolozhakaya et al. dans Zh. Org. Khim., 1971, 7 (5), 923-929.

La préparation d'un composé $R'_4Hal$ utile pour la synthèse d'un composé (I) dans lequel $R_4$ est le groupement a) peut être effectuée par l'une des méthodes connues, choisie par l'homme de l'art et adaptée selon la nature des substituants $R_6$, $R_6''$, $R_7$, $R_8$, $R_8$ entrant dans la composition du groupement a).

Ainsi, le mode opératoire décrit pas J. Gyr dans Chem. Ber., 1908, 41, 4321, permet de préparer l'acide p-tolyl-2 phényl acétique ($R_4H$) ; on peut aussi utiliser la méthode décrite par M.E. Grundy et al. dans J. Chem. Soc., 1960, 372-376. On prépare un ester de cet acide par un procédé connu, puis le dérivé halogéné correspondant $HalR'_4$ (3), par exemple, par action du N-bromosuccinimide selon le procédé décrit dans la demande de brevet européen EP 324377.

On peut de la même façon préparer des composés $R_4H$ et $R'_4$ Hal dans lesquels l'un et/ou l'autre des noyaux phényles porte un substituant $R_6$ et/ou $R'_6$ et/ou $R''_6$.

L'ester d'un acide p-tolyl-2 alkyl-2 phénylacétique est obtenu par alkylation de l'ester de l'acide p-tolyl-2 phénylacétique, par exemple par l'action d'un iodure d'alkyle et d'hydrure de sodium.

L'acide diphényl-2,2 p-tolyl-2 acétique ($R_4H$) est préparé à partir de l'acide benzilique et du toluène selon la méthode décrite par A. Bistrzycki dans Chem. Ber., 1901, 34, 3080. On prépare ensuite l'ester puis le dérivé halogéné $R'_4$ Hal par des méthodes connues.

L'acide p-tolyl-3 phényl-3 propionique ($R_4H$) est préparé selon W. Karsten dans Chem. Ber., 1893, 26, 1579-1583. Il permet de préparer le composé $R'_4$ Hal correspondant.

L'ester éthylique de l'acide p-tolyl-2 dihalogénoacétique est préparé selon R.N. Mc Donald et al. dans J. Org. Chem., 1980, 45 (15), 2976-2984 ; il permet de préparer le dérivé halogéné correspondant.

Le p-méthylcyanure de benzyle est utilisé pour la préparation du (p-méthyl benzyl)-5 tétrazole par action de l'azide de tributylétain, ou de l'azidure de sodium. On prépare ensuite le (p-méthyl benzyl)-5 trityle tétrazole ($R'_4H$) par action du chlorure de trityle.

On peut préparer un cyanure de monoalkyl-2 , de dialkyl-2,2 ou de cycloalkyl-2 p-méthylbenzyle selon J. Org. Chem. 1969, 34, 227, par action d'un bromure d'alkyle ou de cycloalkyle sur le p-méthyl cyanure de benzyle. Ce composé peut être utilisé pour préparer l'acide correspondant puis son ester par des méthodes connues. Il peut également être utilisé par la transformation du groupe cyano en tétrazolyle par action de l'azide de tributylétain selon une méthode connue.

Le dibenzyl-2,2 p-méthyl cyanure de benzyle est obtenu par action d'un halogénure de benzyle, par exemple le bromure de benzyle, sur le p-méthyl cyanure de benzyle, en milieu basique ; on prépare ensuite le dérivé halogénure correspondant ($R'_4$ Hal).

De façon similaire, le cyano-1(methyl-4 phényl)-1 cyclohexane est obtenu par action du dibromo-1,5 pentane sur le p-méthyl cyanure de benzyle, selon le mode opératoire décrit par J. Org. Chem., 1971, 36, (9), 1308.

Pour préparer un p-tolyl cyanure d'alkyle, on peut procéder par différentes étapes successives connues. Ainsi, par exemple en opérant selon Tetrahedron, 1959, 7, 236-240, on prépare l'ester éthylique de l'acide p-tolyl-3 cyano-2 buten-2-oique ; celui-ci est transformé en ester éthylique de l'acide methyl-3 p-tolyl-3 cyano-2 butanoïque, selon J. Am. Chem. Soc., 1957, 79, 4487, puis en acide correspondant ; on utilise ensuite la méthode décrite dans la demande de brevet japonais, Japan Kokai 78-124248 pour préparer le cyanure de p-tolyl-2 méthyl-2 propyle. Eventuellement, on peut transformer ce produit en cyanure de p-tolyl-2 triméthyl-1,1,2 propyle par action d'iodure de méthyle en présence de lithium diisopropylamide.

Les composés de formule (I) pour lesquels $R_4$ représente le groupe a), dans lequel $R_7$ représente $-(CH_2)_rCOOH$, permettent de préparer les composés de formule (I) dans lesquels $R_7$ représente $-(CH_2)_r-CONHOH$ par action du chlorure de thionyle et de l'hydroxylamine.

Pour préparer un composé $R_4Hal$ dont le groupe $R_4$ représente le groupe a), dans lequel le substituant $R_8$ est un hydroxyle ou un alcoxy en $C_1$-$C_4$ et $R_7$ et $R_9$ ont les valeurs désignées ci-dessus pour (I), on peut utiliser la méthode décrite par A. Mc Kenzie et al. dans J. Chem. Soc., 1934, 1070-1075. Pour préparer les composés de formule (I) dans lesquels $R_8$ est un groupement acétoxy $R_{15}COO$, on peut effectuer la réaction d'acylation soit sur un composé de formule (I), dans laquelle $R_8$ est un hydroxyle, soit sur un composé $R'_4H$, dans lequel $R_8$ est un hydroxyle.

Un composé $R'_4Hal$ utile pour la synthèse d'un composé (I) dans lequel $R_4$ est le groupement (b) est obtenu à partir d'un composé $R'_4$ H. Par exemple, pour obtenir un groupement $R_4$ dans lequel $R_{10}$ est un phényle et

$R'_7$ est $CH_2CO_2H$, on utilise comme composé $R'_4H$ le N-phényl N-(p.tolyl) trifluoroacétamide. Celui-ci est préparé à partir de la p-tolyl N-phénylamine et d'anhydride trifluoroacétique. La p-tolyl N-phénylamine elle-même est préparée selon C. Izard-Verchere et al. dans Chim. Therap., 1971, 5, 346-351. A l'étape c1) du procédé, le groupement protecteur de l'amine, le trifluorométhylcarbonyle est remplacé par l'hydrogène en milieu basique, puis on introduit un groupement acétate de tert-butyle, par action du bromoacétate de tertiobutyle; puis l'on transforme en groupement méthylcarboxylique pour obtenir le composé (I) attendu. Cette méthode est adaptée selon la valeur des substituants ($R_6$, $R''_6$, $R'_7$ et $R_{10}$) appartenant à $R_4$.

Ainsi, pour obtenir un groupement $R_4$ dans lequel $R_7$ est un cyano et $R_{10}$ est un phényle, on utilise la méthode décrite par J.R. Robinson dans Can. J. Chem., 1954, 32, 901-905 ; on peut ensuite transformer le cyano en tétrazolyle selon les méthodes habituelles. Un composé de formule (I), dans lequel $R_4$ est le groupe b) avec $R'_7$ représentant un groupe cyano, peut être traité par du cyanure de potassium selon la méthode décrite par A. Bonetti et al. dans J. Org. Chem., 1972, 27 (21), 3352, pour préparer un composé de formule (I) dans lequel $R_7$ représente -C(= NH) $OCH_3$ ; on peut alors utiliser la méthode de M.A. Perez et al., Synthesis, 1983, 483 pour transformer $R'_7$ en oxadiazolyle.

Pour préparer un composé (I) dans lequel $R_4$ est le groupement c), on peut, soit introduire la double liaison dans le groupement $R'_4$, soit l'introduire seulement à l'étape c1) du procédé par transformation du groupement précurseur $R'_4$.

Ainsi, on peut par exemple utiliser le bromométhyl-4 benzonitrile comme composé $R'_4$ Hal ; l'étape c1) du procédé comprend alors les étapes successives suivantes : on transforme le nitrile en aldéhyde par action d'un agent réducteur comme le Nickel de Raney, puis on fait agir l'acide phénylacétique en milieu basique, selon le procédé décrit par H.E. Zimmerman et al., J. Am. Chem. Soc. 1959, 81, 2086. Dans le composé (I) ainsi obtenu, le groupement $R_4$ est le radical de l'acide p-tolyl-2 phényl-1 acrylique.

On peut également utiliser la réaction de H.E. Zimmerman pour préparer un composé $R'_4$ Hal tel que l'ester *tert*-butylique de l'acide phényl-1 [(bromométhyl-4) phényl]-2 acrylique.

Selon un autre mode de préparation, lorsque le groupement $R_4$ est c) comprenant un tétrazolyle, on peut préparer le précurseur comprenant le groupe cyano, par exemple par action d'un aldéhyde sur le p-méthyl cyanure de benzyle (Ivanov et al., Compt. Rend. Acad. Bulgar. Sci.., 1957, 10 (1), 53). Ainsi, lorsque l'aldéhyde est l'aldéhyde pivaloïque, on obtient le diméthyl-4,4 p-tolyl-2 pentène-2 nitrile. Le composé $R'_4$ Hal obtenu ensuite par les transformations habituelles est le (p-bromométhylphényl)-1 diméthyl-3,3 (trityl-1 tétrazol-5-yl)-1 butène-1.

Un autre mode de préparation du composé $R'_4$ H consiste à faire agir sur le p-tolualdéhyde ou sur une p-tolyl alkylcétone, un cyanure d'alkylphosphonate selon la réaction décrite dans Chem. Pharm. Bull., 1980, 1394. La préparation de p-tolylalkylacétone est décrite dans J. Am. Chem. Soc., 1950, 4169 et la préparation de cyanure d'alkylphosphonate est décrite par E. Schaumann et al. dans Synthesis, 1983, 449-450.

Selon un autre procédé, pour préparer un composé $R'_4H$ permettant d'obtenir un composé de formule (I), dans lequel $R_4$ est le groupement c) avec $R_{12}$ et $R_{13}$ représentant un carboxy, un alcoxycarbonyle dans lequel l'alcoxy est en $C_1$-$C_4$ ou un tétrazolyle-5, on peut faire agir un dérivé approprié de l'acide malonique sur un composé de formule :

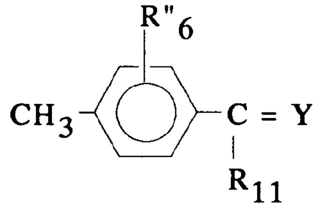

dans laquelle Y représente un oxygène ou un groupe NH et $R''_6$ et $R_{11}$ ont les valeurs indiquées ci-dessus pour (I).

La préparation d'un composé $R'_4$ Hal, dans lequel $R'_4$ est le groupement d), est effectuée à partir du p-méthyl benzonitrile qui permet d'obtenir le p-tolyl-5 tétrazole par action d'un azide, par exemple l'azidure de sodium, selon les méthodes habituelles.

Lorsque le groupement $R_4$=d, comporte une fonction carboxyle ou carboxylalkyle, la préparation du composé (I) peut être effectuée en substituant le tétrazole par la fonction appropriée dans une étape finale (étape c1) du procédé.

La préparation d'un composé $R'_4$Hal dans lequel $R'_4$ est le groupement k) est effectuée selon le mode opératoire décrit par E. Burker dans Bull. Soc. Chim. Fr., 1888, 449 et Weizmann et al. dans Chem. Ind., 1940, 402.

La préparation d'un composé $R'_4Hal$ dans lequel $R'_4$ est le groupement I) est effectuée selon le mode opératoire décrit dans la demande de brevet international WO 91-12001.

Alternativement, la préparation d'un composé (I) portant un substituant $R_4$ de formule g) est effectuée en utilisant le procédé 1 de l'invention dans lequel, à l'étape a1) le composé $R'_4$ Hal est remplacé par un composé $R'_4$ OH (3'). Le composé $R'_4$ OH est préparé par des méthodes connues. Ainsi par exemple, l'acide *tert*-butoxycarbonyl-2 biphényl-4 carboxylique, est préparé selon S. Cacchi et al. dans Chem. Ind., 1986, 286.

Les composés de formule (I) peuvent être préparés selon un autre procédé appelé procédé 2 qui est également un objet de la présente invention. Ce procédé est caractérisé en ce que:

a2) on fait réagir un aminoacide de formule :

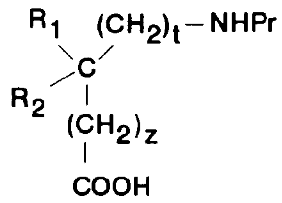

(7)

dans laquelle z, t, $R_1$ et $R_2$ ont les significations indiquées ci-dessus pour (I) et dont la fonction amine est protégée par le groupe Pr, sur un dérivé de formule :

$H_2N\text{-}R'_4$ (8)

dans laquelle $R'_4$ représente soit $R_4$ soit un groupe précurseur de $R_4$ ;

b2) après déprotection de l'amine, le composé ainsi obtenu de formule :

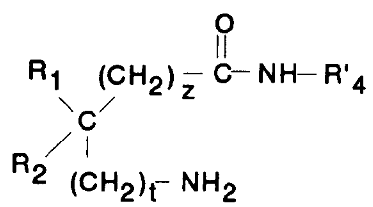

(9)

est ensuite traité par un ortho-ester d'alkyle de formule $R_3C(OR)_3$ (10) dans laquelle $R_3$ a la signification indiquée ci-dessus pour (I) et R est un alkyle en $C_1$-$C_4$ ;

c2) éventuellement, le composé ainsi obtenu de formule :

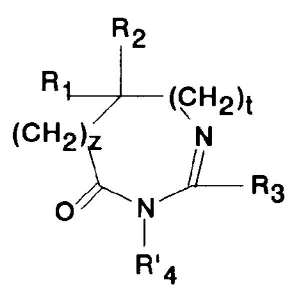

(4)

est traité par le réactif de Lawesson [bis(méthoxy-4 phényl)-2,4-dithia-1,3-diphosphétane-2,4-disulfure-2,4] ;

d2) le composé ainsi obtenu en b2) ou en c2) de formule :

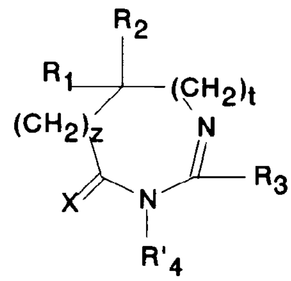

(5)

est ensuite traité dans des conditions convenables pour préparer le composé (I) par transformation du groupe $R'_4$ en groupe $R_4$.

Les composés (7) sont connus ou préparés par des méthodes connues (Chemistry of the Amino Acids, Greenstein and Winitz, John Wiley ed., 1961, vol. I, 697). Eventuellement, ces composés peuvent être obtenus optiquement purs en utilisant des méthodes de synthèse asymétrique ou de résolution du mélange racémique, telles que décrites dans "Synthesis of Optically Active alpha-aminoacids" R.M. Williams, Pergamon Press, 1989.

Les composés (8) sont connus ou préparés selon des méthodes connues. L'étape a2) est réalisée dans les conditions habituelles du couplage d'un acide sur un amine, par exemple en présence de BOP et de DIPEA.

L'étape b2) qui est la cyclisation du composé (9) en présence de (10) est effectuée selon Jacquier et al. (Bull. Soc. Chim. France, 1971, (3), 1040-1051) et selon Brunken et Bach (Chem. Ber., 1956, 89, 1363-1373).

Dans la description ci-après, le procédé comprenant les étapes a2) à d2) est appelé procédé (2).

Selon une variante du procédé (2), à l'étape b2), on peut éventuellement isoler un intermédiaire (9') de formule :

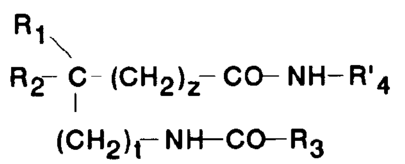

(9')

puis préparer le composé (4) par cyclisation en milieu acide. Selon un autre variante du procédé (2) et pour préparer un composé (I) dans lequel z=0 et $R_1R_2$ représente un groupe $=CR'_1R'_2$ on peut faire réagir en milieu acide, un aminoacide de formule :

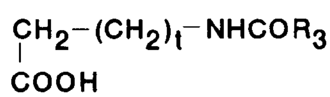

(7')

sur un aldehyde ou une cétone de formule :

$$R'_1COR'_2$$

dans laquelle $R'_1$ et $R'_2$ ont les significations données ci-dessus pour (I), puis par action du composé (8), on obtient un composé de formule :

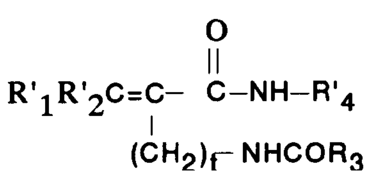

(9")

La cyclisation de ce composé en milieu acide conduit au composé (4).

De façon préférentielle, le procédé (2) est utilisé pour la préparation des composés (I) dans lesquels $R_4$ représente l'un des groupements e), f) ou h).

Pour la préparation d'un composé $R'_4$ $NH_2$ (8) dans lequel $R'_4$ est un biphényl substitué, transformé en $R_4$=e) à l'étape c2), on utilise le mode opératoire décrit par J. Witte et al. dans J. Org. Chem., 1972, 37 (18),

2849.

Lorsque $R_4$ est le groupement f), la préparation de $R'_4$ $NH_2$ est effectuée selon A.F. Mc Kay et al., Can. J. Chem, 1960, 38, 343.

Enfin, lorsque $R_4$ est le groupement h), la préparation de $R'_4$ H est effectuée selon S.A. Glover et al., J. Chem. Soc., Perkin I, 1981, 842 ; on prépare ensuite $R'_4$ $NH_2$ selon J. Witte et al., J. Org. Chem., 1972, 37 (18), 2849.

Les composés (I) selon l'invention, dans lesquels $R_1$ et $R_2$ liés ensemble représentent un groupe de formule $-(CH_2)_pY\ (CH_2)_q-$ dans lequel Y est un groupe NH, peuvent être préparés par hydrogénolyse catalytique d'un composé (I) correspondant dans lequel Y est un groupe $N-R_5$, $R_5$ étant un benzyle.

L'affinité des produits selon l'invention pour les récepteurs de l'angiotensine II a été étudiée sur un test de liaison de l'angiotensine II marquée à l'iode 125 à des récepteurs membranaires de foie de rats. La méthode utilisée est celle décrite par S. Keppens et al. dans Biochem. J., 1982, 208, 809-817.

On mesure la $CI_{50}$ : concentration qui donne 50% de déplacement de l'angiotensine II marquée, liée spécifiquement au récepteur. La $CI_{50}$ des composés selon l'invention est inférieure à $10^{-6}$ M.

De plus, l'effet antagoniste de l'angiotensine II des produits selon l'invention a été constaté sur différentes espèces animales dans lesquelles le système rénine-angiotensine a été préalablement activé (C. Lacour et al., J. Hypertension, 1989, 7 (suppl. 2), S33-S35).

Les composés selon l'invention sont actifs après administation par différentes voies, notamment par voie orale.

Aucun signe de toxicité n'est observé avec ces composés aux doses pharmacologiquement actives.

Ainsi les composés selon l'invention peuvent être utilisés dans le traitement de différentes affections cardiovasculaires, notamment l'hypertension, la défaillance cardiaque, ainsi que dans le traitement du glaucome, des rétinopathies diabétiques et de différentes affections du système nerveux central, les déficits mnésiques ou la maladie d'Alzheimer par exemple.

Le présente invention a également pour objet des compositions pharmaceutiques contenant une dose efficace d'un composé selon l'invention ou de l'un de ses sels pharmaceutiquement acceptables et des excipients convenables. Lesdits excipients sont choisis selon la forme pharmaceutique et le mode d'administration souhaités.

Dans les compositions pharmaceutiques de la présente invention pour l'administration orale, sublinguale, sous-cutanée, intramusculaire, intraveineuse, topique, intratrachéale, intranasale, transdermique ou rectale, les principes actifs de formule (I) ci-dessus, ou leurs sels éventuels, peuvent être administrés sous forme unitaire d'administration, en mélange avec des supports pharmaceutiques classiques, aux animaux et aux êtres humains pour la prophylaxie ou le traitement des troubles ou des maladies ci-dessus. Les formes unitaires d'administration appropriées comprennent les formes par voie orale telles que les comprimés, les gélules, les poudres, les granules et les solutions ou suspensions orales, les formes d'administration sublinguale, buccale, intratrachéale, intranasale, les formes d'administration sous-cutanée, intramusculaire ou intraveineuse et les formes d'administration rectale. Pour l'application topique, on peut utiliser les composés selon l'invention dans des crèmes, pommades ou lotions.

Afin d'obtenir l'effet prophylactique ou thérapeutique désiré, la dose de principe actif put varier entre 0,01 et 50 mg par kg de poids du corps et par jour.

Chaque dose unitaire peut contenir de 0,1 à 1000 mg, de préférence de 1 à 500 mg, d'ingrédients actifs en combinaison avec un support pharmaceutique. Cette dose unitaire peut être administrée 1 à 5 fois par jour de façon à administrer un dosage journalier de 0,5 à 5000 mg, de préférence de 1 à 2500 mg.

Lorsqu'on prépare une composition solide sous forme de comprimés, on mélange l'ingrédient actif principal avec un véhicule pharmaceutique tel que la gélatine, l'amidon, la lactose, le stéarate de magnésium, le talc, la gomme arabique ou analogues. On peut enrober les comprimés de saccharose, d'un dérivé cellulosique, ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'elixir ou pour l'administration sous forme de gouttes peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les compositions de la présente invention peuvent contenir, à côté des produits de formule (I) ci-dessus ou d'un de ses sels pharmaceutiquement acceptables, d'autres principes actifs tels que, par exemple, des tranquillisants ou d'autres médicaments qui peuvent être utiles dans le traitement des troubles ou maladies indiquées ci-dessus.

Ainsi, la présente invention a pour objet des compositions pharmaceutiques contenant plusieurs principes actifs en association dont l'un est un composé selon l'invention et le ou les autres peuvent être un composé béta-bloquant, un antagoniste calcique, un diurétique, un antiinflammatoire non stéroïdien ou un tranquillisant.

Les exemples suivants illustrent l'invention sans toutefois la limiter. Dans ces exemples, on utilise les abréviations suivantes :

TA signifie température ambiante, $KHSO_4$-$K_2SO_4$ signifie une solution aqueuse contenant 16,6 g de bisulfate de potassium et 33,3 g de sulfate de potassium pour 1 litre.

Les points de fusion (Fc) sont donnés en degrés Celsius, sauf indication contraire ; ils ont été mesurés sans recristallisation du produit.

La pureté des produits est vérifiée en chromatographie sur couche mince (CCM) ou par HPLC. Les produits sont caractérisés par leur point de fusion ou par leur spectre RMN enregistré à 200 MHz dans DMSO deutérié, la référence interne étant le tétraméthylsilane.

Pour l'interprétation des spectres de RMN, on emploie :

| | |
|---|---|
| s | pour singulet |
| s.e. | pour singulet élargi |
| d | pour doublet |
| t | pour triplet |
| q | pour quadruplet |
| quint | pour quintuplet |
| sext | pour sextuplet |
| m | pour massif ou multiplet |

effet N.O.E. signifie Nuclear Overhauser Effect.

Pour chacun des exemples, on indique la valeur du substituant $R_4$ du composé (I) préparé.

Sauf indication contraire, les composés présentant 1 ou plusieurs atomes de carbone asymétriques sont obtenus sous forme de racémiques ou d'un mélange de diastéréoisomères.

EXEMPLE 1

Acide [((n-butyl-2 oxo-5 spirocyclopentane-4 imidazolin-2-yl-1) méthyl)-4 phényl]-2 phénylacétique.

$R_4$ = 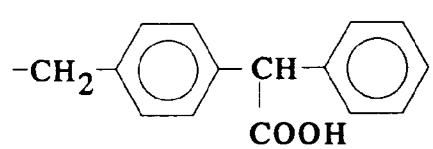

A) Acide *p*-tolyl-2 phénylacétique.

Cet acide est préparé à partir de l'acide mandélique par action du toluène selon le mode opératoire décrit par J. Gyr dans Chem. Ber., 1908, 41, 4308-4322. Après cristallisation dans un mélange éther-hexane, on obtient un produit qui contient, d'après son spectre RMN, environ 15 % d'acide *o*-tolyl-2 phénylacétique.
Fc = 100-105°C

B) Ester *tert*-butylique de l'acide *p*-tolyl-2 phénylacétique.

On dissout 12 g de l'acide obtenu ci-dessus dans 55 ml de dichlorométhane et l'on ajoute goutte à goutte à 0°C 5,3 ml de chlorure d'oxalyle puis on laisse sous agitation 5 heures à TA. Le milieu réactionnel est ensuite concentré sous vide puis évaporé 2 fois avec du benzène. Le résidu obtenu est dissous dans 150 ml de THF, on ajoute, à 0°C, 6,9 g de *tert*-butylate de potassium puis on agite 1 heure et demie à TA. On concentre le

résidu sous vide à moitié, puis on le reprend dans un mélange eau-éther ; les phases éthérées sont décantées puis lavées par une solution saturée de chlorure de sodium, séchées sur sulfate de sodium et concentrées sous vide. Après chromatographie sur silice, on obtient 7,90 g du produit attendu identifié par ses spectres RMN et IR.

C) Ester *tert*-butylique de l'acide *p*-bromométhylphényl-2 phénylacétique.

3,7 g de l'ester obtenu ci-dessus sont dissous dans 150 ml de tétrachlorure de carbone et traités par 2,33 g de N-bromosuccinimide en présence de 200 mg de peroxyde de benzoyle, à reflux, sous irradiation U.V. pendant 1 heure. Le succinimide formé est éliminé par filtration puis le filtrat est concentré. Le dérivé bromé est obtenu sous forme d'une huile rouge.

D) n-butyl-2 spirocyclopentane-4 imidazoline-2 one-5.

L'ester éthylique de l'acide amino-1 cyclopentane carboxylique est préparé selon Adkins et Billica (J. Amer. Chem. Soc., 1948, 70, 3121-3125).

Le valérimidate d'éthyle est préparé selon Mac Elvain (J. Amer. Chem. Soc., 1942, 64, 1825-1827) puis libéré de son chlorhydrate par action du carbonate de potassium et extraction par le DCM.

L'ester éthylique de l'acide amino-1 cyclopentane carboxylique (1,57 g) et le valérimidate (1,56 g) sont dissous dans 12 ml de xylène contenant 6 gouttes d'acide acétique. Après 6 heures et demie de chauffage à reflux, le milieu réactionnel est concentré sous vide puis le résidu est chromatographié sur gel de silice en éluant par un mélange chloroforme/méthanol/acide acétique (94/4/2 ; v/v/v). La fraction contenant le produit attendu est évaporée plusieurs fois en présence de xylène puis de benzène pour éliminer l'acide acétique. On obtient 1,91 g de produit sous forme d'une huile épaisse.

- IR ($CHCl_3$):
  - 1720 $cm^{-1}$ : C = O
  - 1635 $cm^{-1}$ : C = N

Remarque: le fait de ne pas voir de bande entre 1500 et 1600 $cm^{-1}$ indique que, dans la solution de chloroforme, le produit est une imidazolinone-5.

- RMN:
  - 0,92 ppm : t : 3 H : $CH_3$ (nBu)
  - 1,35 ppm : sext : 2 H : $CH_3$-$C\underline{H_2}$-
  - 1,50-1,93 ppm : m : 10 H : $CH_3$-$CH_2$-$C\underline{H_2}$ et cyclopentane
  - 2,33 ppm : t : 2 H : $CH_3$-$CH_2$-$CH_2$-$C\underline{H_2}$-
  - 10,7 ppm : s.e. : N$\underline{H}$
- spectre de masse : $MH^+$ : 195

Pour préparer le chlorhydrate du n-butyl-2 spirocyclopentane-4 imidazoline-2 one-5, on dissout 8,5 g de ce composé dans 100 ml d'isopropanol et l'on ajoute goutte à goutte 3,7 ml d'acide chlorhydrique concentré (d=1,18). Le milieu est rendu homogène par chauffage pendant quelques minutes à 70°C puis on refroidit à 15°C. Les cristaux formés sont essorés, lavés à l'isopropanol, puis séchés.

E) Ester *tert*-butylique de l'acide [((n-butyl-2 oxo-5 spirocyclopentane-4 imidazolin-2-yl-1) méthyl)-4 phényl]-2 phényl-2 acétique.

On dissout 800 mg du produit préparé à l'étape D dans 4 ml de DMF et on ajoute 240 mg de méthylate de sodium puis, après 15 minutes, on ajoute 2,16 g du produit préparé à l'étape C, dissous dans 3 ml de DMF, puis on chauffe pendant 3 heures et demie à 40°C. Le milieu réactionnel est repris dans un mélange eau-acétate d'éthyle, la phase organique est décantée puis relavée par une solution saturée de chlorure de sodium et évaporée sous vide. Le résidu est chromatographié sur silice en éluant par un mélange toluène-acétate d'éthyle. On obtient 320 mg du produit attendu.

- IR ($CHCl_3$) :
  - 1710-1720: C=O : imidazolinone et ester
  - 1625 $cm^{-1}$ : C = N imidazolinone
- RMN :
  - 7,30-6,90 ppm : m : 9 H: aromatiques
  - 4,90 ppm : s : 1 H : C$\underline{H}$-$CO_2$(tBu)
  - 4,55 ppm : s : 2 H : N-$C\underline{H_2}$-$C_6H_4$-
  - 2,20 ppm : t : 2 H : $CH_3$-$CH_2$-$CH_2$-$C\underline{H_2}$-

1,6-1,8 ppm : m : 8 H : cyclopentane
1,20 ppm : quint : 2 H : $CH_3$-$CH_2$-C<u>H</u>$_2$-
1,15 ppm : s : 9 H : (tBu)
1,10 ppm : sext : 2 H : $CH_3$-C<u>H</u>$_2$-
0,75 ppm : t : 3 H : $CH_3$ (nBu)

F) Acide [((n-butyl-2 oxo-5 spirocyclopentane-4 imidazolin-2-yl-1) méthyl)-4 phényl]-2 phényl-2 acétique.

On traite le produit obtenu à l'étape ci-dessus par 4 ml de TFA dans 3 ml de DCM pendant 45 minutes à 25°C. Après évaporation sous vide, le résidu est traité par de l'hexane, il se forme un précipité. Le solide obtenu est chromatographié sur silice en éluant par un mélange AcOEt/MeOH/AcOH (97/3/0,3 ; v/v/v). On obtient 110 mg du produit attendu.
Fc = 146-148°C.
- spectre de masse: $MH^+$ = 419
- RMN :
7,10-7,40 ppm : m : 9 H : aromatiques
5,05 ppm : s : 1 H : C<u>H</u>-$CO_2H$
4,65 ppm : s : 2 H : N-C<u>H</u>$_2$-$C_6H_4$-
2,30 ppm : t : 2 H : $CH_3$-$CH_2$-$CH_2$-C<u>H</u>$_2$-
1,60-2 ppm : m : 8 H : cyclopentane
1,50 ppm : quint : 2 H : $CH_3$-$CH_2$-C<u>H</u>$_2$-$CH_2$
1,25 pm : sext : 2 H : $CH_3$-C<u>H</u>$_2$-$CH_2$-$CH_2$
0,80 ppm : t : 3 H : $CH_3$ (n-Bu).

EXEMPLE 2

Acide [((n-butyl-2 oxo-5 spirocyclopentane-4 imidazolin-2-yl-1) méthyl)-4 phényl]-2 phényl-2 propionique.

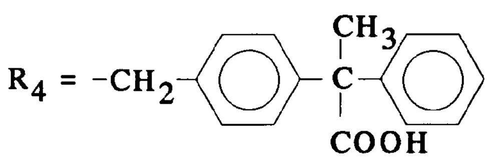

A) Ester *tert*-butylique de l'acide *p*-tolyl-2 phényl-2 propionique.

On dissout dans 80 ml de THF 5,9 g d'ester *tert*-butylique de l'acide *p*-tolyl-2 phényl-2 acétique, préparé à l'exemple 1 étape B, et l'on ajoute à 0°C 3,15 ml d'iodure de méthyle puis 1,2 g d'hydrure de sodium à 50% dans l'huile. Après une nuit sous agitation à TA, le milieu réactionnel est dilué par 300 ml d'acétate d'éthyle puis 50 ml d'eau. La phase organique est décantée, relavée par une solution saturée de chlorure de sodium, séchée sur sulfate de sodium puis concentrée sous vide. Le résidu est chromatographié sur silice par AcOEt/hexane. On obtient 4,7 g du produit attendu sous forme d'une huile identifiée par ses spectres IR et RMN.

B) Ester *tert*-butylique de l'acide *p*-bromométhylphényl-2 phényl-2 propionique.

2,96 g de l'ester obtenu ci-dessus sont dissous dans 120 ml de tétrachlorure de carbone et traités par 1,78 g de NBS, en présence de péroxyde de benzoyle, pendant 1 heure à reflux. Le succinimide formé est éliminé par filtration puis le filtrat est évaporé sous vide. On obtient 3,7 g du produit attendu identifié par son spectre RMN.

C) Ester *tert*-butylique de l'acide [((n-butyl-2 oxo-5 spirocyclopentane-4 imidazolin-2-yl-1) méthyl)-4 phényl]-2 phényl-2 propionique.

A 120 mg d'hydrure de sodium à 80 % dans l'huile en suspension dans 4 ml de DMF, on ajoute 500 mg d'imidazoline-2 one-5, préparée à l'exemple 1, étape D, dissoute dans 5 ml de DMF. Après 15 minutes, on ajoute 1,02 g du dérivé bromé, préparé à l'étape précédente, dissous dans 5 ml de DMF et on laisse sous agitation à TA pendant 2 heures. Le milieu réactionnel est dilué par 100 ml d'AcOEt, puis 25 ml d'eau. La phase organique

est décantée, lavée par une solution saturée de chlorure de sodium, séchée sur sulfate de sodium puis concentrée sous vide. Le résidu est chromatographié sur silice en éluant par un mélange AcOEt-toluène. On obtient 550 mg du produit attendu.

- IR ($CHCl_3$) :
  - 1720-1715 $cm^{-1}$ : C = O : imidazolinone et ester
  - 1625 $cm^{-1}$ : C = N imidazolinone
- RMN
  - 1,95 ppm : s : 3 H : $C\underline{H_3}$-C-$CO_2$tBu

D) Acide [((n-butyl-2 oxo-5 spirocyclopentane-4 imidazolin-2-yl-1) méthyl)-4 phényl]-2 phényl-2 propionique.

530 mg de l'ester obtenu à l'étape précédente sont traités pendant 45 minutes par 5 ml de TFA dans 4 ml de DCM. Après concentration sous vide, le résidu est repris dans un mélange éther-hexane. Après une nuit à 0°C, les cristaux obtenus sont filtrés, lavés à l'hexane puis séchés sous vide. On obtient 460 mg du produit attendu.

Fc = 118-120°C.

- RMN :
  - 7,0-7,30 ppm : m : 9 H : aromatiques
  - 4,75 ppm : s : 2 H : N-$C\underline{H_2}$-$C_6H_4$
  - 2,50 ppm : t : 2 H : $CH_3$-$CH_2$-$CH_2$-$C\underline{H_2}$-
  - 1,60-1,95 ppm : m : 11 H : cyclopentane + $C\underline{H_3}$-C-$CO_2$H
  - 1,40 ppm : quint : 2 H : $CH_3$-$CH_2$-$C\underline{H_2}$-$CH_2$-
  - 1,20 ppm : sext : 2 H : $CH_3$-$C\underline{H_2}$-$CH_2$-$CH_2$-
  - 0,70 ppm : t : 3 H : $CH_3$ (n-Bu)

EXEMPLE 3

Acide [((n-butyl-2 oxo-5 spirocyclopentane-4 imidazolin-2-yl-1) méthyl)-4 phényl]-3 phényl-3 propionique.

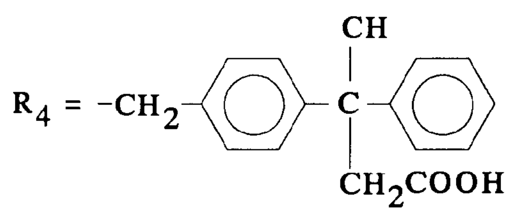

A) Acide *p*-tolyl-3 phényl-3 propionique.

Cet acide est préparé selon Karsten (Chem. Ber., 1893, 26, 1579-1583).

B) Ester *tert*-butylique de l'acide *p*-tolyl-3 phényl-3 propionique.

On dissout 5 g de l'acide préparé ci-dessus dans 30 ml de DCM ; on ajoute, à 0°C, 2 ml de chlorure d'oxalyle et on laisse 3 heures sous agitation à TA. Le milieu réactionnel est évaporé à sec puis reévaporé 2 fois avec du benzène. Le résidu est dissous dans 30 ml de THF ; on ajoute, à 0°C, 2,9 g de *tert*-butylate de potassium et on agite 1 heure à TA. On ajoute ensuite 150 ml d'éther et 50 ml d'eau puis la phase organique est lavée par une solution saturée de chlorure de sodium, séchée sur sulfate de sodium et concentrée sous vide. On obtient le produit attendu sous forme d'une huile qui cristallise peu à peu (rendement 67%).

Fc = 45°C.

Le produit est identifié par ses spectres IR et RMN.

C) Ester *tert*-butylique de l'acide (bromométhyl-4 phényl)-3 phényl-3 propionique.

A 4,1 g de l'ester précédent dissous dans 150 ml de tétrachlorure de carbone, on ajoute 200 mg de péroxyde de benzoyle, 2,49 g de NBS et on porte au reflux pendant 2 heures sous un rayonnement U.V. Après filtration du succinimide, le filtrat est concentré sous vide. L'analyse du spectre RMN montre qu'il s'est également formé un dérivé dibromé, à savoir l'ester *tert*-butylique de l'acide bromo-3 (bromométhyl-4 phényl)-3 phényl-3 propionique.

D) Ester *tert*-butylique de l'acide [((n-butyl-2 oxo-5 spirocyclopentane-4 imidazolin-2-yl-1) méthyl)-4 phényl]-3 phényl-3 propionique et ester *tert*-butylique de l'acide [((n-butyl-2 oxo-5 spirocyclopentane-4 imidazolin-2-yl-1) méthyl)-4 phényl]-3 phényl-3 acrylique.

Le mélange de composés obtenus à l'étape précédente est ajouté au n-butyl-2 spirocyclopentane-4 imidazoline-2 one-5, préparé à l'exemple 1, étape D. La réaction est effectuée dans le DMF en présence d'hydrure de sodium. Le produit obtenu est un mélange qui comprend 2/3 de l'ester de l'acide propionique et 1/3 de l'ester de l'acide acrylique.

- RMN :
  - ester de l'acide propionique :
  - 4,40 ppm : t : 1 H : $-C\underline{H}-CH_2-$
  - 3,0 ppm : d : 2 H : $-CH-C\underline{H}_2-$
  - ester de l'acide acrylique :
  - 6,40 ppm : s : H : éthylénique

E) Acide [((n-butyl-2 oxo-5 spirocyclopentane-4 imidazolin-2-yl-1) méthyl)-4 phényl]-3 phényl-3 propionique.

Le mélange d'esters *tert*-butyliques préparés à l'étape précédente est hydrolysé par action de l'acide trifluoroacétique dans le DCM selon la méthode précédemment décrite.

300 mg du mélange d'acides ainsi obtenu sont dissous dans 10 ml d'éthanol et on ajoute 100 mg de Palladium à 5 % sur sulfate de baryum. On agite sous atmosphère d'hydrogène pendant 3 heures. Le catalyseur est éliminé par filtration, le filtrat est concentré à sec puis repris par de l'acétate d'éthyle, on ajoute de l'eau et on amène à pH 5 par addition d'une solution d'hydrogenocarbonate de sodium.

La phase organique est lavée par une solution saturée de chlorure de sodium, séchée sur sulfate de sodium puis concentrée sous vide. Le résidu obtenu est repris par de l'éther, le précipité formé est filtré, lavé à l'éther et séché.

m = 140 mg

Fc = 138-140°C

- spectre de masse : $MH^+$ : 433.
- RMN :
  - 7,10-7,50 ppm : m : 9 H : aromatiques
  - 4,75 ppm : s : 2 H : $N-C\underline{H}_2-C_6H_4-$
  - 4,45 ppm : t : 1 H : $C\underline{H}-CH_2-CO_2H$
  - 3,05 ppm : d : 2 H : $CH-C\underline{H}_2-CO_2H$
  - 2,40 ppm : t : 2 H : $-C\underline{H}_2-CH_2-CH_2-CH_3$
  - 2,05-1,70 ppm : m : 8 H : cyclopentane
  - 1,45 ppm : q : 2 H : $CH_2-C\underline{H}_2-CH_2-CH_3$
  - 1,15 ppm : sext : 2 H : $CH_2-CH_2-C\underline{H}_2-CH_3$
  - 0,80 ppm : t : 3 H : $CH_2-CH_2-CH_2-C\underline{H}_3$

EXEMPLE 4

Acide [((n-butyl-2 oxo-5 spirocyclopentane-4 imidazolin-2-yl-1) méthyl)-4 phényl]-2 diphényl-2,2 acétique.

$R_4$ = 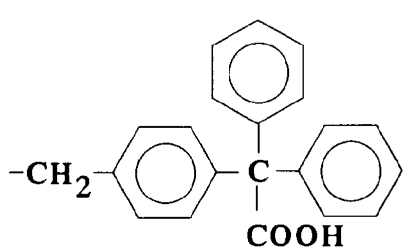

A) Acide diphényl-2,2 *p*-tolyl-2 acétique.

Cet acide est préparé selon la méthode de A. Bistrzycki (Chem. Ber. 1901, 34, 3079-3080) par action du

toluène sur l'acide benzilique.

B) Ester *tert*-butylique de l'acide diphényl-2,2 *p*-tolyl-2 acétique.

On place 5 g de l'acide précédent dans 30 ml de DCM et 30 ml de benzène et l'on ajoute à 0°C, 2,31 g de chlorure d'oxalyle puis on laisse 3 heures sous agitation à TA. Le milieu réactionnel est concentré sous vide puis évaporé 2 fois avec du benzène, le résidu est repris dans 50 ml de THF, on ajoute 2,5 g de *tert*-butylate de potassium et on agite 1 heure à TA. Le milieu réactionnel est dilué par 200 ml d'acétate d'éthyle et 50 ml d'eau, la phase organique est décantée, lavée par une solution saturée de chlorure de sodium, séchée sur sulfate de sodium et évaporée à sec. L'ester attendu est obtenu sous forme d'une huile et identifié par son spectre de RMN.

C) Ester *tert*-butylique de l'acide *p*-bromométhyl phényl-2 diphényl-2,2 acétique.

On dissout 1 g de l'ester obtenu à l'étape précédente dans 40 ml de tétrachlorure de carbone et l'on ajoute 50 mg de péroxyde de benzoyle et 534 mg de NBS. Après 3 heures et demie de chauffage à reflux sous irradiation U.V., le succinimide formé est filtré puis le filtrat est concentré sous vide. On obtient 1,13 g d'huile.

D) Ester *tert*-butylique de l'acide [((n-butyl-2 oxo-5 spirocyclopentane-4 imidazolin-2-yl-1) méthyl)-4 phényl]-2 diphényl-2,2 acétique.

On utilise le chlorhydrate du n-butyl-2 spirocyclopentane-4 imidazoline-2 one-5 obtenu à l'exemple 1, étape D.

578 mg de ce composé sont placés dans 10 ml de DMF en présence de 150 mg d'hydrure de sodium à 80% dans l'huile. Après 20 minutes, on ajoute le dérivé bromé obtenu à l'étape précédente (1,13 g) dans 10 ml de DMF et on laisse 30 minutes sous agitation. Le milieu réactionnel est concentré de moitié puis dilué par 100 ml d'acétate d'éthyle et 15 ml d'eau. La phase organique est décantée, lavée par une solution saturée de chlorure de sodium, séchée sur sulfate de sodium puis concentrée sous vide. Le résidu est chromatographié sur silice en éluant par un mélange acétate d'éthyle/toluène. On obtient 580 mg du produit attendu sous forme de cire, caractérisé par les spectres RMN et IR.

- IR ($CHCl_3$) :
  - 1725-1715 $cm^{-1}$ : C=O : imidazolinone et ester
  - 1630 $cm^{-1}$ : C = N : imidazolinone

E) Acide [((n-butyl-2 oxo-5 spirocyclopentane-4 imidazolin-2-yl-1) méthyl)-4 phényl]-2 diphényl-2,2 acétique.

Le produit obtenu à l'étape précédente est traité pendant 45 minutes à TA par 3 ml de TFA dans 3 ml de DCM. Après évaporation à sec, le résidu est repris dans l'éther, le précipité formé est filtré, lavé à l'éther, séché. On obtient 500 mg de poudre blanche.

Fc = 197°C.

- RMN :
  - 7,10-7,40 ppm : m : 14 H : aromatiques
  - 4,85 ppm : s : 2 H : N-C<u>H</u>$_2$-$C_6H_4$-
  - 2,70 ppm : t : 2 H : $CH_3$-$CH_2$-$CH_2$-C<u>H</u>$_2$-
  - 1,80-2,10 ppm : m : 8 H : cyclopentane
  - 1,50 ppm : quint : 2 H : $CH_3$-$CH_2$-C<u>H</u>$_2$-$CH_2$-
  - 1,30 ppm : sext : 2 H : $CH_3$-C<u>H</u>$_2$-$CH_2$-$CH_2$-
  - 0,80 ppm : t : 3 H : $CH_3$ (nBu)

EXEMPLE 5

[(dibenzyl tétrazol-5-yl méthyl)-4 benzyl]-1 n-butyl-2 spirocyclopentane-imidazoline-2 one-5.

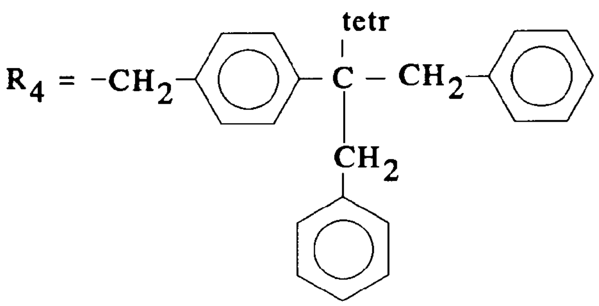

A) diphényl-1,3 *p*-tolyl-2 cyano-2 propane.

A une solution de 10,7 ml de bromure de benzyle dans 25 ml de DMSO, on ajoute simultanément en 15 minutes 3,93 g de *p*-méthyl cyanure de benzyle dans 7 ml de DMSO et 4,8 g de soude dans 5 ml d'eau ; on refroidit par un bain d'eau à 20°C de façon à ce que la température du milieu réactionnel ne dépasse pas 60°C. 1 heure 45 minutes après la fin de l'addition, le milieu réactionnel est dilué par 150 ml d'éther et 50 ml d'eau, la phase organique est décantée, lavée par une solution saturée de chlorure de sodium, séchée sur sulfate de sodium et concentrée sous vide ; le résidu solide est repris dans de l'éther, filtré et séché. On obtient 7 g de solide blanc.

Fc = 128-129°C.

- IR ($CHCl_3$):

  2200 $cm^{-1}$ : C ≡ N

B) diphényl-1,3 (bromométhyl-4 phényl)-2 cyano-2 propane.

On dissout 3,11 g du produit obtenu à l'étape précédente dans 150 ml de tétrachlorure de carbone, on ajoute 1,78 g de NBS, 90 mg de péroxyde de benzoyle et on chauffe à reflux pendant 2 heures 15 minutes. Le succinimide formé est filtré et le filtrat est concentré sous vide. Le dérivé bromé attendu est obtenu sous forme de cire et identifié par son spectre RMN. La réaction est quantitative.

C) [(diphényl-cyanométhyl)-4 benzyl]-1 n-butyl-2 spirocyclopentane-4 imidazoline 2 one-5.

On dissout 920 mg de chlorhydrate de n-butyl-2 spirocyclopentane-4 imidazoline-2 one-5 dans 15 ml de DMF, on ajoute 300 mg d'hydrure de sodium à 80% dans l'huile et, après 10 minutes, on ajoute 1,86 g du dérivé bromé préparé à l'étape précédente, dissous dans 10 ml de DMF. Après 2 heures et demie d'agitation, le milieu réactionnel est dilué par 150 ml d'acétate d'éthyle et 20 ml d'eau. La phase organique est décantée, lavée par une solution saturée de chlorure de sodium, séchée sur sulfate de sodium et concentrée sous vide. Le résidu est chromatographié sur silice en éluant par un mélange acétate d'éthyle/toluène. On obtient 1,80 g du produit attendu sous forme de mousse.

- IR ($CHCl_3$) :

  1625 $cm^{-1}$ : C = N imidazolinone

  2200 $cm^{-1}$ : C ≡ N faible.

D) [(dibenzyl tétrazol-5-yl méthyl)-4 benzyl]-1 n-butyl-2 spirocyclopentane-imidazoline-2 one-5.

On prépare l'azide de tributylétain selon la méthode décrite par Kricheldorf et Leppert dans Synthesis, 1976, 329.

On chauffe à reflux pendant 95 heures dans 20 ml de xylène, 1,8 g du composé préparé à l'étape C, et 1,1 équivalent d'azide de tributylétain. Après refroidissement, le milieu réactionnel est dilué par 30 ml de xylène puis extrait 3 fois par 20 ml de soude 1N. Les 3 phases aqueuses sont réunies puis extraites 2 fois par 20 ml d'éther. On ajoute 200 ml de DCM à la phase aqueuse puis on amène à pH 5,0 par addition d'acide chlorhydrique 3 N. La phase organique est décantée, lavée 1 fois à l'eau, 1 fois par une solution saturée de chlorure de sodium, séchée sur sulfate de sodium puis évaporée à sec. Le résidu est repris par de l'éther et le solide

obtenu est filtré et séché.
m = 700 mg
Fc = 157-159°C.
- RMN :
7,6-7,10 ppm : m : 14 H : aromatiques
4,60 ppm : s : 2 H : N-C<u>H</u>$_2$-$C_6H_4$
3,35-3,65 ppm : 4 s : 4 H : 2C<u>H</u>$_2$ (benzyles)
2,25 ppm : t : 2 H : $CH_3$-$CH_2$-$CH_2$-C<u>H</u>$_2$
1,95 ppm : m : 8 H : cyclopentane
1,50 ppm : quint : 2 H : $CH_3CH_2$--C<u>H</u>$_2$-$CH_2$
1,25 ppm : sext : 2 H : $CH_3$-C<u>H</u>$_2$-$CH_2$-$CH_2$-
0,80 ppm : t : 3 H : $CH_3$ (n-Bu)

EXEMPLE 6

Acide (E) [(n-butyl-2 oxo-5 spirocyclopentane-4 imidazolin-2-yl-1) méthyl)-4 phényl]-3 phényl-2 acrylique.

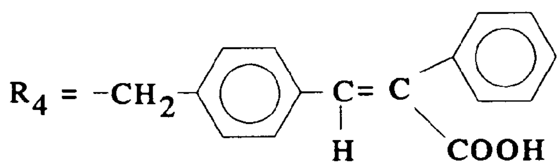

A) [(n-butyl-2 oxo-5 spirocyclopentane-4 imidazolin-2-yl-1) méthyl]-4 benzonitrile.

On met en suspension dans 15 ml de DMF anhydre, sous argon, 1,14 g d'hydrure de sodium à 80 % dans l'huile et l'on ajoute goutte à goutte à 0°C, sous argon, 4 g de n-butyl-2 spirocyclopentane-4 imidazoline-2 one-5 dans 15 ml de DMF anhydre. Après 20 minutes d'agitation à TA sous argon, on ajoute 3,74 g de bromométhyl-4 benzonitrile. Après 20 minutes, on concentre le DMF sous vide puis on reprend par 150 ml d'acétate d'éthyle et on lave avec successivement 100 ml d'eau, 100 ml de solution aqueuse de $KHSO_4$- $K_2SO_4$ à 5 %, 50 ml de solution saturée de chlorure de sodium, 100 ml de solution saturée d'hydrogénocarbonate de sodium,et à nouveau 50 ml de solution saturée de chlorure de sodium. On sèche sur sulfate de sodium, on filtre et on concentre le filtrat. On obtient 5,64 g du produit attendu sous forme d'une huile.
- RMN :
0,80 ppm : t : 3 H : $CH_3$ (nBu)
1,25 ppm : sext : 2 H : $CH_3$-C<u>H</u>$_2$-
1,50 ppm : m : 2 H : $CH_3$-$CH_2$-C<u>H</u>$_2$-
1,75-2,0 ppm : m : 8 H : cyclopentane
2,35 ppm : t : 2 H : $CH_3$-$CH_2$-$CH_2$-C<u>H</u>$_2$-
4,8 ppm : s : 2 H : C<u>H</u>$_2$-$C_6H_4$-
7,6 ppm : q : 4 H : aromatiques

B) [(n-butyl-2 oxo-5 spirocyclopentane-4 imidazolin-1-yl) méthyl]-4 benzaldéhyde.

Dans un ballon, on place 4,5 g de Nickel de Raney, 4,5 g du composé obtenu à l'étape précédente et 70 ml d'acide formique aqueux à 75 % et on chauffe à reflux pendant 1 heure. Le Nickel est filtré sur Célite® puis on dilue le filtrat avec 100 ml d'eau et on extrait 3 fois par 50 ml d'acétate d'éthyle. On rassemble les phases organiques et on les lave 3 fois par 100 ml d'une solution saturée d'hydrogénocarbonate de sodium puis par 100 ml de solution saturée de chlorure de sodium puis on sèche sur sulfate de sodium, on filtre et on concentre le filtrat. L'huile obtenue est purifiée par chromatographie sur silice en éluant par un mélange hexane/acétate d'éthyle (6/4 ; v/v). On obtient 1,93 g du composé attendu sous forme d'une huile.
- RMN :
0,89 ppm : t : 3 H : $CH_3$ (nBu)
1,15 ppm : sext : 2 H : $CH_3$-C<u>H</u>$_2$-
1,40 ppm : m : 2 H : $CH_3$-$CH_2$-C<u>H</u>$_2$-
1,5-1,8 ppm : m : 8 H : cyclopentane

2,20 ppm : t : 2 H : $CH_3$-$CH_2$-$CH_2$-C<u>H</u>$_2$-
4,8 ppm : s : 2 H : C<u>H</u>$_2$-$C_6H_4$-
7,6ppm : q : 4 H : aromatiques
9,85 ppm : s : 1 H : C<u>H</u>O

C) Acide (E) [((n-butyl-2 oxo-5 spirocyclopentane-4 imidazolin-1-yl) méthyl)-4 phényl]-3 phényl-2 acrylique.

Cette étape est réalisée selon H.E Zimmerman et al., J. Am. Chem. Soc., 1959, 81, 2086.

On porte à reflux, pendant 35 minutes, une solution contenant 1,93 g de l'aldéhyde préparé à l'étape précédente, 850 mg d'acide phénylacétique, 6 ml d'anhydride acétique et 6 ml de triéthylamine. On refroidit le milieu réactionnel par un bain de glace puis on ajoute lentement, à 0°C, 10 ml d'acide chlorhydrique concentré, puis 100 ml d'eau. On extrait la phase aqueuse par 3 fois 50 ml de DCM, on rassemble les phases organiques, on lave par 100 ml d'eau, on sèche sur sulfate de sodium, on filtre et on concentre le filtrat. La gomme obtenue est reprise par 100 ml d'éther éthylique et on extrait 3 fois par 50 ml de soude à 10 %. On acidifie la phase alcaline à pH 3-4 par addition d'acide chlorhydrique concentré puis on extrait 3 fois par 50 ml d'acétate d'éthyle. On sèche sur sulfate de sodium, on filtre et on concentre. Le produit obtenu est purifié par chromatographie sur silice en éluant par AcOEt/toluène/AcOH (2/8/0,3 ; v/v/v). On obtient une huile (m = 850 mg) qui précipite par trituration dans l'éther.
Fc = 153°C
- RMN :
0,75 ppm : t : 3 H : $CH_3$ (nBu)
1,20 ppm : sext : 2 H : $CH_3$-C<u>H</u>$_2$-
1,40 ppm : m : 2 H : $CH_3$-$CH_2$-C<u>H</u>$_2$-
1,50-1,90 ppm : m : 8 H : cyclopentane
2,2 ppm : t : 2H : $CH_3$-$CH_2$-$CH_2$-C<u>H</u>$_2$-
4,60 ppm : s : 2 H : C<u>H</u>$_2$-$C_6H_4$
6,90-7,70 ppm : m : 10 H : aromatiques et 1 H éthylénique

EXEMPLE 7

Acide (Z) [(n-butyl-2 oxo-5 spirocyclopentane-4 imidazolin-2-yl-1) méthyl)-4 phényl]-3 phényl-2 acrylique.

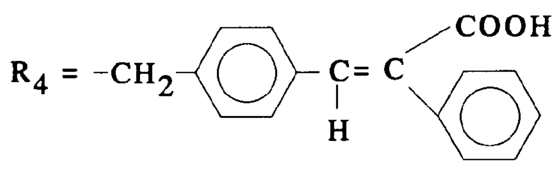

A) Acide (Z) phényl-2*p*-tolyl-3 acrylique.

On porte à reflux à 110°C pendant 22 heures un mélange contenant 25 g d'acide phénylacétique, 35 ml de méthyl-4 benzaldéhyde, 20 ml d'anhydride acétique et 20 ml de triéthylamine. On refroidit par un bain de glace, on acidifie par 40 ml d'acide chlorhydrique concentré, puis on dilue par addition d'eau. On extrait 3 fois par 300 ml d'éther éthylique, rassemble les phases éthérées, on les lave par 400 ml d'eau puis on extrait par un litre de solution de soude à 2 % (pH 14). La phase alcaline est acidifiée par de l'acide acétique jusqu'à pH 7 et on filtre le précipité qui est l'acide (E). On amène le filtrat à pH 4, on extrait par 3 fois 300 ml de DCM et on concentre à sec. L'huile obtenue précipite par addition d'un mélange éther/hexane (1/5 ; v/v). Le composé attendu (acide Z) est obtenu pur par recristallisation dans le toluène à chaud et addition d'hexane à froid.
F=86°C
m=4,3 g
RMN :
2,3 ppm : s : 3 H : $CH_3$
7,0-7,6 ppm : m : 10 H : aromatiques + H éthylénique
13,3 ppm : s : 1 H : $CO_2H$

B) Ester *tert*-butylique de l'acide (Z) phényl-2 *p*-tolyl-3 acrylique.

2 g du composé obtenu à l'étape précédente sont dissous dans 40 ml de DCM, on ajoute sous azote 800 µl de chlorure d'oxalyle et on agite sous azote, à TA, pendant 5 heures. Après évaporation à sec, on évapore à nouveau 2 fois après addition de 40 ml de toluène. Le résidu est dissous dans 40 ml de THF anhydre et l'on ajoute à froid, sous azote, 1,04 g de *tert*-butylate de potassium. Après 1 heure d'agitation à TA, sous azote, on dilue avec 50 ml d'eau puis 50 ml d'éther, on décante et on extrait la phase aqueuse 2 fois par 30 ml d'éther. Les phases éthérées sont rassemblées et lavées par une solution sodique à 2 % puis par une solution saturée de chlorure de sodium. On sèche sur sulfate de sodium, on filtre et on concentre le filtrat. L'huile obtenue est purifiée par chromatographie sur silice en éluant par un mélange DCM/hexane (1/1 ; v/v). On obtient 780 mg de produit attendu sous forme d'une huile.

- RMN :
  - 1,2 ppm : s : 9 H :(tBu)
  - 2,2 ppm : s : 3 H : $CH_3$
  - 6,9-7,4 ppm : m : 10 H : H aromatiques et H éthylénique

C) Ester *tert*-butylique de l'acide (Z) phényl-2 *p*-bromométhylphényl-3 acrylique.

On met en suspension dans 50 ml de tétrachlorure de carbone, 780 mg du composé préparé à l'étape précédente, 470 mg de NBS et 10 mg de péroxyde de benzoyle, on laisse sous agitation, sous un rayonnement U.V. pendant 2 heures en maintenant un léger reflux par chauffage. On refroidit le milieu réactionnel dans un bain de glace puis on filtre le succinimide formé. Par concentration du filtrat, on obtient 1,22 g d'huile qui est utilisée telle quelle dans l'étape suivante.

D) Ester *tert*-butylique de l'acide (Z) [((n-butyl-2 oxo-5 spirocyclopentane-4 imidazolin-2-yl-1)méthyl)-4 phényl]-3 phényl-2 acrylique.

165 mg d'hydrure de sodium à 80 % dans l'huile sont mis en suspension sous argon dans 20 ml de DMF anhydre et l'on ajoute, à 0°C, par petites fractions, 780 mg de n-butyl-2 spirocyclopentane-4 imidazoline-2 one-5. Après 20 minutes d'agitation sous argon, on ajoute 1,22 g du dérivé bromé préparé à l'étape précédente et on laisse 5 heures sous agitation, à TA, sous argon. Après concentration sous vide du milieu réactionnel, on reprend par 100 ml d'acétate d'éthyle et on lave par 50 ml d'eau, 30 ml d'une solution de $KHSO_4$-$K_2SO_4$ à 5%, 30 ml d'une solution saturée de chlorure de sodium, 30 ml d'hydrogénocarbonate de sodium puis 30 ml d'une solution saturée de chlorure de sodium. On sèche la phase organique sur sulfate de sodium, on filtre et on concentre le filtrat. L'huile obtenue est purifiée par chromatographie sur silice en éluant par un mélange DCM-Acétone (100/4; v/v). On obtient 890 mg du composé attendu pur.

E) Acide (Z) [((n-butyl-2 oxo-5 spirocyclopentane-4 imidazolin-2-yl-1) méthyl)-4 phényl]-3 phényl-2 acrylique.

890 mg du composé obtenu à l'étape précédente sont dissous dans 10 ml de DCM et 5 ml de TFA et on laisse 2 heures sous agitation à TA. Après concentration à sec du milieu réactionnel, on triture l'huile obtenue dans de l'éther puis on évapore l'éther ; en renouvellant cette opération, un précipité blanc se forme, il est filtré puis séché au dessicateur. On obtient 350 mg du composé attendu.

Fc = 158-161°C.

- RMN :
  - 0,8 ppm : t : 3 H : $CH_3$ (nBu)
  - 1,25 ppm : sext : 2 H : $CH_3$-$C\underline{H_2}$-
  - 1,50 ppm : m : 11 H : tBu et $CH_3$-$CH_2$-$C\underline{H_2}$-
  - 1,8-2,0 ppm : m : 8 H : cyclopentane
  - 2,3 ppm : t : 2 H : $CH_3$-$CH_2$-$CH_2$-$C\underline{H_2}$-
  - 4,8 ppm : s : 2 H : $C\underline{H_2}$-$C_6H_4$-
  - 6,9-7,7 ppm : m : 10 H : H aromatiques et H éthylénique

EXEMPLE 8

Acide N-phényl N-[((n-butyl-2 spirocyclopentane-4 oxo-5 imidazolin-2-yl-1) méthyl)-4 phényl] amino-acétique.

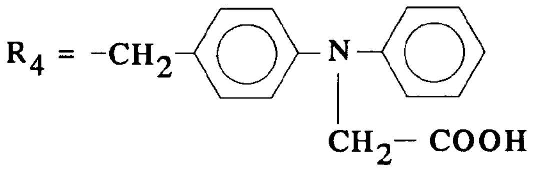

A) La N-(*p*-tolyl) aniline est préparée selon le mode opératoire décrit par C. Izard-Verchere et al., dans Chim. Therap., 1971, (5), 346-351.

B) N-phényl-N-*p*-tolyl trifluoroacétamide.

On dissout 3,66 g de l'amine préparée à l'étape A dans 50 ml de DCM, on ajoute à 0°C, 3,04 ml de triéthylamine puis, en 2 minutes, 2,96 ml d'anhydride trifluoroacétique. Après 1 heure d'agitation à TA, le milieu réactionnel est dilué par 200 ml d'éther et 200 ml d'eau. La phase organique est décantée puis lavée successivement par une solution de bicarbonate de soude demi-saturée à 0°C, une solution de sulfate-bisulfate de potassium à 5 %, et une solution saturée de chlorure de sodium. Après séchage sur sulfate de sodium, la phase organique est évaporée sous vide et l'on obtient 5,4 g du produit attendu sous forme d'une huile ; il est identifié par ses spectres RMN et IR.

C) N-*p*-bromométhylphényl, N-phényl trifluoroacétamide.

2,8 g du produit obtenu à l'étape C sont dilués dans 120 ml de tétrachlorocarbone et on ajoute 100 mg d'azobisisobutyronitrile, 100 mg de péroxyde de benzoyle et 1,96 g de NBS. Après 2 heures et demie de chauffage à reflux, le milieu réactionnel est refroidi et filtré. Le filtrat est concentré sous vide et on obtient une huile ; le produit attendu est identifié par son spectre RMN.

D) N-[((n-butyl-2 spirocyclopentane-4 oxo-5 imidazolin-2-yl-1) méthyl)-4 phényl] -N-phényl trifluoroacétamide.

A 1,15 g de chlorhydrate de n-butyl-2 spirocyclopentane-4 imidazoline-2 one-5 dans 14 ml de DMF, on ajoute, en 10 minutes, 300 mg d'hydrure de sodium à 80 % dans l'huile puis, après 15 minutes, on ajoute en 15 minutes, le produit préparé à l'étape C dissous dans 5 ml de DMF. Après 3 heures d'agitation à TA, le milieu réactionnel est dilué dans 100 ml d'acétate d'éthyle et 20 ml d'eau ; la phase organique est décantée, lavée à l'eau puis par une solution saturée de chlorure de sodium, séchée sur sulfate de sodium puis concentrée sous vide. Le résidu est chromatographié sur silice en éluant par un mélange AcOEt/toluène (2/3 ; v/v). On obtient 1,5 g du produit attendu sous forme de cire, il est identifié par ses spectres IR et RMN.

E) (phénylamino-4-benzyl)-1-n-butyl-2-spirocyclopentane-4-imidazoline-2-one 5.

Le produit obtenu à l'étape précédente est dilué dans 15 ml de méthanol et on ajoute 3,5 ml de soude 2N. Après 50 minutes d'agitation, le milieu réactionnel est dilué par 100 ml d'acétate d'éthyle et 20 ml d'eau ; la phase organique est décantée, lavée par une solution saturée de chlorure de sodium, puis concentrée sous vide. On obtient 1,15 g du produit attendu sous forme d'une cire. Il est identifié par ses spectres IR et RMN.

F) Ester *tert*-butylique de l'acide N-phényl N-[((n-butyl-2 spirocyclopentane-4 oxo-5 imidazolin-2-yl-1) méthyl)-4 phényl] amino acétique.

1,12 g du produit obtenu à l'étape précédente sont dissous dans 10 ml de DMF et l'on ajoute 100 mg d'hydrure de sodium à 80 % dans l'huile puis après 15 minutes, on additionne 643 mg de bromoacétate de tertiobutyle dilués dans 1 ml de DMF. On rajoute les mêmes quantités de bromoacétate d'éthyle et d'hydrure de sodium, 3 fois, après 2 heures, 6 heures et 22 heures. Après 28 heures, le milieu réactionnel est dilué par 100 ml d'acétate d'éthyle et 20 ml d'eau ; la phase organique est décantée puis lavée par une solution saturée de

chlorure de sodium, séchée sur sulfate de sodium et concentrée sous vide. Le résidu est chromatographié sur silice en éluant avec un mélange chloroforme/hexane (4/6 ; v/v) contenant 4 % de méthanol. Le produit attendu est identifié par ses spectres IR et RMN.

- RMN :
  - 4,70 ppm : s : 2 H : N-C$\underline{H}_2$-$C_6H_4$
  - 4,45 ppm : s : 2 H : N-C$\underline{H}_2$-$CO_2$tBu

G) Acide N-phényl N-[((n-butyl-2 spirocyclopentane-4 oxo-5 imidazolin-2-yl-1) méthyl)-4 phényl] amino-acétique.

Le produit obtenu à l'étape précédente est traité pendant 10 minutes par un mélange de 15 ml de DCM et 20 ml de TFA. Après concentration sous vide, le résidu est repris dans 40 ml d'eau et 20 ml d'éther et amené à pH 10 par addition de soude 2N. La phase aqueuse est décantée et amenée à pH 5 par addition d'acide chlorhydrique 2N, en présence d'acétate d'éthyle. La phase organique est lavée par une solution saturée de chlorure de sodium, séchée sur sulfate de sodium puis concentrée sous vide. Le résidu est chromatographié sur silice en éluant par un mélange AcOEt/toluène (2/1 ; v/v) contenant 0,3 % d'acide acétique. On obtient 400 mg du produit attendu.

Fc = 140-142°C

- spectre de masse : $MH^+$ = 434
- RMN :
  - 6,00 -7,35 ppm : m : 9 H : aromatiques
  - 4,65 ppm : s : 2 H : N-C$\underline{H}_2$-$C_6H_4$-
  - 4,45 ppm : s : 2 H : N-C$\underline{H}_2$-$CO_2H$
  - 2,35 ppm : t : 2 H : $CH_3$-$CH_2$-$CH_2$-C$\underline{H}_2$
  - 1,60-2,00 ppm : m : 8 H : cyclopentane
  - 1,50 ppm : quint : 2 H : $CH_3$-$CH_2$-C$\underline{H}_2$-$CH_2$-
  - 1,30 ppm : sext : 2 H : $CH_3$-C$\underline{H}_2$-$CH_2$-$CH_2$-

EXEMPLE 9

Acide dichloro-2,2 [((n-butyl-2 oxo-5 spirocyclopentane-4 imidazolin-2-yl-1) méthyl)-4 phényl]-2 acétique.

$R_4$ = 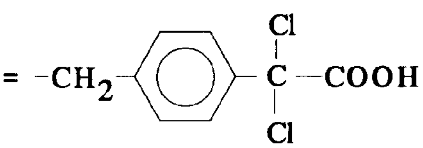

A) On prépare le *p*-tolyl dichloroacétate d'éthyle selon le mode opératoire décrit par R.N. Mc Donald et R.C. Cousins dans J. Org. Chem., 1980, 45, 2976-2984.

B) Le *p*-bromométhyl dichloroacétate d'éthyle est obtenu par action du péroxyde de benzoyle et du NBS sur le composé préparé à l'étape A, selon le mode opératoire décrit dans les exemples précédents.

C) L'ester éthylique de l'acide dichloro-2,2 [((n-butyl-2 oxo-5 spirocyclopentane-4 imidazolin-2-yl-1)-4 phényl]-2 acétique est préparé par action du dérivé bromé obtenu à l'étape précédente sur le chlorhydrate de n-butyl-2 spirocyclopentane-4 imidazoline-2 one-5, selon le mode opératoire décrit dans les exemples précédents. Le composé obtenu est caractérisé par ses spectres IR et RMN.

- IR ($CHCl_3$) :
  - 1710-1715 $cm^{-1}$ : C = O : ester et imidazolinone
  - 1630 $cm^{-1}$ : C = N : imidazolinone

D) Acide dichloro-2,2 [((n-butyl-2 oxo-5 spirocyclopentane-4 imidazolin-2-yl-1) méthyl)-4 phényl]-2 acétique.

0,95 g de l'ester obtenu à l'étape C sont dissous dans 4 ml de méthanol et 4 ml de dioxanne. On ajoute 1,1 ml de soude 2N et laisse sous agitation 40 minutes à TA. Le milieu réactionnel est dilué par 60 ml d'AcOEt et 10 ml d'eau et on ajoute de l'acide chlorhydrique 1N pour l'amener à pH 4,8. Le précipité formé est filtré, lavé à l'eau, puis à l'éther et séché sous vide. On obtient 290 mg du produit attendu.

Fc = 185-187°C

Une autre partie du produit attendu est obtenu à partir du milieu de neutralisation : celui-ci est décanté, la phase organique est lavée par de l'eau puis par une solution saturée de chlorure de sodium, séchée sur sulfate de sodium et concentrée sous vide. On obtient 290 mg du produit attendu.

Fc = 178-180°C.

Les spectres IR et RMN sont identiques pour les produits obtenus.

- RMN :
  - 7,60-7,15 ppm : système AA'BB' : 4 H : aromatiques
  - 4,65 ppm : s : 2 H : $N\text{-}C\underline{H}_2\text{-}C_6H_4$
  - 2,30 ppm : t : 2 H : $CH_3\text{-}CH_2\text{-}CH_2\text{-}C\underline{H}_2\text{-}$
  - 1,50-1,90 ppm : m : 8 H : cyclopentane
  - 1,40 ppm : quint : 2 H : $CH_3\text{-}CH_2\text{-}C\underline{H}_2\text{-}CH_2$
  - 1,15 ppm : sext : 2 H : $CH_3\text{-}C\underline{H}_2\text{-}CH_2\text{-}CH_2$
  - 0,70 ppm : t : 3 H : $C\underline{H}_3\text{-}CH_2\text{-}CH_2\text{-}CH_2\text{-}$

EXEMPLE 10

n-butyl-2 spirocyclopentane-4 [((tétrazol-5-yl)-2-isopropyl)-4 benzyl]-1 imidazoline-2 one-5.

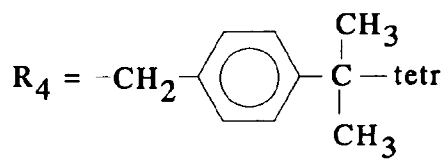

A) (p-tolyl)-2-cyano-2-propane.

Ce composé est préparé selon J. Org. Chem. 1969, 34, 227.

On fait barboter du bromométhane dans 50 ml de DMSO, pendant 15 minutes et l'on ajoute goutte à goutte 6,56 g de cyanure de méthyl-4 benzyle et 16 g de soude à 50% dans l'eau. On maintient l'arrivée de gaz pendant 1 heure puis on ajoute 125 ml d'eau et on extrait par de l'éther, on lave par une solution de carbonate de sodium puis par une solution saturée de chlorure de sodium, on sèche puis on évapore sous vide. Le liquide obtenu distille à 130°C. On recueille 5 g du produit attendu.

B) p-tolyl-2-(tétrazol-5-yl)-2-propane.

On porte à reflux pendant 96 heures dans 20 ml de xylène, 2,1 g du composé préparé à l'étape précédente et 2,1 g d'azide de tributylétain. Après évaporation des solvants, on reprend par de l'eau, on extrait 2 fois par AcOEt, on lave la phase organique par de l'eau puis on extrait la phase organique 2 fois par de la soude 2N. On lave la phase aqueuse basique par de l'éther, on la refroidit puis on l'acidifie par addition d'acide chlorhydrique 5N jusqu'à pH 3-4. Par extraction avec AcOEt, on obtient 1,57 g du produit attendu qui est caractérisé par son spectre RMN.

C) p-tolyl-2-[(trityl-1)-tétrazol-5-yl]-2-propane.

On porte à reflux pendant 5 heures, dans du DCM, 1,5 g du composé obtenu à l'étape précédente, 2,29 g de chlorure de trityle et 1,3 ml de triéthylamine. Après évaporation des solvants, on reprend par AcOEt, on lave successivement par une solution d'hydrogénosulfate de potassium à 3 %, par de la soude 1N, de l'eau et une solution de chlorure de sodium saturée. Le produit obtenu est chromatographié sur une colonne d'alumine en éluant par un mélange hexane/DCM (70/30 ; v/v). On obtient 1,92 g du produit attendu caractérisé par ses spectres IR et RMN.

D) (p-bromométhylphényl)-2-[(trityl-1)-tétrazol-5-yl]-2-propane.

On porte à reflux pendant 3 heures dans 30 ml de tétrachlorocarbone, un mélange contenant 1,92 g du composé préparé à l'étape précédente, 846 mg de NBS et 100 mg de peroxyde de benzoyle. Lorsque le milieu réactionnel est revenu à TA, on filtre le succinimide formé, on évapore le tétrachlorure, on reprend par de l'éther,

on lave à l'eau puis par une solution saturée de chlorure de sodium. On obtient 2,06 g du composé attendu.

E) n-butyl-2 spirocyclopentane-4 [(((trityl-1) tétrazol-5-yl)-2-isopropyl)-4 benzyl]-1 imidazoline-2 one-5.

On place sous azote 10 ml de DMF contenant 50 mg d'hydrure de sodium à 80 % dans l'huile.On ajoute goutte à goutte 194 mg de n-butyl-2 spirocyclopentane-4 imidazoline-2 one-5 dans 10 ml de DMF puis après 30 minutes à TA, 748 mg du dérivé bromé préparé à l'étape D dans 10 ml de DMF. Après 2 heures et demie, sous agitation, à TA, on évapore le milieu, on reprend par AcOEt et de l'eau, on décante puis on sèche sur sulfate de sodium. Le produit obtenu est chromatographié sur alumine en éluant par un mélange hexane/AcOEt (90/10 ; v/v). On obtient 390 mg du produit attendu.

F) n-butyl-2 spirocyclopentane-4 [((tétrazol-5-yl)-2-isopropyl)-4 benzyl]-1 imidazoline-2 one-5.

On laisse sous agitation pendant 4 heures 380 mg du composé obtenu à l'étape précédente dans 4 ml de méthanol et 0,3 ml d'acide chlorhydrique 4N, puis on chauffe à 30°C pendant 18 heures. Après évaporation, on reprend par de l'eau, on alcalinise par de la soude, lave par de l'éther, du toluène, à nouveau de l'éther puis on acidifie la phase aqueuse à pH 5 par addition d'acide chlorhydrique 1N et l'on extrait à l'acétate d'éthyle. On obtient 150 mg du produit attendu.
Fc = 95°C.
- RMN :

0,85 ppm : t : 3 H : $CH_3$ (tBu)
1,2 ppm : sext : 2 H : $CH_3$-$C\underline{H}_2$-
1,55 ppm : quint : 2 H : $CH_3$-$CH_2$-$C\underline{H}_2$-
1,65-2,1 ppm : m : 14 H : cyclopentane + 2$CH_3$ (diméthyl)
2,45 ppm : t : 2 H : $CH_3$-$CH_2$-$CH_2$$C\underline{H}_2$-
4,75 ppm : s : 2 H : N-$C\underline{H}_2$-$C_6H_4$-
7,25 ppm : m : 4 H : aromatiques

EXEMPLE 11

n-butyl-2 spirocyclopentane-4 [((tétrazol-5-yl)-2-isopropyl)-3 benzyl]-1 imidazoline-2 one-5.

$R_4$ =
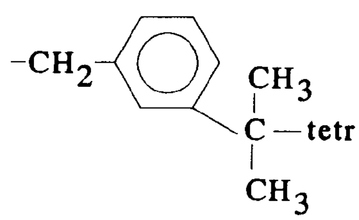

Ce composé est préparé selon le mode opératoire décrit à l'exemple précédent en utilisant comme produit de départ le cyanure de *m*-méthylbenzyle.
Fc = 55°C.
- RMN :

0,7 ppm : t : 3 H : $CH_3$ (nBu)
1,2 ppm : sext : 2 H : $CH_3$-$C\underline{H}_2$-
1,35 pm : quint : 2 H : $CH_3$-$CH_2$-$C\underline{H}_2$-
1,5-2 ppm : m : 14 H : cyclopentane + 2$CH_3$ (diméthyl)
2,2 ppm : t : 2H : $CH_3$-$CH_2$-$CH_2$-$C\underline{H}_2$-
4,6ppm : s : 2 H : N-$C\underline{H}_2$-$C_6H_4$-
6,7-7,4 ppm : m : 4 H : aromatiques

EXEMPLE 12

n-butyl-2 spirocyclopentane-4 [(tétrazol-5-yl)-1 propyl-1 butyl)-4 benzyl]-1 imidazoline-2 one-5.

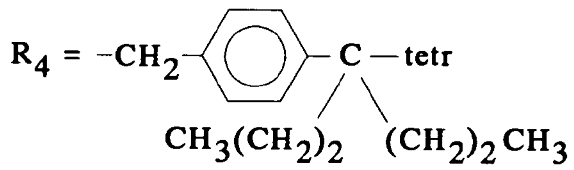

Ce composé est préparé selon le mode opératoire décrit à l'exemple 10. Il est caractérisé par son point de fusion.

Fc = 50°C.

A la première étape, le cyanure de dipropyl-1,1 *p*-méthylbenzyle est obtenu par action du bromopropane sur le cyanure de *p*-méthylbenzyle.

- RMN :

0,5-2,5 ppm : m : 31 H :(nBu) cyclopentane $2C_3\underline{H}_7$ (dipropyl)
4,75 ppm : s : 2 H : N-$C\underline{H}_2$-$C_6H_4$-
7,2 ppm : m : 4 H : aromatiques

EXEMPLE 13

n-butyl-2 spirocyclopentane-4 [(tétrazol-5-yl)-4 benzyl]-1 imidazoline-2 one-5.

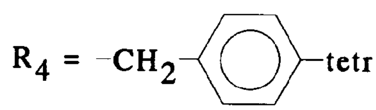

A) p-(tétrazol-5-yl)-toluène.

On chauffe à 120°C dans le DMF un mélange contenant 2,34 g de cyanure de *p*-tolyle, 1,45 g d'azidure de sodium et 1,28 g de chlorure d'ammonium. On évapore le solvant, reprend par de l'eau, extrait 2 fois par AcOEt, lave la phase organique par de l'eau, extrait la phase organique 2 fois par de la soude 2N puis lave la phase alcaline par de l'éther. On refroidit la phase alcaline dans un mélange eau-glace puis on l'acidifie jusqu'à pH 3 par addition d'acide chlorhydrique 5N. On filtre, lave à l'eau, et sèche sous vide en présence de potasse. On obtient 2,5 g du produit attendu caractérisé par son spectre RMN.

B) p-[(trityl-1)-tétrazol-5-yl]-toluène.

On porte à reflux pendant 16 heures dans le DCM 2,5 g du composé obtenu à l'étape précédente, 5,22 g de chlorure de trityle et 3,2 ml de triéthylamine. On évapore le solvant, reprend par AcOEt, lave par une solution à 3 % d'hydrogénosulfate de potassium, par de la soude 1N, de l'eau, puis par une solution saturée de chlorure de sodium. Le produit obtenu est chromatographié sur alumine en éluant par un mélange hexane/DCM (80/20 ; v/v). On obtient 2,58 g du produit attendu.

C) p-[(trityl-1)-tétrazol-5-yl]-bromotoluène.

On obtient 2,58 g de composé par traitement de 2,5 g du composé obtenu à l'étape précédente avec du NBS et du péroxyde de benzoyle selon la méthode habituelle.

D) n-butyl-2 spirocyclopentane-4 [(trityl-1 tétrazol-5-yl)-4 benzyl]-1 imidazoline-2 one-5.

475 mg de ce composé est préparé par la méthode habituelle en traitant 775 mg du composé bromé obtenu à l'étape précédente par 194 mg de n-butyl-2 spirocyclopentane-4 imidazoline-2 one-5.

E) n-butyl-2 spirocyclopentane-4 [(tétrazol-5-yl)-4 benzyl]-1 imidazoline-2 one-5.

Le composé attendu est préparé à partir de celui obtenu à l'étape précédente en opérant comme à l'exemple 10 étape F.
Fc = 90°C
- RMN :
  - 0,7 ppm : t : 3 H : $CH_3$ (nBu)
  - 1,25 ppm : sext : 2 H : $CH_3$-$\underline{CH_2}$-
  - 1,45 ppm : quint : 2 H : $CH_3$-$CH_2$-$\underline{CH_2}$-
  - 1,55-2,0 ppm : m : 8 H : cyclopentane
  - 2,3 ppm : t : 2 H : $CH_3$-$CH_2$-$CH_2$-$\underline{CH_2}$-
  - 4,7 ppm : s : 2 H : N-$\underline{CH_2}$-$C_6H_4$-
  - 7,2-8,1 ppm : m : 4 H : aromatiques

EXEMPLE 14

Acide [((n-butyl-2 oxo-5 spirocyclopentane-4 imidazolin-2-yl-1) méthyl-4 phényl)-5 tétrazol-2-yl] acétique.

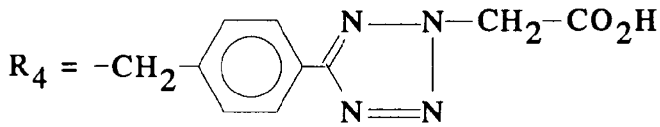

On laisse sous agitation à TA, sous atmosphère d'azote, 250 mg d'hydrure de sodium à 80 % dans l'huile et 10 ml de DMF et on ajoute goutte à goutte 1,49 g du composé préparé à l'exemple précédent (exemple 13) dissous dans 20 ml de DMF, on laisse 30 minutes sous agitation à TA. On ajoute ensuite 925 mg de bromacétate de *tert*-butyle dans 10 ml de DMF. Après 1 heure sous agitation à TA, on évapore le DMF, reprend le résidu par un mélange AcOEt-eau et extrait avec AcOEt. La phase aqueuse est acidifiée jusqu'à pH 5 par addition d'acide chlorhydrique 1N, puis on extrait par AcOEt. On sèche sur sulfate de sodium, filtre et évapore. On chromatographie le résidu sur silice en éluant par un mélange DCM/MeOH/AcOH (95/5/1 ; v/v/v). On obtient 650 mg du produit attendu.
Fc = 196-198°C.
- RMN :
  - 0,65 ppm : t : 3 H : $CH_3$
  - 1,15 ppm : sext : 2 H : -$\underline{CH_2}$-$CH_3$
  - 1,35 ppm : quint : 2 H : -$\underline{CH_2}$-$CH_2$-$CH_3$
  - 1,5-1,95 ppm : m : 8 H : cyclopentane
  - 2,25 ppm : t : 2H : $CH_3$-$CH_2$-$CH_2$-$\underline{CH_2}$
  - 4,65 ppm : s : 2 H : N-$\underline{CH_2}$-$C_6H_4$-
  - 5,65 ppm : s : 2 H : -N-$\underline{CH_2}$-COOH
  - 7,1-8,1 ppm : m : 4 H: aromatiques

EXEMPLE 15

n-butyl-2 spirocyclopentane-4 [((tétrazol-5-yl) méthyl)-4 benzyl]-1 imidazoline-2 one-5.

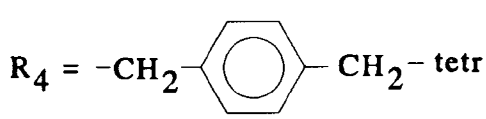

Ce composé est préparé selon le mode opératoire décrit à l'exemple 10. Il est caractérisé par son point de fusion.
Fc = 120-121°C.
- RMN :
  - 0,9 ppm : t : 3 H : $CH_3$ (tBu)
  - 1,4 ppm : sext : 2 H : $CH_3$-$\underline{CH_2}$-

1,6 ppm : m : 2 H : $CH_3$-$CH_2$-$C\underline{H}_2$-
1,8-2,3 ppm : m : 8 H : cyclopentane
2,9 ppm : t : 2 H : -$C\underline{H}_2$-$CH_2$-$CH_2$-$CH_3$
4,35 ppm : s : 2 H : -$C\underline{H}_2$-$C_6H_4$-
5 ppm : s : 2 H : -N-$C\underline{H}_2$-$C_6H_4$-
7,35 ppm : m : 4 H : aromatiques

EXEMPLE 16

n-butyl-2 spirocyclopentane-4 [((tétrazol-5-yl)-1cyclohexyl)-4 benzyl]-1 imidazoline-2 one-5.

$R_4$ = 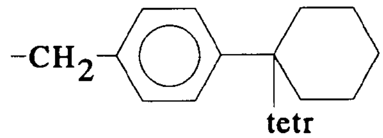

A) cyano-1 *p*-tolyl-1 cyclohexane.

Ce composé est préparé selon le mode opératoire décrit dans J. Org. Chem., 1971, 36 (9), 1308.
On place, sous argon, 2,7 g d'hydrure de sodium à 80 % dans l'huile dans 40 ml de DMSO et l'on ajoute goutte à goutte un mélange de 5,24 g de cyanure de *p*-méthylbenzyle et 10,12 g de dibromo-1,5 pentane dans 20 ml d'éther en maintenant la température entre 25 et 35°C par un bain d'eau froide. Après la fin de la réaction exothermique, on laisse sous agitation 1 heure à 25°C puis on refroidit par un bain de glace. On ajoute 3 ml d'alcool isopropylique et 30 ml d'eau. Après décantation, on extrait par l'éther et AcOEt. Les phases organiques sont réunies et lavées 3 fois à l'eau puis par une solution saturée de chlorure de sodium, ensuite on sèche sur sulfate de sodium et on évapore.
Le produit est ensuite distillé sous pression réduite (0,2 mm Hg) : Eb = 98-102°C. On obtient 4,2 g du produit pur.

B) n-butyl-2 spirocyclopentane-4 [((tétrazol-5-yl)-1 cyclohexyl)-4 benzyl]-1 imidazoline-2 one-5.

On opère ensuite selon le mode opératoire habituel en suivant les différentes étapes décrites à l'exemple 10 pour préparer le produit attendu.
Fc = 80-100°C.
- RMN :

0,6 ppm : t : 3 H : $CH_3$ (nBu)
0,9-2,6 ppm : m : 24 H : cyclopentane + $3CH_2$ (nBu) + cyclohexyle
4,5 ppm : s : 2 H : N-$C\underline{H}_2$-$C_6H_4$-
6,8-7,2 ppm : m : 4 H : aromatiques
15 ppm : s.e. : 1 H : N$\underline{H}$ (tétrazolyl)

EXEMPLE 17

Acide [(n-butyl-2 spirocyclopentane-4 oxo-5 imidazol-2-yl-1) méthyl-4-phényl]-2 méthyl-2 propionique.

$R_4$ = 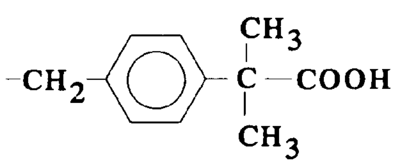

A) (p-tolyl)-2-cyano-2-propane.

La préparation de ce composé est semblable à celle décrite à l'exemple 10.

B) Acide *p*-tolyl-2 méthyl-2 propionique.

On porte à reflux pendant 16 heures 24,4 g du composé préparé à l'étape précédente dans 1,44 l de diéthyléthylèneglycol et 1,88 l de potasse à 40 % dans l'eau. Le milieu réactionnel est dilué avec de l'eau puis extrait à l'éther. On acidifie la phase aqueuse avec de l'acide chlorhydrique concentré, extrait à l'acétate d'éthyle, lave à l'eau, sèche sur sulfate de sodium et concentre. Le produit obtenu est chromatographié sur silice en éluant par un mélange AcOEt/hexane/acide acétique (20/80/1 ; v/v/v). On recueille 22 g du produit attendu.

C) Ester méthylique de l'acide *p*-tolyl-2 méthyl-2 propionique.

On porte à reflux pendant 2 heures 4,3 g de l'acide préparé à l'étape précédente et 10 ml de chlorure de thionyle. On évapore le chlorure de thionyle en excès, on reprend plusieurs fois par du toluène puis on évapore le toluène. On ajoute goutte à goutte à TA 20 ml de méthanol puis 1,9 ml de pyridine et on porte 1 heure à reflux. On évapore le milieu, reprend par de l'acétate d'éthyle et de l'eau, lave par de l'acide chlorhydrique 1N, de l'eau et par une solution saturée de chlorure de sodium puis on sèche la phase organique sur sulfate de sodium. Le produit obtenu est chromatographié sur silice en éluant par le mélange acétate d'éthyle/heptane (10/90 ; v/v). On recueille 3,6 g du produit attendu.

D) Ester méthylique de l'acide (*p*-bromométhylphényl)-2 méthyl-2 propionique.

Le composé préparé à l'étape précédente est traité par le NBS et le péroxyde de benzoyle selon le mode opératoire habituel.

E) Ester méthylique de l'acide [(n-butyl-2 spirocyclopentane-4 oxo-5 imidazol-2-yl-1) méthyl-4-phényl]-2 méthyl-2 propionique.

Le composé préparé à l'étape précédente est traité par le n-butyl-2 spirocyclopentane-4 imidazoline-2 one-5 selon le mode opératoire habituel.

F) Acide [(n-butyl-2 spirocyclopentane-4 oxo-5 imidazol-2-yl-1) méthyl-4-phényl]-2 méthyl-2 propionique.

500 mg de l'ester obtenu à l'étape précédente sont portés à reflux pendant 3 heures dans 5 ml de dioxanne, 1,3 ml d'eau et 112 mg de soude. On évapore le milieu, reprend par de l'eau, extrait à l'éther puis on acidifie la phase aqueuse à pH 5. On extrait à l'acétate d'éthyle, sèche sur sulfate de sodium et évapore.
Le produit obtenu est purifié sur colonne de silice en éluant par un mélange hexane/AcOEt (5/5 ; v/v). On recueille 300 mg du produit attendu.
Fc = 150°C
- RMN :
  - 0,85 ppm : t : 3 H : $CH_3$ (nBu)
  - 1,1-2,1 ppm : m : 18 H : $CH_3$-$C\underline{H_2}$-$C\underline{H_2}$- + 2$CH_3$ (diméthyl) + cyclopentane
  - 2,35 ppm : t : 2 H : -$C\underline{H_2}$-$CH_2$-$CH_2$-$CH_3$
  - 4,7 ppm : s : 2 H : N-$C\underline{H_2}$-$C_6H_4$-
  - 7-7,6 ppm : m : 4 H : aromatiques
  - 12,4 ppm : s.e. : 1 H : COOH-

EXEMPLE 18

n-butyl-2 spirocyclopentane-4 [((tétrazol-5-yl)-méthyl-2-isopropyl)-4 benzyl]-1 imidazoline-2 one-5.

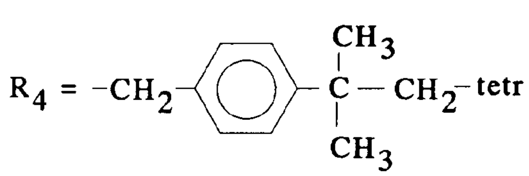

A) Ester éthylique de l'acide cyano-2*p*-tolyl-3 butène-2 carboxylique.

Ce composé est préparé selon Tetrahedron, 1959, 7; 236-240.

Dans 50 ml de benzène anhydre, on mélange 22,8 g d*p*-méthylacétophénone, 19,2 g de cyanacétate d'éthyle, 2,62 g d'acétate d'ammonium et 8,17 g d'acide acétique, et on porte à reflux pendant 20 heures dans un ballon équipé d'un séparateur d'eau. On lave la phase organique avec de l'eau, sèche sur sulfate de sodium, filtre et évapore. Le produit attendu est obtenu par distillation sous pression réduite :

Eb = 136°C sous 0,6 mm Hg

m = 22,1 g

B) Ester éthylique de l'acide cyano-2 méthyl-3 *p*-tolyl-3 butyrique.

Ce composé est préparé d'après J. Am. Chem. Soc., 1957, 79, 4487.

On place dans un ballon 39,3 ml de solution 3M de chlorure de méthylmagnésium dans le THF, 692 mg d'iodure de cuivre et on ajoute goutte à goutte 20, 8 g du nitrile préparé à l'étape précédente dans 50 ml d'éther. Après 4 heures de chauffage à reflux, on hydrolyse par une solution de chlorure d'ammonium saturée puis on extrait à l'éther, sèche les phases organiques sur sulfate de sodium et évapore. Le produit obtenu est purifié par chromatographie sur silice en éluant par un mélange AcOEt/hexane (9/1 ; v/v). On recueille 10,8 g du produit attendu.

C) Acide cyano-2 méthyl-3 *p*-tolyl-3 butyrique.

On porte à reflux pendant 3 heures 10,8 g du composé préparé à l'étape précédente dans 175 ml de dioxanne et 45 ml d'eau contenant 3,94 g de soude. Après évaporation, on reprend par de l'eau, extrait à l'éther et acidifie la phase aqueuse à pH 5. On extrait à l'éther, sèche sur sulfate de sodium et évapore. On obtient 9 g du produit attendu.

D) méthyl-3 *p*-tolyl-3 butyronitrile.

On opère selon la demande de brevet japonais 78-124248. On porte à reflux pendant 5 heures 9 g du composé obtenu à l'étape précédente dans 50 ml de pyridine. On évapore, reprend par de l'acétate d'éthyle, lave à l'eau, sèche sur sulfate de sodium et concentre. Le produit obtenu est chromatographié sur silice en éluant par le mélange hexane-AcOEt (90/10 ; v/v). On recueille 3,84 g du produit attendu.

E) n-butyl-2 spirocyclopentane-4 [((tétrazol-5-yl)-méthyl-2-isopropyl)-4 benzyl]-1 imidazoline-2 one-5.

On opère ensuite selon les méthodes habituelles pour préparer le composé attendu caractérisé par son spectre RMN.

- RMN :

0,85 ppm : t : 3 H : $C\underline{H}_3$ (nBu)

1,1-1,6 ppm : m : 10 H : $CH_3$-$C\underline{H}_2$-$C\underline{H}_2$- + 2$CH_3$ (diméthyl)

1,6-2,1 ppm : m : 8 H : cyclopentane

2,35 ppm : t : 2 H : $CH_3$-$CH_2$-$CH_2$-$C\underline{H}_2$

3,25 ppm : s : 2 H : $C\underline{H}_2$-tétrazole

4,7 ppm : s : 2 H : N-$C\underline{H}_2$-$C_6H_4$-

7-7,5 ppm : m : 4 H : aromatiques.

EXEMPLES 19 ET 20

n-butyl-2 [(diméthyl-3,3 (tétrazol-5-yl)-1 buten-1-yl-1)-4 benzyl]-1 spirocyclopentane-4 imidazoline-2 one-5, 2 isomères.

$R_4$ = 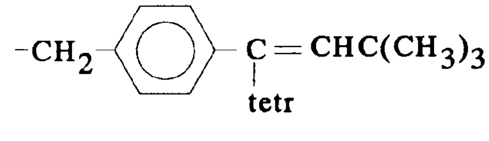

A) Diméthyl-4,4 *p*-tolyl-2 penten-2 nitrile.

On place sous argon 7 ml de LDA (1,5 M dans le cyclohexane) et 15 ml de THF anhydre, on refroidit à -70°C et on ajoute goutte à goutte 1,31 g de cyanure de *p*-méthyl benzyle dans 10 ml de THF ; après 30 minutes sous agitation, on ajoute goutte à goutte 0,95 g d'aldéhyde pivaloique dissous dans 10 ml de THF, on laisse sous agitation 1 heure à -70°C puis 1 heure à TA. On verse sur un mélange glace/acide chlorhydrique, on extrait par AcOEt puis on lave par une solution saturée de chlorure de sodium. Le produit obtenu est chromatographié sur silice en éluant par un mélange DCM/hexane (2/8 ; v/v). On recueille 1,3 g du produit attendu.

B) diméthyl-3,3 (tétrazol-5-yl)-1 *p*-tolyl-1 butène-1.

On chauffe à reflux pendant 90 heures 1,27 g du nitrile préparé à l'étape précédente et 3,2 g d'azide de tributylétain dans 20 ml de xylène. Après retour à TA, on extrait la phase organique par de la soude 1N, lave à l'éther, acidifie la phase aqueuse jusqu'à pH 2, extrait par AcOEt, sèche et évapore. On obtient 1,2 g du produit attendu.

C) diméthyl-3,3 (trityl-1 tétrazol-5-yl)-1 *p*-tolyl-1 butène-1.

On porte à reflux pendant 3 heures 1,2 g du produit obtenu à l'étape précédente dans 25 ml de DCM contenant 1,52 g de chlorure de trityle et 1 ml de triéthylamine. On évapore, reprend par de l'acétate d'éthyle, lave à l'eau puis par une solution à 3 % d'hydrogénosulfate de potassium, sèche sur sulfate de sodium et évapore. On obtient 2,6 g du produit attendu qui cristallise.

D) *p*-bromométhylphényl-1 diméthyl-3,3 (trityl-1 tétrazol-5-yl)-1 butène-1.

On porte à reflux pendant 4 heures 2,6 g du produit obtenu à l'étape précédente et 906 mg de NBS dans 40 ml de tétrachlorocarbone contenant 50 mg de péroxyde de benzoyle. Après retour à TA on filtre et évapore pour obtenir le produit brut attendu.

E) n-butyl-2 [(diméthyl-3,3 (trityl-1 tétrazol-5-yl)-1 buten-1-yl-1)-4 benzyl]-1 spirocyclopentane-4 imidazoline-2 one-5.

On place sous argon 370 mg d'hydrure de sodium à 80 % dans l'huile et 10 ml de DMF et l'on ajoute goutte à goutte 680 mg de n-butyl-2 spirocyclopentane-4 imidazoline-2 one-5 dans 10 ml de DMF; on ajoute le composé bromé obtenu à l'étape précédente dissous dans 20 ml de DMF et on laisse 1 heure à TA. On évapore, reprend par AcOEt et de l'eau, sèche la phase organique sur sulfate de sodium, filtre et évapore. Le produit brut obtenu est chromatographié sur silice en éluant par un mélange hexane-AcOEt (7/3 ; v/v). On obtient 2 isomères du produit attendu dont les configurations sont déterminées par effet N.O.E.

composé a : 550 mg : isomère Z

composé b : 650 mg : isomère E

F) n-butyl-2 [(diméthyl-3,3 (tétrazol-5-yl)-1 buten-1-yl-1)-4 benzyl]-1 spirocyclopentane-4 imidazoline-2 one-5, isomère (Z).

490 mg du composé a), obtenu ci-dessus, sont placés dans 10 ml de THF, on ajoute quelques gouttes d'acide chlorhydrique 3N pour atteindre pH 2 et on laisse 2 heures sous agitation à TA. On évapore, reprend par de l'éther, une solution de soude 1N puis on extrait par AcOEt. On acidifie la phase aqueuse à pH 5 et extrait par AcOEt. On sèche sur sulfate de sodium, filtre et évapore. Le produit brut est élué sur une colonne de silice par le mélange DCM/méthanol (95/5 ; v/v). On obtient m = 290 mg.

Fc = 85-90°C.

- RMN :

0,8 ppm : t : 3 H : $CH_3$ (nBu)
0,9 ppm : s : 9 H : (tBu)
1,25 ppm : sext : 2 H : $CH_3$-C$\underline{H_2}$-
1,5 ppm : quint : 2H : $CH_3$-$CH_2$-C$\underline{H_2}$-
1,6-2 ppm : m : 8 H : cyclopentane
2,3 ppm : t : 2 H : $CH_3$-$CH_2$-$CH_2$-C$\underline{H_2}$-
4,75 ppm : s : 2 H : N-C$\underline{H_2}$-$C_6H_4$-

6,8 ppm : s : 1 H : = C - H
7,1-7,3 ppm : m : 4 H : aromatiques

G) n-butyl-2 [(diméthyl-3,3 (tétrazol-5-yl)-1 buten-1-yl-1)-4 benzyl]-1 spirocyclopentane-4 imidazoline-2 one-5, isomère (E).

510 mg du composé b obtenu à l'étape E, sont placés dans 10 ml de méthanol et 10 ml de THF, on ajoute de l'acide chlorhydrique 2N pour atteindre pH 2 et on laisse sous agitation pendant 5 heures. Le produit obtenu est traité, comme à l'étape F puis chromatographié sur silice en éluant par le mélange DCM/méthanol (97/3 ; v/v). On obtient m = 235 mg.

Fc = 160-162°C.
- RMN
0,7 ppm : t : 3 H : $CH_3$ (nBu)
0,8 ppm : s : 9 H : (tBu)
1,-1,9 ppm : m : 12 H : $CH_3$-C$\underline{H_2}$-C$\underline{H_2}$- + cyclopentane
2,2 ppm : t : 2 H : $CH_3$-$CH_2$-$CH_2$-C$\underline{H_2}$-
4,55 ppm : s : 2 H : N-C$\underline{H_2}$-$C_6H_4$-
6,45 ppm : s : 1 H : = C$\underline{H}$
7 ppm : s : 4 H : aromatiques

EXEMPLE 21

(Z) n-butyl-2 [(diméthyl-3,3-(tétrazol-5-yl)-2 buten-1-yl-1)-4 benzyl]-1 spirocyclopentane-4 imidazoline-2 one-5.

$R_4$ =
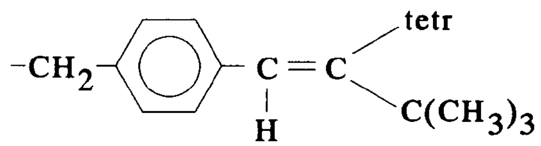

A) (bis méthylthio-2,2 cyano-1 éthène) phosphonate de diéthyle.

La préparation de ce composé est décrite par E. Schaumann et al. dans Liebigs Ann. Chem., 1979, 1715-1733.

B) (cyano-1 diméthyl-2,2 propane) phosphonate de diéthyle.

La préparation de composé est décrite par E. Schaumann et al. dans Synthesis, 1983, 449-450.

C) (Z) *p*-tolyl-1 cyano-2 diméthyl-3,3 butène-1.

Sous azote, on mélange 1,6 g du composé préparé à l'étape précédente dans 10 ml de THF et une suspension de 226 mg d'hydrure de sodium à 80 % dans l'huile dans 20 ml de THF. On agite pendant 30 minutes à TA puis on ajoute 0,89 ml d*p*-tolualdéhyde dans 20 ml de THF. Après 18 heures à TA et 2 heures de chauffage à reflux, on verse sur de la glace, extrait par DCM, sèche sur sulfate de sodium et concentre. Le produit brut obtenu est chromatographié sur silice en éluant par un mélange DCM/heptane (8/2 ; v/v). On isole 920 mg d'un produit huileux. On reconnait l'isomère Z par analyse du spectre RMN par effet NOE.

D) (Z) *p*-bromométhylphényl-1 cyano-2 diméthyl-3,3 butène-1.

On porte à reflux pendant 3 heures un mélange contenant 850 mg du composé préparé à l'étape précédente, 760 mg de NBS, 80 mg de péroxyde de benzoyle et 40 ml de tétrachlorure de carbone. Après refroidissement, on essore, lave par du tétrachlorure de de carbone et concentre à sec. On obtient 1,4 g de produit brut utilisé tel quel à l'étape suivante

E) (Z) n-butyl-2 [(diméthyl-3,3 cyano-2 buten-1-yl-1)-4 benzyl]-1 spirocyclopentane-4 imidazoline-2 one-5.

Ce composé est préparé selon le mode opératoire habituel par addition du n-butyl-2 spirocyclopentane-4 imidazoline-2 one-5 sur le produit préparé à l'étape précédente.

F) (Z) n-butyl-2 [(diméthyl-3,3 (tétrazol-5-yl)-2 buten-1-yl-1)-4 benzyl]-1 spirocyclopentane-4 imidazoline-2 one-5.

On porte à reflux pendant 90 heures un mélange contenant 1 g du composé obtenu à l'étape précédente et 1,6 g de tributylétain dans 30 ml de xylène.

On extrait par de la soude 2N puis on acidifie la phase aqueuse par de l'acide chlorhydrique 2N jusqu'à pH 5 ; on extrait par AcOEt, sèche sur sulfate de sodium et concentre. Le produit obtenu est chromatographié sur silice en éluant par un mélange DCM/méthanol (95/5, v/v). On isole 220 mg d'un solide blanc.

Fc = 80°C.

On reconnait l'isomère Z par analyse du spectre RMN avec effet N.O.E. Ainsi l'hydrogène et le *tert*-butyle sont en position cis sur la double liaison.

- RMN :

0,8 ppm : t : 3 H : $CH_3$ (nBu)
1-1,35 ppm : m : 11 H :( tBu) + $CH_3$-$C\underline{H_2}$
1,45 ppm : quint : 2 H : $CH_3$-$CH_2$-$C\underline{H_2}$-
1,45-2 ppm : m : 8 H : cyclopentane
2,2 ppm : t : 2 H : $CH_3$-$CH_2$-$CH_2$-$C\underline{H_2}$-
4,6 ppm : s : 2 H : N-$C\underline{H_2}$-$C_6H_4$-
6,6-7,1 ppm : m : 5 H : aromatiques + =C$\underline{H}$

EXEMPLE 22

trifluoroacétate de n-butyl-2 [(carboxy-2′ biphényl-4-yl) méthoxy]-1 spirocyclopentane-4 imidazoline-2 one-5.

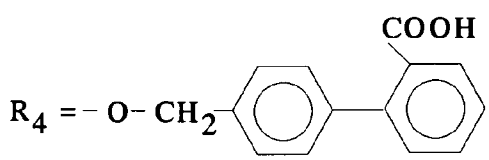

Les deux premières étapes de ce procédé sont réalisées selon A.F. Mc Kay et al., dans Can. J. Chem., 1960, <u>38</u>, 343.

A) N-[(*tert*-butoxycarbonyl-2′ biphényl-4-yl) méthoxy] phtalimide.

A une solution de 2,95 g de N-hydroxy phtalimide dans 30 ml de DMF, on ajoute successivement 4,66 ml de DIPEA et 6,26 g de bromométhyl-4 (*tert*-butoxycarbonyl-2′) biphényle. On laisse sous agitation 2 heures à TA et 4 heures à 80°C puis le milieu réactionnel est repris par 250 ml d'acétate d'éthyle, et lavé successivement par 50 ml d'eau, une solution saturée d'hydrogénocarbonate de sodium, par une solution saturée de chlorure de sodium, une solution de $KHSO_4$-$K_2SO_4$ à 5 % puis une solution saturée de chlorure de sodium. Après séchage et concentration, le résidu est recristallisé dans un mélange méthanol-eau.

m = 7,15 g
Fc = 140-145°C.

B) (*tert*-butoxycarbonyl-2′ biphényl-4-yl) méthoxyamine.

On porte à reflux pendant 4 heures, 6,96 g du composé obtenu à l'étape précédente, dans 15 ml de DCM et 1 ml d'éthanolamine. Le milieu est concentré, repris par DCM, lavé successivement à l'eau, par une solution saturée de $NaHCO_3$ et une solution saturée de NaCl. Après séchage sur sulfate de sodium, la phase organique est concentrée et le résidu est trituré dans l'éther. Un insoluble est éliminé puis le filtrat est concentré et purifié par chromatographie en éluant par le mélange DCM/MeOH (8/2 ; v/v). On obtient 4 g du composé attendu sous forme d'huile.

C) N-(*tert*-butoxy carbonyl-2′ biphényl-4-yl méthoxy)-(N-benzyloxycarbonylamino)-1 cyclopentane carboxamide.

On prépare l'acide N-benzyloxycarbonylcyclopentylcarboxylique selon Tet. Lett., 1966, 4765.

On ajoute 1,65 g de cet acide et 2,79 g de BOP à 1,51 g du composé préparé à l'étape précédente en solution dans 20 ml de DCM et on maintient à pH 7 par addition de DIPEA. Après 8 heures à TA, le milieu réactionnel est concentré, repris par de l'acétate d'éthyle et lavé successivement par de l'eau, une solution de $KHSO_4$-$K_2SO_4$ à 5 %, une solution saturée de NaCl, une solution saturée de $NaHCO_3$ et une solution saturée de NaCl. Après séchage sur sulfate de sodium, la phase organique est concentrée et purifiée sur colonne de silice en éluant par le mélange hexane/AcOEt (6/4 ; v/v). Les fonctions pures sont concentrées pour donner le produit attendu sous forme d'un solide blanc.

m = 2,31 g
Fc = 137-138°C

D) N-(*tert*-butoxycarbonyl-2′ biphényl-4-yl méthoxy)-amino-1-cyclopentane-carboxamide.

2,2 g du composé préparé à l'étape précédente sont mis en solution dans 20 ml de THF et hydrogénés à pression atmosphérique pendant 2 heures en présence de 400 mg de Palladium sur charbon à 10 %. Le catalyseur est essoré et le filtrat concentré puis le résidu est trituré dans un mélange éther-hexane (1/1 ; v/v). Le solide obtenu est essoré et rincé par de l'hexane.
m = 800 mg.

E) n-butyl-2 [(*tert*-butoxycarbonyl-2′ biphényl-4-yl) méthoxy]-1 spirocyclopentane-4 imidazoline-2 one-5.

750 mg du composé préparé à l'étape précédente sont traités par 270 µl d'orthovalérate de méthyle en présence de quelques gouttes d'acide acétique à 110°C pendant 1 heure. Le milieu réactionnel est repris par 50 ml d'AcOEt, lavé de la façon habituelle puis séché sur sulfate de sodium. On obtient 860 mg du composé attendu sous forme d'huile.
- RMN :
  0,8 ppm : t : 3 H : $CH_3$ (nBu)
  1,2 ppm : s : 9 H : (tBu)
  1,2-1,8 ppm : m : 12 H : cyclopentane + $C\underline{H_2}$-$C\underline{H_2}$-$CH_3$
  2,2 ppm : t : 2 H : $C\underline{H_2}$-$CH_2$-$CH_2$-$CH_3$
  5,1 ppm : s : 2 H : $OC\underline{H_2}$
  7,2-7,65 ppm : m : 8 H : aromatiques

F) trifluoroacétate de n-butyl-2 [(carboxy-2′ biphényl-4-yl) méthoxy]-1 spirocyclopentane-4 imidazoline-2 one-5.

830 mg du composé précédent sont traités à TA pendant 30 minutes par 10 ml de TFA dans 7 ml de DCM. La solution est concentrée à sec et le résidu est recristallisé dans un mélange hexane-éther pour obtenir le produit attendu.
m = 825mg
Fc = 143-145°C
- RMN :
  0,9 ppm : t : 3 H : $CH_3$ (nBu)
  1,3 ppm : sext : 2 H : $CH_3$-$C\underline{H_2}$-
  1,5 ppm : quint : 2 H : $CH_3$-$CH_2$-$C\underline{H_2}$-
  1,6-2 ppm : m : 8 H : cyclopentane
  2,35 ppm : t : 2 H : $C\underline{H_2}$-$CH_2$-$CH_2$-$CH_3$
  5,2 ppm : s : 2 H : $OC\underline{H_2}$
  7,39-7,8 ppm : m : 8 H : aromatiques

EXEMPLE 23

dichlorhydrate de n-butyl-2 [(carboxy-2′ biphényl-4-yl) amino]-1 spirocyclohexane-4 imidazoline-2 one-5.

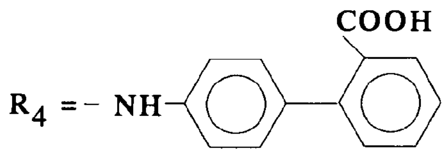

A) On prépare l'acide amino-4′ biphényl-2 carboxylique selon S.A. Glover et al. (J. Chem. Soc., Perkin I, 1981, 842).

B) acide hydrazino-4′ biphényl-2 carboxylique.

Ce composé est préparé selon J. Witte et al., J. Org. Chem., 1972, 37 (18), 2849. On ajoute 235 mg de nitrite de sodium dissous dans 2 ml d'eau à une solution à -10°C de 716 mg du composé préparé à l'étape A dans 2 ml d'acide chlorhydrique et 2 g de glace. Après 10 minutes, on ajoute en 4 fois une solution à -10°C de 1,8 g de $SnCl_2$, $2H_2O$ dans 8,5 ml d'acide chlorhydrique concentré. Après 30 minutes, on porte à pH 7 par addition de lessive de soude. Le solide obtenu est essoré, le filtrat est concentré et repris par MeOH pour éliminer le précipité formé. Après concentration du filtrat et trituration dans l'éthanol on obtient un solide rose (270 mg) utilisé immédiatement pour l'étape suivante.

C) chlorhydrate de l'ester méthylique de l'acide hydrazino-4′ biphényl-2 carboxylique.

On mélange à froid 0,5 mg de chlorure de thionyle dans 5 ml de méthanol, après 15 minutes d'agitation, on ajoute 310 mg du composé préparé précédemment dans 5 ml de méthanol et l'on porte à reflux pendant 2 heures. Le milieu est concentré, repris par de l'éthanol absolu froid, un insoluble est éliminé et le filtrat est concentré pour donner le produit attendu sous forme d'une huile (310 mg).

D) L'acide amino-1 cyclohexanecarboxylique est commercial, il permet de préparer l'acide N-benzyloxycarbonyl amino-1 cyclohexanecarboxylique.

E) dichlorhydrate de n-butyl-2 [(carboxy-2′ biphényl-4-yl) amino]-1 spirocyclohexane-4 imidazoline-2 one-5.

On procède ensuite selon les différentes étapes décrites à l'exemple précédent pour préparer l'ester méthylique du produit attendu puis le produit lui-même.
- spectre de masse : $MH^+$ : 420
- RMN :
  - 0,9 ppm : t : 3 H : $CH_3$ (nBu)
  - 1,15-2-2 ppm : m : 14 H : cyclohexane et $-C\underline{H_2}-C\underline{H_2}-CH_3$
  - 2,6 ppm : t : 2 H : $-C\underline{H_2}-CH_2-CH_2-CH_3$
  - 6,7-7,8 ppm : m : 8 H : aromatiques

EXEMPLE 24

n-butyl-2 (carboxy-2′ biphényl-4-yl)-1 spirocyclohexane-4 imidazoline-2 one-5.

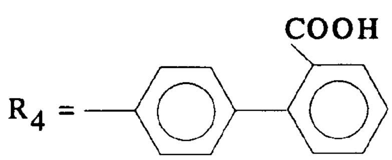

A) Ester méthylique de l'acide amino-4′ biphényl-2 carboxylique.

Ce composé est obtenu à partir de l'acide correspondant décrit à l'étape A de l'exemple précédent. 700 mg de l'acide sont traités par du méthanol saturé en gaz chlorhydrique et portés à reflux pendant 3 heures. Le milieu est concentré puis repris par 50 ml d'eau. La phase aqueuse est lavée 2 fois par 10 ml de l'AcOEt puis on ajoute une solution concentrée de $NaHCO_3$. On extrait 3 fois par 20 ml d'AcOEt, sèche et concentre. Le produit attendu est obtenu sous forme d'huile (330 mg) utilisé tel quel à l'étape suivante (330 mg).

B) n-butyl-2 (méthoxycarbonyl-2′ biphényl-4-yl)-1 spirocyclohexane-4 imidazoline-2 one-5.

On prépare l'acide N-benzyloxycarbonylamino-1 cyclohexane carboxylique puis cet acide est couplé au produit préparé à l'étape A et l'on procède selon les étapes décrites à l'exemple 22 pour obtenir le produit attendu.

- RMN :

0,85 ppm : t : 3 H : $CH_3$ (nBu)
1,25-1,8 ppm : m : 14 H : cyclohexane et $C\underline{H}_2$-$C\underline{H}_2$-$CH_3$
2,4 ppm : t : 2 H : $C\underline{H}_2$-$CH_2$-$CH_2$-$CH_3$
3,65 ppm : s : 3 H : $CO_2C\underline{H}_3$
7,4-7,9 ppm : m : 8 H : aromatiques

C) n-butyl-2 (carboxy-2′ biphényl-4-yl)-1 spirocyclohexane-4 imidazoline-2 one-5.

320 mg du composé préparé à l'étape précédente sont mis en solution dans 10 ml d'éthanol et traités par 2,1 ml de soude à 10 %, pendant 1 heure au reflux. On concentre le milieu puis on reprend par 5 ml d'eau et acidifie par addition d'acide acétique. Le précipité formé est essoré, rincé par de l'eau et séché sur anhydride phosphorique. On obtient 200 mg du produit attendu.
Fc = 85-90°C.

- RMN :

0,7 ppm : t : 3 H : $CH_3$ (nBu)
1,1-1,7 ppm : m : 14 H : cyclohexane et -$C\underline{H}_2$-$C\underline{H}_2$-$CH_3$
2,2 ppm : t : 2 H : $C\underline{H}_2$-$CH_2$-$CH_2$-$CH_3$
7,15-7,7 ppm : m : 8 H : aromatiques
12,6 ppm : s : 1 H : $CO_2H$

EXEMPLE 25

n-butyl-2 [(carboxy-2′ biphényl-4-yl) carbonyl]-1 spirocyclopentane-4 imidazoline-2 one-5.

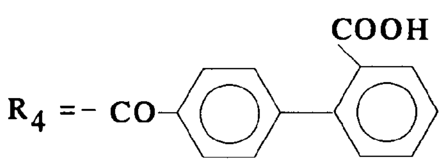

A) Acide *tert*-butoxycarbonyl-2′ biphényl-4 carboxylique.

Ce composé est préparé selon S. Cacchi et al., Chem. Ind., 1986, 286.

On traite 3 g de bromométhyl-4 (*tert*-butoxycarbonyl-2′) biphényle en solution dans 37 ml d'acétonitrile et 5 ml de DCM par 27,6 ml d'une solution aqueuse d'hypochlorite de sodium à 15 % en chlore en présence de 2,95 g de tétrabutylammonium hydrogénosulfate. Après 24 heures d'agitation à TA, on ajoute 200 ml d'éther et 50 ml d'eau. La phase organique est lavée par 100 ml d'eau, séchée et concentrée pour donner une huile jaune qui est purifiée par chromatographie sur silice en éluant par le mélange heptane/AcOEt/AcOH (80/20/2 ; v/v/v). Après recristallisation dans un mélange étherhexane on obtient 1,02 g du produit attendu.
Fc = 174-176°C.

B) n-butyl-2 [(tert-butoxycarbonyl-2' biphényl-4-yl) carbonyl]-1 spirocyclopentane-4 imidazoline-2 one-5.

485 mg du composé préparé à l'étape précédente en solution dans DCM sont traités par 335 mg de DCC pendant 15 minutes puis on ajoute 315 mg de n-butyl-2 spirocyclopentane-4 imidazoline-2 one-5 puis 50 mg de DMAP. Après 24 heures sous agitation, le milieu réactionnel est filtré, le filtrat est purifié et concentré par chromatographie sur silice en éluant par le mélange hexane/AcOEt (9/1, v/v). On obtient le produit attendu sous forme d'huile.
m = 200 mg.
- RMN :
0,8 ppm : t, : 3H : $CH_3$ (nBu)
1,2 ppm : s, : 9H : (tBu)
1,25-2 ppm : m, : 12H : cyclopentane + $CH_3$-$C\underline{H}_2$-$C\underline{H}_2$-
2,7 ppm : t, : 2H : $C\underline{H}_2$-$CH_2$-$CH_2$- $CH_3$
7,35-7,85 ppm : m, : 8H : aromatiques

C) n-butyl-2 [(carboxy-2' biphényl-4-yl) carbonyl]-1 spirocyclopentane-4 imidazoline-2 one-5.

222 mg du composé obtenu à l'étape précédente sont traités pendant 1 heure et demie par 4 ml de TFA et 4 ml de DCM. Après concentration du milieu, le résidu est trituré dans un mélange éther-hexane (1/1, v/v). Le produit attendu est obtenu sous forme d'une poudre blanche instable à TA.
- RMN :
0,8 ppm : t, 3H : $CH_3$ (nBu)
1,2-2,0 ppm : m, 12H : cyclopentane, $CH_2$-$C\underline{H}_2$-$C\underline{H}_2$
2,6 ppm : t, 2H : $C\underline{H}_2$-$CH_2$-$CH_2$-$CH_3$
7,2-7,9 ppm : m, 8H : aromatiques

EXEMPLES 26 et 27

n-butyl-2 spirocyclopentane-4 [((tetrazol-5-yl)-1 butyl-1)-4 benzyl]-1 imidazoline-2 one-5

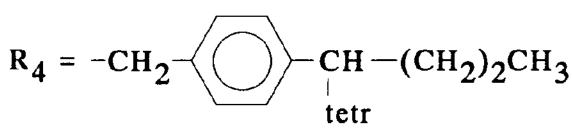

et n-butyl-2 spirocyclopentane-4 [((tetrazol-5-yl)-1 buten-1-yl-1)-4 benzyl]-1 imidazoline-2 one-5.

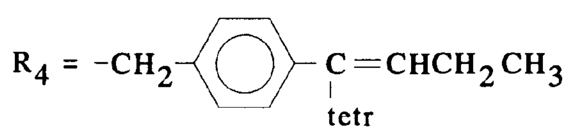

A) (p-tolyl)-1-[trityl-1-tétrazol-5-yl]-1-butane.

En procédant comme dans les premières étapes décrites pour les exemples 10 et 16, on prépare le *p*-tolyl-2 pentanenitrile butyle puis on transforme le groupe cyano en tetrazole puis en trityl-1 tetrazole.

B) (p-bromométhylphényl)-1-[trityl-1-tétrazol-5-yl]-1-butane et (p-bromométhylphényl)-1-[trityl-1-tétrazol-5-yl]-1-bromo-2-butane.

4 g du composé préparé à l'étape A) sont traités par 1,7 g de NBS en présence de 205 mg de peroxyde de benzoyle dans 55 ml de tetrachlorocarbone. Après 3 heures à reflux et retour à TA, on filtre le succinimide formé puis on évapore. La présence des 2 composés bromés est repérée par chromatographie sur couche mince. Ils sont utilisés tels quels à l'étape suivante.

C) n-butyl-2 spirocyclopentane-4 [((trityl-1 tétrazol-5-yl)-1 butyl-1)-4 benzyl]-1 imidazoline-2 one-5 et n-butyl-2 spirocyclopentane-4 [((trityl-1 tetrazol-5-yl)-1 buten-1-yl-1)-4 benzyl]-1 imidazoline-2 one-5.

On place 10 ml de DMF sous atmosphère d'argon, on ajoute 1,24 g d'hydrure de sodium à 80% dans l'huile et 504 mg d'imidazolinone dans 10 ml de DMF. Après 30 minutes à TA, on ajoute goutte à goutte 2,09 g du mélange de produits obtenu à l'étape B). Après 5 heures à TA sous agitation, on évapore, reprend par de l'acétate d'éthyle, lave à l'eau puis par une solution saturée de NaCl, on sèche sur sulfate de sodium et concentre. Le produit obtenu est chromatographié sur alumine en éluant par un mélange hexane/AcOEt (8/2, v/v). On obtient 1,7 g de mélange des produits attendus.

D) n-butyl-2 spirocyclopentane-4 [((tetrazol-5-yl)-1 butyl-1)-4 benzyl]-1 imidazoline-2 one-5 et n-butyl-2 spirocyclpentane-4 [((tetrazol-5-yl)-1 buten-1-yl-1)-4 benzyl]-1 imidazoline-2 one-5.

1 g du mélange obtenu à l'étape précédente est placé dans 5 ml de méthanol ; on refroidit à 0°C et on ajoute 0,8 ml d'acide chlorhydrique 4N puis on laisse sous agitation pendant 3 heures et demie. Après évaporation, on reprend par de l'eau, ajoute de la soude pour amener à pH 13, puis on lave à l'éther, au toluène puis à nouveau à l'éther. On ajuste à pH 5 par addition d'acide chlorhydrique puis on extrait à l'acétate d'éthyle, sèche sur sulfate de sodium et on concentre. Le mélange obtenu est chromatographié sur silice et l'on sépare les 2 produits en éluant par un mélange DCM/MeOH/AcOH (98/2/1, v/v/v). On recueille 90 mg du composé de l'exemple 26 (en butyl) et 50 mg du composé de l'exemple 27 (en butène).

- RMN (exemple 26) :
  - 0,5-0,9 ppm : m : 6 H : $CH_3$ (nBu)
  - 0,9-1,45 ppm : m : 6 H : $CH_3$-$CH_2$-$CH_2$-(nBu)$^+$ $CH_3$-$CH_2$-$CH_2$ butyl
  - 1,45-2 ppm : m : 10 H : $CH_3$-$CH_2$-$CH_2$-$CH_2$-(Butyl)+ cyclopentane
  - 2,2 ppm : t : 2 H : $CH_3$-$CH_2$-$CH_2$-$CH_2$ -(nBu)
  - 4,25 ppm : t : 1 H : -CH-$C_6H_4$-
  - 4,55 ppm : s : 2 H : -N-$CH_2$-$C_6H_4$-
  - 6,9-7,2 ppm : m : 4 H : Aromatiques
- RMN (exemple 27) :
  - 0,5-1,05 ppm : m : 6H : $CH_3$ (nBu) $CH_3$ (butène)
  - 1,2 ppm : sext : 2H : $CH_3$-$CH_2$ (nBu)
  - 1,4 ppm : quint : 2H : $CH_3$-$CH_2$-$CH_2$ (nBu)
  - 1,5-1,9 ppm: m: 8 H: cyclopentane
  - 1,9-2,4: m: 4H: $CH_3$-$CH_2$-$CH_2$-$CH_2$ (nBu) + $CH_3$-$CH_2$ (Butène)
  - 4,65 : s dédoublé: 2H: -N-$CH_2$-$C_6H_4$-
  - 6,4-6,8 :t dédoublé : 1H : =CH
  - 6,9-7,4 : m : 4H : H aromatiques

EXEMPLE 28

n-butyl [(cyclohexyl-(tetrazol-5-yl)-méthyl)-4 benzyl]-1 spirocyclopentane-4 imidazoline-2 one-5.

$R_4$ = 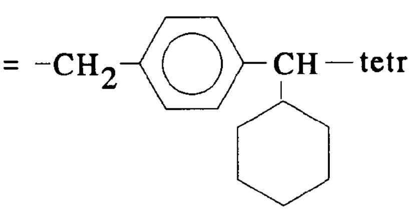

En opérant selon l'exemple 16, on prépare d'abord le [(cyano-p-tolyl)méthyl]-1-cyclohexane par action du bromocyclohexane sur le cyanure de p-méthylbenzyle. Les étapes suivantes effectuées dans les conditions habituelles permettent d'obtenir le produit attendu.

- RMN :
  - 0,6 ppm : t : 3 H : $CH_3$ (nBu)
  - 0,65-1,9 ppm : m : 22 H : $CH_3$-$CH_2$-$CH_2$- + cyclopentane + $CH_2$- (cyclohexyle)
  - 1,9-2,3 ppm : m : 3 H : $CH_2$-$CH_2$-$CH_2$-$CH_3$ + CH (cyclohexyle)
  - 2,5 ppm : m : 2 H : $CH_2$-$CH_2$-$CH_2$-$CH_3$

3,95 ppm : d : 1 H : CH- $C_6H_4$
4,35 ppm : s : 2 H : -N-$CH_2$- $C_6H_4$-
6,8-7,4 ppm : m 4 H : H aromatiques

EXEMPLE 29

n-butyl-2 spirocyclopentane-4 [((tetrazol-5-yl)-3 diméthyl-2,3-butyl-2)-4 benzyl]-1 imidazoline-2 one-5.

$R_4$ =
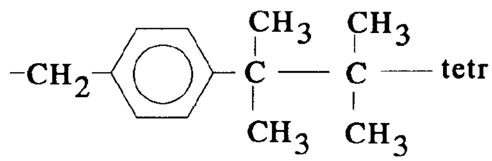

A) méthyl-3 p-tolyl-3 butyronitrile.

Ce composé est préparé à l'exemple 18, étape D).

B) triméthyl-2,2,3 p-tolyl-3 butyronitrile.

On place 20 ml de THF anhydre à 0°C, sous atmosphère d'azote puis on ajoute goutte à goutte 32 ml de LDA (1,5 M dans le cyclohexane) dans 10 ml de THF et 2,1 g du nitrile préparé à l'étape A) dans 5 ml de THF. Après 15 minutes sous agitation, on ajoute goutte à goutte 6,1 ml d'iodure de méthyle dans 5 ml de THF. On laisse revenir à TA puis on porte à reflux pendant 2 heures et demie. Après évaporation, on reprend par de l'acétate d'éthyle, lave à l'eau puis successivement par une solution de $KHSO_4$ de l'eau, une solution saturée de NaCl. On sèche sur sulfate de sodium puis on concentre les phases organiques. On chromatographie ensuite sur silice en éluant par un mélange AcOEt/hexane (5/95, v/v). On obtient 2 g du produit attendu.

C) n-butyl-2 spirocyclopentane-4 [((tetrazol-5-yl)-3 diméthyl-2,3-butyl-2)-4 benzyl]-1 imidazoline-2 one-5.

En procédant selon les méthodes habituelles, on prépare le produit attendu.
- RMN
0,7 ppm : t : 3 H : $CH_3$ (nBu)
1-1,5 ppm : m : 16 H : $CH_3$-$CH_2$-$CH_2$-+4 $CH_3$
1,5-1,9 : m : 8 H : cyclopentane
2,2 ppm : t : 2H : $CH_3$-$CH_2$-$CH_2$-$CH_2$-
4,55 : s : 2 H : -N-$CH_2$-$C_6H_4$-
6,55 : m : 4 H : H aromatiques

EXEMPLE 30

n-butyl-2 [(diméthyl-3,3 (tétrazol-5-yl)-1 buten-1-yl-2)-4 benzyl]-1 spirocyclopentane-4 imidazoline-2 one-5.

$R_4$ =
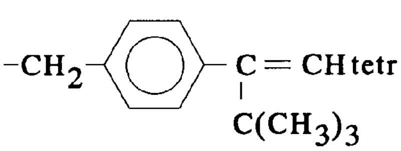

A) *tert*-butyl p-tolylcetone.

Ce composé est préparé selon J. Am. Chem. Soc.; 1950, 4169.

B) diméthyl-4,4 *p*-tolyl-3 penten-2 nitrile.

Ce composé est préparé selon Chem. Pharm. Bull., 1980, 1394.
On dissout 2,15 g de sodium dans 70 ml d'éthanol puis on refroidit la solution par un bain de glace et l'on

ajoute goutte à goutte 16,46 g de diethylcyanométhylphosphonate. Après 10 minutes sous agitation à 10°C, on ajoute 13,9 g de la cétone préparée à l'étape précédente et on laisse sous agitation 1 heure à TA, puis 18 heures à 60°C. Après évaporation, on reprend par de l'éther, lave à l'eau, par une solution saturée de chlorure de sodium puis on sèche sur sulfate de sodium. Après concentration des phases organiques, on les purifie par chromatographie sur silice en éluant par un mélange AcOEt/hexane (5/95, v/v). On obtient 7,57 g du produit attendu.

C) n-butyl-2 [(diméthyl-3,3 (tétrazol-5-yl)-1 buten-1yl-2)-4 benzyl]-1 spirocyclopentane-4 imidazoline-2 one-5.

On procède selon les étapes habituelles pour préparer le produit attendu, caractérisé par son point de fusion.
Fc = 145°C

EXEMPLES 31 et 31bis

n-butyl-2 [((α-tétrazol-5-yl)-benzyl)-4 benzyl]-1 spirocyclopentane-4 imidazoline-2 one-5.

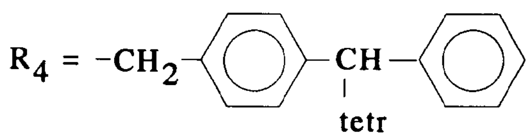

et n-butyl-2-[((α-hydroxy α-tétrazol-5-yl)benzyl)-4 benzyl]-1 spirocyclopentane-4 imidazoline-2 one-5.

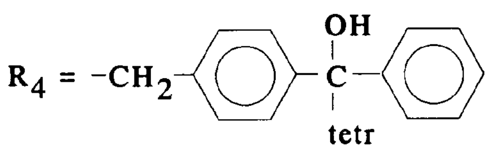

A) Le p-tolyl phényl acétonitrile est préparé selon K. SISIDO dans J. Org. Chem., 1954, 19, 1699.

B) (Phényl p-tolyl tétrazol-5-yl) méthane.

1,12 g du composé de l'étape A sont traités par 1,8 g d'azide de tributyletain dans 10 ml de xylène et portés à reflux pendant 3 jours. On reprend par 50 ml d'une solution aqueuse de soude à 5% et par 50 ml d'éther. La phase éthérée est lavée par 50 ml de solution aqueuse de soude à 5% ; les phases aqueuses réunies sont lavées par 20 ml d'éther puis acidifiées par de l'acide chlorhydrique concentré jusqu'à pH 2. Le précipité obtenu est essoré, rincé par de l'eau et séché sous vide sur anhydride phosphorique. On obtient 1,15 g du produit attendu sous forme d'un solide blanc. PF: 148-150°C
- RMN

2,2 ppm : s : 3 H :$-C_6H_4-C\underline{H}_3$
5,85 ppm : s : 1 H : $-C_6H_4-C\underline{H}-C_6H_5$
7,1-7,4 ppm : m : 9H : Aromatiques

C) [Phényl p-tolyl (trityltétrazol-5-yl)] méthane.

On mélange 1,15 g du produit préparé à l'étape précédente dans 25 ml de DCM avec 1,54 g de chlorure de trityle et 1,1 ml de triethylamine et l'on chauffe à reflux pendant 3 heures. La solution est concentrée sous vide, reprise par de l'acétate d'éthyle, lavée 2 fois par de l'eau puis par une solution saturée de chlorure de sodium. On sèche sur sulfate de sodium, concentre partiellement puis on filtre sur silice. Après concentration, on obtient une mousse blanche utilisée telle quelle à l'étape suivante.
- RMN

2,3 ppm : s : 3 H : $-C_6H_4-C\underline{H}_3$
6,0 ppm : s : 1 H : $-C_6H_4-C\underline{H}-C_6H_5$
7,0-7,5 ppm : m : 24H : Aromatiques

D) [(bromométhyl-4 phényl) phényl (trityltetrazol-5-yl)] méthane.

Le produit brut obtenu à l'étape précédente (4,6 mmoles) est traité par 820 mg de NBS et 30 mg de peroxyde de benzoyle dans 20 ml de tetrachlorocarbone et le mélange est porté à reflux sous un rayonnement UV pendant 1 heure et demie. On essore le milieu, rince le solide par du tetrachlorure de carbone, concentre le filtrat sous vide. La mousse obtenue est utilisée telle qu'elle à l'étape suivante.

E) n-butyl-2 [((α-trityltetrazol-5-yl) benzyl)-4 benzyl]-1 spirocyclopentane-4 imidazoline-2 one-5.

740 mg de chlorhydrate de n-butyl-2 spirocyclopentane-4 imidazoline-2 one-5 sont ajoutés à 0,193 g d'hydrure de sodium à 80% dans l'huile et 10 ml de DMF. Après 15 minutes à TA sous argon, on ajoute à 0°C le produit brut obtenu à l'étape précédente. Après 3 heures d'agitation, sous argon à TA, on concentre sous vide le milieu réactionnel puis on reprend le résidu dans de l'acétate d'éthyle et on lave successivement par de l'eau, une solution saturée d'hydrogenocarbonate de sodium, une solution saturée de chlorure de sodium. Après séchage sur sulfate de sodium et concentration sous vide, l'huile obtenue est chromatographiée sur silice en éluant par un mélange heptane/acétate d'éthyle (7/3 ; v/v). On obtient 2 fractions ; la fraction la moins polaire est constituée par 400 mg du produit attendu sous forme de mousse blanche.

- RMN :

  0,85 ppm : t : 3 H : $C\underline{H}_3$ (nBu)
  1,25 ppm : sext : 2H : $CH_3$-$C\underline{H}_2$
  1,45 ppm : quint : 2H : $CH_3$-$CH_2$-$C\underline{H}_2$
  1,6-2,0 ppm : m : 8 H : cyclopentane
  2,35 ppm : t : 2H : $CH_3$-$CH_2$-$CH_2$-$C\underline{H}_2$-
  4,7 ppm : s : 2 H : -N-$C\underline{H}_2$- $C_6H_4$-
  6,05 ppm : s : 1 H : -$C_6H_4$-$C\underline{H}$-$C_6H_5$
  7,0-7,5 ppm : m : 24H : Aromatiques

La fraction la plus polaire est constituée par le produit suivant : n-butyl-2 [((α-hydroxy α-trityltetrazol-5-yl) benzyl)-4 benzyl]-1 spirocyclopentane-4 imidazoline-2 one-5.

F) n-butyl-2 [((α-tetrazol-5-yl) benzyl)-4 benzyl]-1 spirocyclopentane-4 imidazoline-2 one-5.

370 mg du produit obtenu à l'étape précédente (fraction la moins polaire) sont placés dans 5 ml de DCM et traités par 5 ml d'acide formique et 0,75 ml d'eau. Après 3 heures, on concentre à sec puis on effectue une chromatographie sur silice en éluant par un mélange heptane/acétone/acide acétique (75/35/1). Les fractions pures sont concentrées à sec. Le résidu est repris dans un mélange éther-hexane et concentré sous vide pour obtenir une mousse sèche.
m=200 mg

- RMN :

  0,75 ppm : t : 3 H : $C\underline{H}_3$ (nBu)
  1,2 ppm : sext : 2H : $CH_3$-$C\underline{H}_2$
  1,4 ppm : quint : 2H : $CH_3$-$CH_2$-$C\underline{H}_2$
  1,6-1,9 ppm : m : 8 H : cyclopentane
  2,3 ppm : t : 2H : $CH_3$-$CH_2$-$CH_2$-$C\underline{H}_2$-
  4,65 ppm : s : 2 H : -N-$C\underline{H}_2$- $C_6H_4$-
  5,9 ppm : s : 1 H : -$C_6H_4$-$C\underline{H}$-$C_6H_5$
  7,0-7,4 ppm : m : 9H : Aromatiques

G) n-butyl-2 [((α-hydroxy α-tetrazol-5-yl) benzyl)-4 benzyl]-1 spiro-cyclopentane-4 imidazoline-2 one-5.

400 g du composé obtenu à l'étape E) (fraction la plus polaire) sont dissous dans 5 ml de DCM et traités par 5 ml d'acide formique et 0,75 ml d'eau à TA, pendant 4 heures. Le milieu réactionnel est concentré et le résidu est chromatographié sur silice en éluant par un mélange heptane/acétone/AcOH (70/30/1 ; v/v/v). Les fractions pures sont réunies et concentrées. Après trituration dans un mélange éther/hexane (1/1 ; v/v), on obtient le produit attendu sous forme d'un solide blanc.

  m = 100 mg
  Fc = 179-181°C.

- RMN :

  0,75 ppm : t : 3 H : $CH_3$ (nBu)

1,2 ppm : sext : 2H : $CH_3$-$C\underline{H_2}$-$CH_2$-$CH_2$-
1,45 ppm : quint : 2H : $CH_3$-$CH_2$-$C\underline{H_2}$-$CH_2$-
1,6-1,9 ppm : m : 8 H : cyclopentane
2,3 ppm : t : 2H : $CH_3$-$CH_2$-$CH_2$-$C\underline{H_2}$-
4,65 ppm : s : 2 H : -N-$C\underline{H_2}$- $C_6H_4$-
7-7,4 ppm : m : 10 H : Aromatiques + OH
14,8 ppm : s : 1 H : NH (tetrazole)

EXEMPLE 32

Acide [((n-butyl-2 oxo-5 spirocyclopentane-4 imidazolin-2-yl-1) méthyl)-4 phénoxy]-2 phényl-2 acétique.

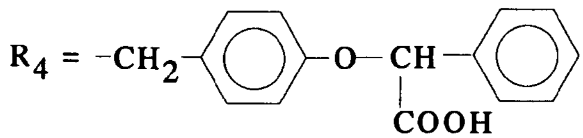

A) Ester éthylique de l'acide bromo-2 phénylacétique.

On dissout 34,5 g d'ester éthylique de l'acide phénylacétique dans 150 ml de tétrachlorure de carbone ; on ajoute 37,5 g de NBS et 38 mg de peroxyde de benzoyle, puis on chauffe à reflux pendant 6 heures. Après refroidissement, le milieu est essoré puis le filtrat est concentré à sec et distillé sous pression réduite. On obtient 43 g du produit attendu.
Eb = 78-80°C sous 0,1 mm de Hg.

B) Ester éthylique de l'acide p-tolyloxy-2 phényl-2 acétique.

On utilise 14,5 g d'une suspension d'hydrure de potassium à 35% dans l'huile et l'on ajoute sous argon 150 ml de DMF. On ajoute 10,8 g de p-cresol dans 100 ml de DMF et 2,7 g d'éther couronne (18 Crown-6). Après 45 minutes d'agitation, on ajoute 24,3 g d'ester préparé à l'étape précédente dans 50 ml de DMF et l'on maintient l'agitation pendant 4 heures à TA. Le milieu est concentré puis on extrait à l'acétate d'éthyle et lave à l'eau. Le produit attendu est obtenu par chromatographie sur silice en éluant par un mélange heptane/AcOEt (95/5 ; v/v).
m = 10,5 g..

C) Ester éthylique de l'acide (bromométhyl-4 phénoxy)-2 phényl-2 acétique.

Dans 216 ml de tétrachlorure de carbone, on introduit 5,38 g du composé préparé à l'étape précédente et 3,18 g de NBS; on ajoute 1 g d'azobisisobutyronitrile et l'on porte à reflux pendant 4 heures et demie. On refroidit, on essore le milieu réactionnel, puis on concentre le filtrat. Le produit attendu est obtenu par chromatographie sur silice en éluant par un mélange heptane/AcOEt (95/5 ; v/v).
m = 2,15 g.

D) Ester éthylique de l'acide [((n-butyl-2 oxo-5 spirocyclopentane-4 imidazolin-2-yl-1) méthyl)-4 phénoxy]-2 phényl-2 acétique.

Sous argon, on mélange 462 mg de chlorhydrate de n-butyl-2 spirocyclopentane-4 imidazoline-2 one-5 et 150 mg d'hydrure de sodium en suspension à 80% dans l'huile dans 10 ml de DMF. Après 30 minutes sous agitation, on ajoute 944 mg du composé préparé à l'étape précédente dans 10 ml de DMF et on laisse 4 heures à TA sous agitation. Le milieu réactionel est concentré à sec puis extrait par AcOEt et la phase organique est lavée à l'eau puis par une solution saturée de chlorure de sodium. Après concentration, le résidu est chromatographié sur silice en éluant par un mélange DCM/MeOH (95,5/0,5 ; v/v).
m = 660 mg.

E) Acide [((n-butyl-2 oxo-5 spirocyclopentane-4 imidazolin-2-yl-1) méthyl)-4 phénoxy]-2 phényl-2 acétique.

Le composé préparé à l'étape précédente (650 mg) est dissous dans 12 ml de soude et la solution est versée dans 3 ml d'eau. Après 3 heures d'agitation à TA, le milieu est concentré à sec, repris par de l'eau puis acidifié vers pH 3 par addition d'hydrogénosulfate de potassium en solution aqueuse à 3%. On extrait par AcOEt puis on lave à l'eau. Après concentration, le résidu est chromatographié sur silice en éluant par un mélange DCM/MeOH (9/1 ; v/v).

m = 110 mg.

Fc = 133°C.

- RMN :

0,7 ppm : t : 3 H : $CH_3$ (nBu)

1-2 ppm : m : 12 H : $CH_2$-$C\underline{H}_2$-$C\underline{H}_2$-$CH_3$ + cyclopentane

2,2 ppm : t : 2H : $C\underline{H}_2$-$CH_2$-$CH_2$-$CH_3$

4,5 ppm : s : 2 H : -N-$C\underline{H}_2$- $C_6H_4$-

5,3 ppm : s : 1 H : -OCH-

6,6-7,6 ppm : m : 9H : Aromatiques

EXEMPLE 33

Acide (chloro-2 phényl)-2 [((n-butyl-2 oxo-5 spirocyclopentane-4 imidazolin-2-yl-1) méthyl)-4 phénoxy]-2 acétique.

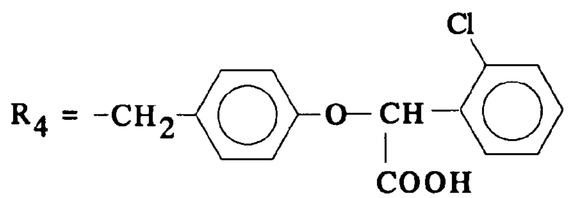

Ce composé est préparé selon le mode opératoire décrit à l'exemple 32, en utilisant comme produit de départ l'ester éthylique de l'acide (chloro-2 phényl) acétique.

- RMN :

0,7 ppm : t : 3 H : $CH_3$ (nBu)

1-2 ppm : m : 12 H : $CH_2$-$C\underline{H}_2$-$C\underline{H}_2$-$CH_3$ + cyclopentane

2,25 ppm : t : 2H : $C\underline{H}_2$-$CH_2$-$CH_2$-$CH_3$

4,6 ppm : s : 2 H : -N-$C\underline{H}_2$- $C_6H_4$-

6 ppm : s : 1 H : O-C$\underline{H}$-

7 ppm : système AA'-BB' : 4 H : Aromatiques

7,2-7,7 ppm : m : 4 H : Aromatiques

EXEMPLE 34

Ester éthylique de l'acide [((n-butyl-2 oxo-5 spirocyclopentane-4 imidazolin-2-yl-1) méthyl)-4 phényl]-3 carbéthoxy-2 cinnamique.

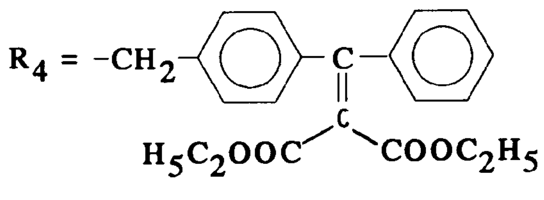

A) p-tolyl-3-carbéthoxy-2-cinnamate d'éthyle.

A 0°C, on prépare une solution contenant 2,5 ml de tétrachlorure de titane, 13 ml de tétrachlorure de carbone et 20 ml de THF anhydre. Après 20 minutes, on ajoute peu à peu le mélange suivant : 1,6 ml de pyridine, 1,48 g de malonate d'éthyle et 1,07 g de méthyl-4 benzophénone dans 10 ml de THF. Après 24 heures à TA, on filtre le milieu réactionnel et l'on extrait par AcOEt ; on lave la phase organique par une solution saturée de bicarbonate de sodium puis par une solution d'acide chlorhydrique normale ; on sèche sur sulfate de sodium et évapore, puis on chromatographie le résidu sur silice en éluant par un mélange hexane/éther (5/1 ; v/v). On obtient 1,28 g du produit attendu.

B) (bromométhyl-4)-phényl-3-carbéthoxy-2-cinnamate d'éthyle.

1 g du produit préparé à l'étape précédente est mis en suspension dans 20 ml de tétrachlorure de carbone ; on ajoute 0,578 g de NBS et 30 mg d'azobisisobutyronitrile. Après 2 heures à reflux sous irradiation UV, on évapore à sec jusqu'à obtention du produit attendu sous forme solide.

C) Ester éthylique de l'acide [((n-butyl-2 oxo-5 spirocyclopentane-4 imidazolin-2-yl-1) méthyl)-4 phény]-3 carbéthoxy-2 cinnamique.

Ce produit est obtenu en procédant selon les méthodes habituelles.
- RMN :
  0,9-1 ppm : m : 9 H : 2 $CH_3$ ($CO_2Et$) + $CH_3$ (nBu)
  1,2-1,4 ppm : m : 2 H : $CH_2$ (nBu)
  1,4-1,6 ppm : m : 2 H : $CH_2$ (nBu)
  1-7-1,9 ppm : m : 8 H : cyclopentane
  2,2 ppm : m : 2H : $CH_2$ (nBu)
  4-4,1 ppm : m : 4 H : 2 $CH_2$ ($CO_2Et$)
  4,8 ppm : s : 2 H : -N-$C\underline{H_2}$- $C_6H_4$-
  7,1-7,5 ppm : m : 9 H : Aromatiques

EXEMPLE 35

Acide [((n-butyl-2 oxo-5 spirocyclopentane-4 imidazolin-2-yl-1) méthyl)-4 phényl]-3 carboxy-2 cinnamique.

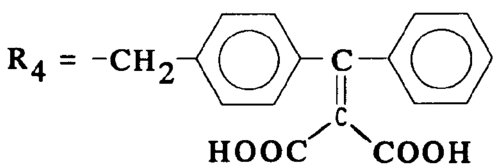

On met en suspension, dans 3 ml de benzène, 106 mg du composé préparé à l'exemple précédent et l'on ajoute 90 mg de potasse anhydre en poudre fine et 32 mg d'éther couronne (18 Crown-6). On laisse sous agitation pendant 18 heures à TA et à l'abri de l'humidité. On ajoute 5 ml de benzène et on acidifie par addition d'acide chlorhydrique N. La gomme formée est reprise par de l'hexane et l'on obtient 60 mg du produit attendu sous forme de poudre.
  Fc = 135-140°C.
- RMN :
  0,8 ppm : m : 3 H : $CH_3$ (nBu)
  1,1-1,6 ppm : m : 4 H : 2$CH_2$ (nBu)
  1-8 ppm : m : 8 H : cyclopentane
  2,3 ppm : m : 2H : $CH_2$ (nBu)
  4,7 ppm : s : 2 H : -N-$C\underline{H_2}$- $C_6H_4$-
  7-7,4 ppm : m : 9 H : Aromatiques
  12,5-13,3 ppm : m : 2 H : 2COO$\underline{H}$

EXEMPLE 36

Chlorhydrate de n-butyl-2 [(phényl-1 (ditétrazol-5-yl)-2,2 vinyl)-4 benzyl]-1 spirocyclopentane-4 imidazolin-2 one-5.

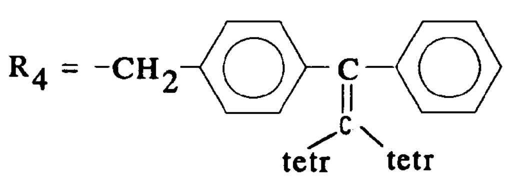

A) Chlorhydrate de p-tolyl phénylcétimine.

On porte à 120°C, pendant 15 heures en tube scellé, un mélange contenant 13,2 g de méthyl-4 benzophénone, 125 ml d'ammoniac liquide, 3,96 g de chlorure d'ammonium dans 60 ml de THF. On chasse l'ammoniac puis on ajoute 80 ml d'éther et 40 ml d'eau. Après décantation, on extrait à l'éther, sèche et évapore. Le produit attendu est obtenu sous forme solide.

B) α, α-dicyano-β-(p-tolyl)-styrène.

Dans 100 ml d'éthanol, on place 10 g du composé préparé à l'étape précédente et l'on ajoute 5,45 ml de N-éthyl morpholine et 2,85 g de malonitrile. Après 5 heures à TA, on concentre de moitié, on filtre l'insoluble et lave par de l'éther. Le produit brut obtenu est repris par du chloroforme ; on filtre, évapore les solvants et l'on obtient 7,1 g du produit attendu.

C) α,α-dicyano-β-(bromométhyl-4-phényl)styrène.

On place 6 g du composé préparé à l'étape précédente dans 100 ml de tétrachlorure de carbone et l'on ajoute 4,82 g de NBS et 250 mg d'azobisisobutyronitrile ; on porte à reflux puis sous irradiation UV pendant 10 minutes. Le milieu réactionnel est ensuite évaporé à sec pour obtenir le produit attendu sous forme d'une huile.

D) n-butyl-2 spirocyclopentane-4 [(dicyano-2,2 phényl-1 vinyl)-4 benzyl]-1 imidazolin-2 one-5.

Ce composé est préparé par la méthode habituelle.
- RMN :
 0,6-0,7 ppm : m : 3 H : $CH_3$ (nBu)
 1,1-1,4 ppm : m : 4 H : $CH_2$-$C\underline{H}_2$-$C\underline{H}_2$-$CH_3$
 1,6-1,9 ppm : m : 8 H : cyclopentane
 2,2-2,4 ppm : m : 2 H : $C\underline{H}_2$-$CH_2$-$CH_2$-$CH_3$
 4,7 ppm : s : 2 H : -N-$C\underline{H}_2$- $C_6H_4$-
 7,1-8,1 ppm : m : 9 H : Aromatiques

E) Chlorhydrate de n-butyl-2 spirocyclopentane-4 [(phényl-1 (ditétrazol-5-yl)-2,2 vinyl)-4 benzyl]-1 imidazolin-2 one-5.

On opère selon la méthode habituelle pour transformer les groupements cyano en tétrazolyl et obtenir ainsi le produit attendu.
- RMN :
 0,8-0,9 ppm : m : 3 H : $CH_3$ (nBu)
 1,2-1,6 ppm : m : 6 H : $CH_2$-$C\underline{H}_2$-$C\underline{H}_2$-$CH_3$
 1,8-2,2 ppm : m : 8 H : cyclopentane
 2,8 ppm : m : 2 H : $C\underline{H}_2$-$CH_2$-$CH_2$-$CH_3$
 4,95 ppm : s : 2 H : -N-$C\underline{H}_2$- $C_6H_4$-
 6,9-7,4 ppm : m : 9 H : Aromatiques

EXEMPLE 37

Acide [n-butyl-2 oxo-5 spirocyclopentane-4 imidazol-2-yl-1 méthyl]-4 benzoïque.

$R_4$ = 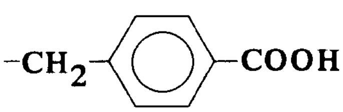

A) *tert*-butylate de l'acide méthyl-4 benzoïque.

26,2 g de chlorure de p-toluyle dans 500 ml de THF sont refroidis à 15°C et traités sous agitation par 22,4 g de *tert*-butylate de potassium en poudre, ajoutés par petites fractions, en maintenant la température à 15-20°C par un bain de glace. On laisse sous agitation à TA pendant 1 heure, puis le milieu est concentré de moitié puis dilué par 500 ml d'eau. On extrait à l'éther, lave les phase éthérées par une solution saturée d'hydrogènocarbonate de sodium, par une solution saturée de chlorure de sodium, puis sèche sur sulfate de sodium et concentre. On obtient 32 g du produit attendu sous forme d'une huile.

B) *tert*-butylate de l'acide [n-butyl-2 oxo-5 spirocyclopentane-4 imidazol-2-yl-1 méthyl]-4 benzoïque.

On opère en 2 étapes selon les méthodes habituelles.

C) Acide [n-butyl-2 oxo-5 spirocyclopentane-4 imidazol-2-yl-1 méthyl]-4 benzoïque.

1,5 g de l'ester *tert*-butylique précédemment obtenu est traité par le TFA en suivant l'exemple 2, étape D). L'huile obtenue est additionnée d'eau puis l'on amène à pH 6 par addition d'hydrogènocarbonate de sodium. On extrait à l'acétate d'éthyle, lave par une solution de chlorure de sodium, sèche sur sulfate de sodium et évapore à sec.
m = 0,9 g.
Fc = 144-148°C.

EXEMPLE 38

[benzoyl-4 benzyl]-1 n-butyl-2 spirocyclopentane-4 imidazoline-2 one-5.

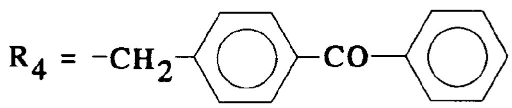

Ce composé est préparé par les méthodes habituelles. A partir de la méthyl-4 benzophénone (commerciale), on prépare la bromométhyl-4 benzophénone puis on additionne ce produit sur le chlorhydrate de n-butyl-2 spirocyclopentane-4 imidazoline-2 one-5.
Fc = 99-101°C.

EXEMPLE 39

[(α-acétoxybenzyl)-4 benzyl]-1 n-butyl-2 spirocyclopentane-4 imidazoline-2 one-5.

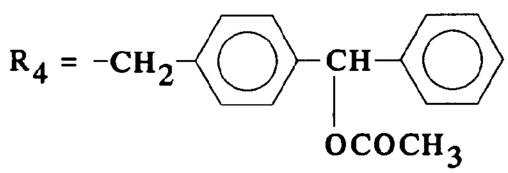

A partir du méthyl-4 benzhydrol (commercial), on prépare l'acétate de (méthyl-4) diphénylméthane, puis on prépare le composé selon l'invention en suivant les procédés habituels.
- RMN :
0,65 ppm : t : 3 H : $CH_3$ (nBu)
1,1 ppm : sext : 2H : $CH_3$-$C\underline{H_2}$-$CH_2$-$CH_2$-
1,35 ppm : quint : 2H : $CH_3$-$CH_2$-$C\underline{H_2}$-$CH_2$-
1,4-1,5 ppm : m : 8 H : cyclopentane
2,05 ppm : s : 3 H : $OCOC\underline{H_3}$
2,2 ppm : t : 2 H : $CH_3$-$CH_2$-$CH_2$-$C\underline{H_2}$-
4,55 ppm : s : 2 H : -N-$C\underline{H_2}$-$C_6H_4$-
6,65 ppm : s : 1 H : -$C_6H_4$-$C\underline{H}$-$C_6H_5$
7-7,4 ppm : m : 9 H : Aromatiques

EXEMPLE 40

Acide difluoro-2,2-para-[(n-butyl-2 oxo-5 spirocyclopentane-4 imidazolin-2-yl-1) méthyl]phénylacétique.

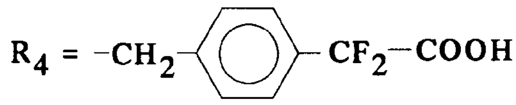

A) Ester éthylique de l'acide difluoro-2,2 p-tolyl-2 acétique.

On prépare l'ester éthylique de l'acide oxo-2 p-tolyl-2 acétique selon R.C. Cousins dans J. Org. Chem., 1980, 45, 2976-2983. A 2,88 g de cet ester, on ajoute goutte à goutte 8 ml de trifluorure de diéthylaminosulfure et on laisse sous agitation une nuit à TA. On dilue le milieu par de la glace puis on extrait à l'acétate d'éthyle et on lave la phase organique par une solution saturée de $NaHCO_3$, une solution de $KHSO_4$-$K_2SO_4$, de l'eau, puis une solution saturée de NaCl.

B) Ester éthylique de l'acide difluoro-2,2-para-[(n-butyl-2 oxo-5 spirocyclopentane-4 imidazolin-2-yl-1) méthyl] phénylacétique.

Ce composé est préparé selon les méthodes habituelles par addition de l'ester éthylique de l'acide (bromométhyl-4 phényl)-2 difluoro-2,2 acétique sur la n-butyl-2 spirocyclopentane-4 imidazoline-2 one-5.

C) Acide difluoro-2,2-para-[(n-butyl-2 oxo-5 spirocyclopentane-4 imidazolin-2-yl-1) méthyl] phénylacétique.

500 mg de l'ester préparé à l'étape précédente sont placés dans 2 ml de méthanol et 2 ml de dioxanne en présence de 1,3 ml de soude N. Après 1 heure et demie, le milieu est dilué par AcOEt et acidifié à pH 4 par addition d'acide chlorhydrique N. Après évaporation, le milieu est repris par de l'éther. Le solide obtenu est filtré puis lavé par de l'eau et une solution saturée de NaCl.

m = 220 mg

Fc = 194-198°C.

EXEMPLE 41

N-hydroxy [((n-butyl-2 oxo-5 spirocyclopentane-4 imidazolin-2-yl-1) méthyl)-4 phényl]-2 phényl-2 acétamide.

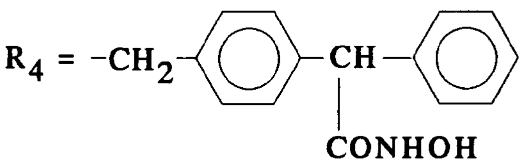

589 mg de l'acide préparé à l'exemple 1 sont placés dans 20 ml de chloroforme et traités à 0°C par 1,2 ml de chlorure de thionyle. Après 1 heure à TA, le milieu réactionnel est concentré et les traces de chlorure de thionyle sont éliminées par co-évaporation avec du toluène. Le chlorure d'acide formé est repris par 10 ml de DCM et ajouté goutte à goutte à 0°C à une solution contenant 250 mg de chlorhydrate d'hydroxylamine et 917 µl de DIPEA dans 10 ml de DMF. Après 1 heure à TA, la solution est concentrée, reprise par DCM puis lavée à l'eau, séchée et concentrée. Le résidu est chromatographié sur silice en éluant par un mélange toluène-méthanol (9/1 ; v/v). Les fractions pures sont réunies et concentrées et le résidu est recristallisé dans un mélange éther/hexane.

m = 400 mg

$MH^+$ : 434

Fc = 90°C avec décomposition.

- RMN :

0,7 ppm : t : 3H : $CH_3$ (nBu)

1,15 ppm : sext : 2H : $CH_3$-$C\underline{H_2}$-$CH_2$-$CH_2$-

1,4 ppm : quint : 2H : $CH_3$-$CH_2$-$C\underline{H_2}$-$CH_2$-

1,5-1,8 ppm : m : 8 H : cyclopentane
2,2 ppm : t : 2 H : $CH_3$-$CH_2$-$CH_2$-C$\underline{H}_2$-
4,55 ppm : s : 2 H : -N-C$\underline{H}_2$-$C_6H_4$-
4,6 ppm : s : 1 H : -$C_6H_4$-C$\underline{H}$-$C_6H_5$
6,95-7,3 ppm : m : 9 H : Aromatiques

EXEMPLE 42

Acide [((n-butyl-2 oxo-5 spirocyclopentane-4 imidazolin-2-yl-1) méthyl-4) phényl]-2 hydroxy-2 phényl-2 acétique.

$R_4$ = 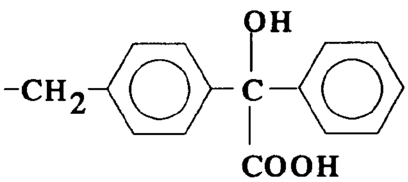

A) Acide p-tolyl-2 hydroxy-2 phénylacétique.

Ce composé est préparé selon A. Mc Kenzie, J. Chem. Soc., 1934, 1070-1075.

B) Ester méthylique de l'acide p-tolyl-2 hydroxy-2 phényl-2 acétique.

On porte à reflux pendant une nuit, 5 g du composé préparé à l'étape A, dissous dans 150 ml de méthanol, en présence de 7,5 ml d'acide sulfurique concentré. On amène la solution à pH 6-7 par addition de soude aqueuse concentrée puis on extrait par de l'acétate d'éthyle. La phase organique est lavée à l'eau puis par une solution concentrée de chlorure de sodium. On sèche sur sulfate de sodium puis concentre. Le résidu est chromatographié sur silice en éluant par un mélange cyclohexane/AcOEt (95/5 ; v/v). On isole 2 produits identifiés par RMN et IR : l'ester méthylique de l'acide p-tolyl-2 méthoxy-2 phénylacétique et l'ester méthylique de l'acide p-tolyl-2 hydroxy-2 phénylacétique.

C) Ester méthylique de l'acide p-bromométhylphényl-2 hydroxy-2 phényl-2 acétique.

Ce composé est préparé par la méthode habituelle à partir de l'ester méthylique de l'acide p-tolyl-2 hydroxy-2 phénylacétique obtenu à l'étape précédente.

D) Ester méthylique de l'acide [((n-butyl-2 oxo-5 spirocyclopentane-4 imidazolin-2-yl-1) méthyl-4) phényl]-2 hydroxy-2 phényl-2 acétique.

Ce produit est obtenu par la méthode habituelle par addition du produit préparé à l'étape C sur le chlorhydrate de n-butyl-2 spirocyclopentane-4 imidazoline-2 one-5.

E) Acide [((n-butyl-2 oxo-5 spirocyclopentane-4 imidazolin-2-yl-1) méthyl-4) phényl]-2 hydroxy-2 phényl-2 acétique.

Dans 15 ml de méthanol, on dissout 1,37 g du composé préparé à l'étape D et l'on ajoute 4 ml de soude N. Après 2 heures 15 minutes d'agitation à TA, on dilue par de l'eau et de l'acétate d'éthyle, on amène à pH 4 par addition d'acide chlorhydrique N. La phase organique est décantée, lavée par de l'eau, puis par une solution aqueuse de chlorure de sodium; on sèche et concentre.

Fc = 98°C.

- RMN :

7,00-7,4 ppm : m : 9 H : Aromatiques
4,65 ppm : s : 2 H : -N-C$\underline{H}_2$-$C_6H_4$-
2,3 ppm : t : 2 H : $CH_3$-$CH_2$-$CH_2$-C$\underline{H}_2$-
1,6-1,9 ppm : m : 8 H : cyclopentane
1,47 ppm : quint : 2H : $CH_3$-$CH_2$-C$\underline{H}_2$-$CH_2$-
1,25 ppm : sext : 2H : $CH_3$-C$\underline{H}_2$-$CH_2$-$CH_2$-

0,75 ppm : t : 3 H : $C\underline{H}_3$-$CH_2$-$CH_2$-$CH_2$-

EXEMPLE 43

Acide acétoxy-2 [((n-butyl-2 oxo-5 spirocyclopentane-4 imidazolin-2-yl-1) méthyl)-4 phényl]-2 phényl-2 acétique.

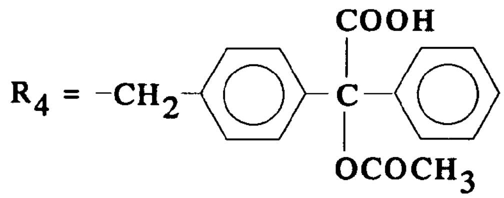

On dilue 200 mg du composé préparé à l'exemple précédent dans 5 ml de DMF et l'on ajoute, par petites quantités, sous azote, 33 mg d'hydrure de sodium : on laisse 20 minutes sous agitation à TA et l'on ajoute lentement 54 mg de chlorure d'acétyle dans 5 ml de DMF. Après 3 heures sous agitation, on dilue le milieu réactionnel par de l'eau et de l'acétate d'éthyle et l'on amène à pH 4 par une solution de $K_2SO_4$-$KHSO_4$. On extrait à l'acétate d'éthyle, lave la phase organique à l'eau puis par une solution saturée de chlorure de sodium ; on sèche sur sulfate de sodium et concentre. Le résidu est repris dans de l'éther ; le précipité formé est essoré puis séché sous vide.

m = 217 mg.

Fc = 147°C.

- RMN :

7,1-7,6 ppm : m : 9 H : Aromatiques

4,7 ppm : s : 2 H : -N-$C\underline{H}_2$-$C_6H_4$-

2,35 ppm : t : 2 H : $CH_3$-$CH_2$-$CH_2$-$C\underline{H}_2$-

2,20 ppm : s : 3 H : CO-$C\underline{H}_3$

1,65-2 ppm : m : 8 H : cyclopentane

1,5 ppm : quint : 2H : $CH_3$-$CH_2$-$C\underline{H}_2$-$CH_2$-

1,3 ppm : sext : 2H : $CH_3$-$C\underline{H}_2$-$CH_2$-$CH_2$-

0,8 ppm : t : 3 H : $C\underline{H}_3$-$CH_2$-$CH_2$-$CH_2$-

EXEMPLE 44

Acide [((n-butyl-2 oxo-5 spirocyclopentane-4 imidazolin-2-yl-1)méthyl)-4 phényl]-2 méthoxy-2 phényl-2 acétique.

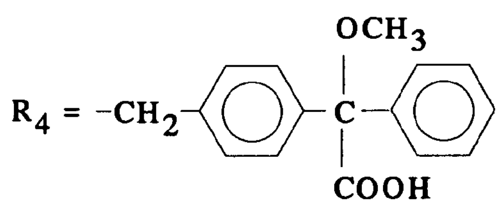

A) Ester méthylique de l'acide p-bromométhylphényl-2 méthoxy-2 phényl-2 acétique.

Ce composé est préparé par la méthode habituelle à partir de l'acide p-tolyl-2 méthoxy-2 phénylacétique obtenu à l'exemple 42, étape B.

B) Ester méthylique de l'acide [((n-butyl-2 oxo-5 spirocyclopentane-4 imidazolin-2-yl)-1 méthyl)-4 phényl]-2 méthoxy-2 phényl-2 acétique.

Ce composé est obtenu par la méthode habituelle en additionnant le composé préparé à l'étape précédente et le chlorhydrate de n-butyl-2 spirocyclopentane-4 imidazoline-2 one-5.

C) Acide [((n-butyl-2 oxo-5 spirocyclopentane-4 imidazolin-2-yl)-1 méthyl)-4 phényl]-2 méthoxy-2 phényl-2 acétique.

On dissout 1,92 g du composé préparé à l'étape précédente dans 20 ml de méthanol et l'on ajoute 5,2 ml de soude 1N. Après 4 heures à reflux, on dilue le milieu réactionnel par de l'acétate d'éthyle et de l'eau puis l'on amène à pH 4 par addition d'acide chrlorhydrique N. La phase organique est décantée, lavée à l'eau, par une solution saturée de chlorure de sodium puis séchée sur sulfate de sodium et concentre. Le résidu est chromatographié sur silice en éluant par un mélange DCM/MeOH/AcOH (92/5/3 ; v/v/v). Le produit attendu est obtenu.

m = 1,014 g.

Fc = 67°C.

- RMN :

7,5 ppm : m : 9 H : Aromatiques
4,75 ppm : s : 2 H : -N-$C\underline{H}_2$-$C_6H_4$-
3,15 ppm : s : 3 H : O-$C\underline{H}_3$
2,4 ppm : t : 2 H : $CH_3$-$CH_2$-$CH_2$-$C\underline{H}_2$-
1,7-2 ppm : m : 8 H : cyclopentane
1,5 ppm : quint : 2H : $CH_3$-$CH_2$-$C\underline{H}_2$-$CH_2$-
1,3 ppm : sext : 2H : $CH_3$-$C\underline{H}_2$-$CH_2$-$CH_2$-
0,8 ppm : t : 3 H : $C\underline{H}_3$-$CH_2$-$CH_2$-$CH_2$-

EXEMPLE 45

Cyano-2 [((n-butyl-2 oxo-5 spirocyclopentane-4 imidazolin-2-yl)-1 méthyl)-4 phényl]-2 phénylacétonitrile.

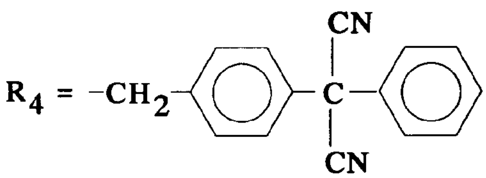

A) phényl p-tolylmalonitrile.

On chauffe pendant 2 heures à 120°C, un mélange de 14,7 g de méthyl-4 benzophénone et 16,4 g de pentachlorure de phosphore. On évapore à sec puis l'on dissout l'huile résiduelle dans 200 ml de DCM et l'on ajoute sous azote 19,3 g de cyanure de triméthylsilyle et 2,1 ml de tétrachlorure d'étain. Après 48 heures sous agitation à TA, on verse le milieu réactionnel dans un mélange eau-glace ; on filtre, on extrait 3 fois au chlorure de méthylène. On lave les phases organiques par une solution de carbonate acide de sodium, sèche puis évapore. Le résidu est chromatographié sur silice par un mélange hexane/acétone (5/1 ; v/v).

m = 9,8 g.

B) Cyano-2 [((n-butyl-2 oxo-5 spirocyclopentane-4 imidazolin-2-yl)-1 méthyl)-4 phényl]-2 phénylacétonitrile.

On opère en 2 étapes selon le procédé habituel pour préparer le produit attendu qui est purifié sur silice, en éluant par un mélange toluène/AcOEt (3/1 ; v/v).

- RMN :

0,8 ppm : t : 3 H : $CH_3$ (nBu)
1,2-1,4 ppm : m : 2 H : $CH_2$ (nBu)
1,4-1,6 ppm : m : 2 H : $CH_2$ (nBu)
1,7-2 ppm : m : 8 H : cyclopentane
2,3-2,4 ppm : m : 2 H : $CH_2$ (nBu)
4,8 ppm : s : 2 H : N-$C\underline{H}_2$-$C_6H_4$-
7,35-7,45 et 7,6-7,7 ppm : m : 9 H : Aromatiques

- IR (DCM)

2200 $cm^{-1}$ (CN)

EXEMPLE 46

Trifluoroacétate de l'acide [(((n-butyl-2 oxo-5 spirocyclopentane-4 imidazolin-2-yl-1) méthyl)-4 phényl)-4 benzoyl]-2 benzoïque.

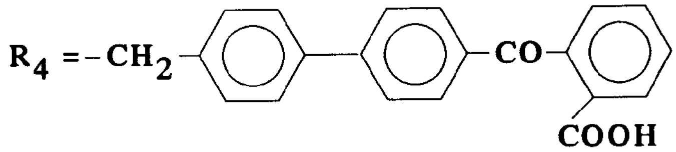

A) Acide [(p-tolyl)-4 benzoyl]-2 benzoïque.

On prépare une solution contenant 9 g de méthyl-4 biphényle et 7,5 g d'anhydride phtalique dans 50 ml d'orthodichlorobenzène et l'on ajoute 13,5 g de trichlorure d'aluminium. Après une nuit d'agitation à 100°C, on coule le milieu réactionnel sur 500 ml d'eau et de glace et 100 ml d'acide chlorhydrique concentré. La phase organique est décantée et diluée à l'hexane. Le solide formé est dissous dans une solution aqueuse d'ammoniaque à 8%, puis on lave à l'éther et l'on acidifie par de l'acide chlorhydrique concentré en présence de glace. Le solide formé est essoré, lavé à l'eau, séché à 60°C sous vide, puis il est finement pulvérisé dans l'éther, essoré et séché.

m = 5,8 g.

Fc = 260°C.

B) *tert*-butylate de l'acide [(p-tolyl)-4 benzoyl]-2 benzoïque.

5 g de l'acide préparé à l'étape précédente sont mis en suspension dans 30 ml de chlorure de thionyle, laissés 20 heures sous agitation à TA puis chauffés 6 heures à 50°C. La solution obtenue est évaporée à sec, le résidu est dissous dans le toluène puis évaporé ; on recommence une fois l'opération dans le toluène à 80°C. Le résidu est dissous dans 30 ml de THF, refroidi au bain de glace et traité sous agitation énergique par 1,35 g de *tert*-butylate de potassium ajouté progressivement de façon à maintenir la température à 15-20°C. Après 1 heure d'agitation à TA, le milieu est versé sur de l'eau, extrait à l'éther, lavé à l'eau salée ; les phase organiques sont séchées sur sulfate de sodium et évaporées à sec.

m = 3,4 g

C) trifluoroacétate de l'acide [(((n-butyl-2 oxo-5 spirocyclopentane-4 imidazolin-2-yl-1) méthyl)-4 phenyl)-4 benzoyl]-2 benzoïque.

On procède ensuite selon les méthodes habituelles pour préparer le produit attendu.

Fc = 77-85°C.

EXEMPLE 47

Acide [((n-butyl-2 oxo-5 spirocyclopentane-4 imidazolin-2-yl-1) méthyl)-4 n-propyl-2 phénoxy]-2 phénylacétique.

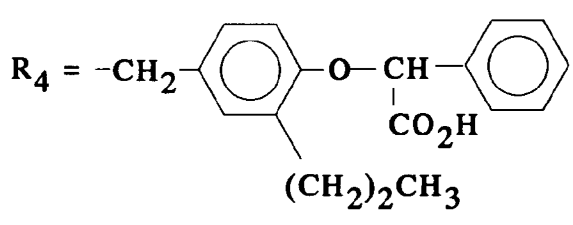

A) Ester éthylique de l'acide [bromométhyl-4 n-propyl-2 phénoxy]-2 phénylacétique.

Cet ester est préparé en suivant le mode opératoire décrit dans la demande de brevet international WO 91-12001.

B) Ester éthylique de l'acide [((n-butyl-2 oxo-5 spirocyclopentane-4 imidazolin-2-yl-1) méthyl)-4 n-propyl-2 phénoxy]-2 phénylacétique

Cet ester est préparé en suivant le procédé habituel et purifié par chromatographie sur silice en élant par un mélange DCM/AcOEt (9/1 ; v/v).

C) Acide [((n-butyl-2 oxo-5 spirocyclopentane-4 imidazolin-2-yl-1) méthyl)-4 n-propyl-2 phénoxy]-2 phénylacétique.

80 mg du composé obtenu à l'étape précédente sont agités 2 heures à TA en présence de 4 ml de méthanol, 1 ml d'eau et 15 mg de soude. Après évaporation des solvants, on reprend le milieu par un mélange eau/éther puis on acidifie la phase aqueuse jusqu'à pH 5 et on extrait à l'acétate d'éthyle. Le produit obtenu est lavé par une solution saturée de chlorure de sodium puis purifié dans l'heptane.

- RMN :

0,6-2,1 ppm : m : 20 H : $-CH_2-C\underline{H}_2-C\underline{H}_3$ (propyl)
$-CH_2-C\underline{H}_2-C\underline{H}_2-C\underline{H}_3$ + cyclopentane
2,4 ppm : t : 2 H : $-C\underline{H}_2-CH_2-CH_2-CH_3$
2,7 ppm : t : 2 H : $-C\underline{H}_2-CH_2-CH_3$ (propyl)
4,4 ppm : s : 2 H : $-N-C\underline{H}_2-C_6H_3-$
5,9 ppm : s : 1 H : $-OC\underline{H}-$
6,7-7,8 ppm : m : 8 H: Aromatiques

EXEMPLE 48

Acide [((n-butyl-2 oxo-5 spirocyclopentane-4 imidazolin-2-yl-1) méthyl)-4' biphényl-4-yl]-4 oxo-4 butyrique.

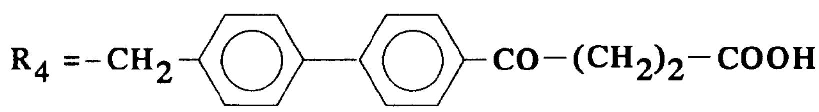

A) Acide (méthyl-4' biphényl-4-yl)-4 oxo-4 butyrique.

Ce composé est préparé d'après les méthodes décrites par E. Burker dans Bull. Soc. Chim. Fr., 1888, 449 et Weizmann et al. dans Chem. Ind., 1940, 402.

6,5 g de chlorure d'aluminium anhydre sont mis en suspension dans 50 ml de dichloroéthane. On ajoute 3,2 g d'anhydride succinique et 4,5 g de méthyl-4 biphényle. Après 24 heures d'agitation à TA, on verse le milieu réactionnel sur 400 ml d'un mélange eau/glace contenant 50 ml d'HCl concentré. Le solide formé est essoré, lavé à l'eau et séché à 60°C sous vide.

m = 6,5 g.
Fc = 188°C.

B) Ester méthylique de l'acide (méthyl-4' biphényl-4-yl)-4 oxo-4 butyrique.

2,85 g de l'acide préparé ci-dessus sont dissous dans 30 ml de DMF et agités pendant 20 minutes à TA avec 3,63 g de carbonate de cesium, puis pendant 3 heures à TA avec 2,4 ml d'iodure de méthyle. L'excès d'iodure de méthyle est évaporé sous vide puis le milieu réactionnel est versé sur 100 g d'un mélange eau/glace. Le solide formé est essoré, dissous dans AcOEt, traité au charbon actif, séché sur sulfate de sodium et évaporé à sec.

m = 1,9 g.

C) Ester méthylique de l'acide (bromométhyl-4' biphényl-4-yl)-4 oxo-4 butyrique.

Ce composé est préparé selon les méthodes habituelles.

D) Ester méthylique del'acide [((n-butyl-2 oxo-5 spirocyclopentane-4 imidazolin-2-yl-1) méthyl)-4′ biphényl-4-yl]-4 oxo-4 butyrique.

Ce composé est préparé selon les méthodes habituelles.

E) Acide [((n-butyl-2 oxo-5 spirocyclopentane-4 imidazolin-2-yl-1) méthyl)-4′ biphényl-4-yl]-4 oxo-4 butyrique.

900 mg de l'ester préparé ci-dessus sont dissous dans 7,5 ml de méthanol, 1,5 ml de potasse et 1 ml d'eau. Après 1 heure 40 minutes d'agitation à TA, le milieu réactionnel est dilué par 25 ml d'eau, lavé 2 fois par AcOEt et neutralisé par 4,8 ml d'HCl N. Le précipité formé est extrait par AcOEt puis on filtre l'insoluble, sèche sur sulfate de sodium et évapore le solvant. Le résidu solide est repris dans l'éther, essoré et séché.

m = 580 mg.
Fc = 167°C.

EXEMPLE 49

n-butyl-2-spirocyclopentane-4-[(N-(tétrazol-5-yl)-anilino)-4-benzyl]-1-imidazolin-2-one-5.

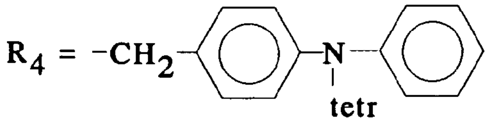

A) N-phényl N-(p-tolyl) urée.

Ce composé est préparé selon J.R. Robinson dans Can. J. Chem., 1954, 32, 901-905.

2,92 g de la N-(p-tolyl) aniline décrite à l'exemple 8, étape A) sont dissous dans 30 ml d'acide acétique ; on ajoute chaque 24 heures, pendant 3 jours, 1,6 g d'isocyanate de potassium. On ajoute ensuite de la lessive de soude pour atteindre pH 5 et 200 ml d'eau. Après refroidissement, le solide obtenu est essoré, rincé par de l'eau, séché sur anhydride phosphorique.

m = 3,4 g
Fc = 141-145°C.

B) N-cyano N-(p-tolyl) aniline.

Ce composé est préparé selon la méthode décrite par J.R. Robinson (réf. citée).

3,33 g du composé préparé précédemment sont dissous dans 22 ml de pyridine et traités par 8,4 g de chlorure de tosyle à 100°C. Après 1 heure, le milieu réactionnel est versé dans de l'eau. On extrait par AcOEt puis on lave par une solution de $KHSO_4$-$K_2SO_4$, par une solution saturée de NaCl, une solution saturée de $NaHCO_3$, une solution saturée du NaCl, puis on sèche. Le résidu est chromatographié sur silice en éluant par un mélange hexane/DCM (5/5 ; v/v). On obtient 2,2 g d'une huile qui cristallise au réfrigérateur.

Fc = 36-37°C.

C) N-cyano N-(bromométhyl-4 phényl) aniline.

Ce composé est préparé selon les méthodes habituelles.

D) n-butyl-2-spirocyclopentane-4-[(N-cyano-anilino)-4-benzyl]-1-imidazolin-2-one-5.

Ce composé est préparé selon les méthodes habituelles.

Fc = 75-78°C.

E) n-butyl-2-spirocyclopentane-4-[(N-(tétrazol-5-yl)-anilino)-4-benzyl]-1-imidazolin-2-one-5.

On transforme, selon les méthodes habituelles, le groupe cyano du composé obtenu à l'étape précédente en groupe tétrazolyle.

Fc = 175-178°C.

- RMN :

0,85 ppm : t : 3 H : $CH_3$(nBu)
1,3 ppm : sext : 2H : $CH_3$-C<u>H</u>$_2$-$CH_2$-$CH_2$-
1,5 ppm : quint : 2H : $CH_3$-$CH_2$-C<u>H</u>$_2$-$CH_2$-
1,6-2 ppm : m : 8 H : cyclopentane
2,4 ppm : t : 2 H : $CH_3$-$CH_2$-$CH_2$-C<u>H</u>$_2$-
4,7 ppm : s : 2 H : N-C<u>H</u>$_2$-$C_6H_4$-
7,2-7,5 ppm : m : 9 H : Aromatiques

EXEMPLE 50

N-phényl N-[((n-butyl-2 spirocyclopentane-4 oxo-5 imidazolin-2-yl-1) méthyl)-4 phényl] méthoxycarboxamidine.

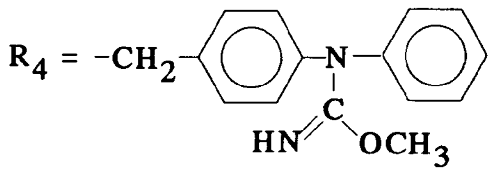

Ce composé est préparé selon la méthode décrite par A. Bonetti et E. Bellora dans J. Org. Chem., 1972, <u>37</u> (21), 3352.

670 mg du composé préparé à l'exemple 49, étape D), sont mis en solution dans 10 ml de méthanol et chauffés par 109 mg de cyanure de potassium. Après 12 heures, on concentre le milieu et extrait par AcOEt ; la phase organique est lavée à l'eau, par une solution de NaCl concentrée puis séchée sur $Na_2SO_4$.

m = 670 mg.

- RMN :

0,8 ppm : t : 3 H : $CH_3$ (nBu)
1,25 ppm : sext : 2H : $CH_3$-C<u>H</u>$_2$-$CH_2$-$CH_2$-
1,5 ppm : quint : 2H : $CH_3$-$CH_2$-C<u>H</u>$_2$-$CH_2$-
1,6-2 ppm : m : 8 H : cyclopentane
2,4 ppm : t : 2 H : $CH_3$-$CH_2$-$CH_2$-C<u>H</u>$_2$-
3,7 ppm : s : 3 H : $OCH_3$-
4,7 ppm : s : 2 H : N-C<u>H</u>$_2$-$C_6H_4$-
5,8 ppm : s : 1 H : -N<u>H</u>
7,2-7,5 ppm : m : 9 H : Aromatiques

EXEMPLE 51

n-butyl-2 spirocyclopentane-4 [(N-(oxo-5-oxadiazol-1,2,4-yl-3)-anilino)-4 benzyl]-1-imidazolin-2-one-5.

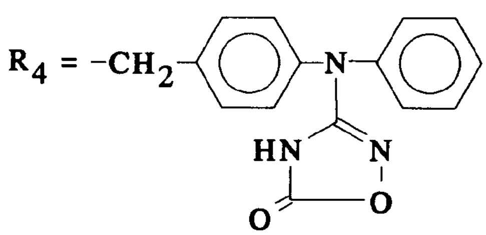

L'oxo-5 oxadiazole-1,2,4 est préparé selon M.A. Perez et al., Synthesis, 1983, 483.

A) N'-acétyl N-phényl N-[((n-butyl-2 spirocyclopentane-4 oxo-5 imidazolin-2-yl-1) méthyl)-4 phényl] méthoxycarboxamidine.

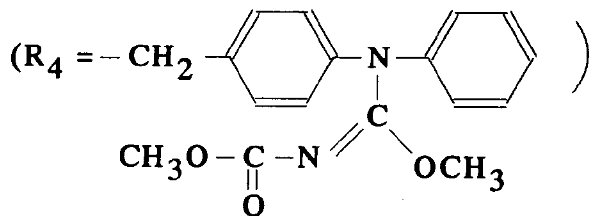

716 mg du composé préparé à l'exemple 50 sont dissous dans 10 ml de DCM et traités à TA par 195 µl de chlorure d'acétyle en présence de 292 µl de collidine. Après 24 heures, le milieu réactionnel est concentré et le résidu est chromatographié sur silice en éluant par un mélange heptane/acétone (75/25 ; v/v).

m = 500 mg.

B) n-butyl-2 spirocyclopentane-4 [(N-(oxo-5-oxadiazol-1,2,4-yl-3)-anilino)-4 benzyl]-1-imidazolin-2-one-5.

Une solution de 77 mg de chlorure d'hydroxylamine dans 5 ml de méthanol est traitée pendant 15 minutes par 60 mg de méthylate de sodium ; on ajoute ensuite 490 mg du composé obtenu à l'étape précédente dissous dans 10 ml de méthanol. Après une nuit de chauffage à reflux, le milieu est concentré puis purifié par chromatographie sur silice en éluant par un mélange heptane/acétone/acide acétique (80/20/1). Après trituration dans un mélange éther/hexane, on obtient une poudre blanche.

m = 70 mg.

Fc = 187-190°C.

- RMN :

0,85 ppm : t : 3 H : $CH_3$ (nBu)

1,3 ppm : sext : 2H : $CH_3$-$C\underline{H}_2$-$CH_2$-$CH_2$-

1,55 ppm : quint : 2H : $CH_3$-$CH_2$-$C\underline{H}_2$-$CH_2$-

2,40 ppm : t : 2 H : $CH_3$-$CH_2$-$CH_2$-$C\underline{H}_2$-

1,6-2 ppm : m : 8 H : cyclopentane

4,75 ppm : s : 2 H : N-$C\underline{H}_2$-$C_6H_4$-

7,2-7,5 ppm : m : 9 H : Aromatiques

12,2 ppm : s : 1 H : -N$\underline{H}$

EXEMPLE 52

Trifluoroacétate de l'acide [((n-butyl-2 cyclohexyl-4 méthyl-4 oxo-5 imidazolin-2-yl-1) méthyl)-4 phényl]-2 phénylacétique.

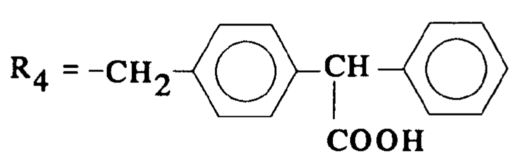

A) Cyclohexyl-5 méthyl-5 hydantoïne.

Ce composé est préparé selon J. Org. Chem., 1960, 25, 1920-1924.

50 g de cyclohexylméthylcétone, dissous dans 400 ml d'alcool 95, sont ajoutés en 30 minutes à 29,4 g de cyanure de sodium et 192 g de carbonate d'ammonium dans 400 ml d'eau. On chauffe à 55-60°C pendant 4 heures puis on évapore de moitié sous vide et on laisse une nuit à +4°C. Le précipité formé est filtré, lavé à l'eau, puis séché sous vide sur anhydride phosphorique. On obtient 65,5 g de l'hydantoïne attendue, identifiée par ses spectres IR et RMN.

Fc = 220°C.

B) Acide amino-2 cyclohexyl-2 propionique.

Ce composé est préparé selon J. Org. Chem., 1960, 25, 1920-1924.

Un mélange contenant 7 g de l'hydantoïne, préparée à l'étape précédente et 28 g d'octahydrate d'hydroxyde de baryum dans 150 ml d'eau,est porté à 160°C pendant 5 heures, dans un tube d'acier. Le milieu réactionnel est saturé avec de la carboglace ; l'insoluble formé est filtré, puis le filtrat est concentre sous vide. Le résidu solide est repris dans de l'acétone, filtré et séché. On obtient 5,25 g de l'acide attendu, identifié par ses spectres IR et RMN. Le produit fond en se décomposant à 350°C.

C) Ester éthylique de l'acide amino-2 cyclohexyl-2 propionique.

3 g de l'acide préparé à l'étape précédente sont ajoutés à 40 ml d'alcool absolu saturé de gaz chlorhydrique, puis on chauffe à reflux, sous agitation, pendant 20 heures. Le milieu réactionnel est évaporé sous vide et le résidu est repris dans un mélange éther/eau que l'on amène à pH 9 par addition d'une solution saturée de bicarbonate de sodium. La phase organique est décantée, lavée par une solution saturée de chlorure de sodium puis évaporée sous vide. On obtient 2,1 g de l'ester attendu sous forme d'huile. Identification par spectres IR et RMN.

D) Valérimidate d'éthyle.

Ce composé est préparé sous forme de chlorhydrate selon Mac Elvain (J. Am. Chem. Soc., 1942, 64, 1825-2827). Il est libéré de son chlorhydrate par action du carbonate de potassium, puis extrait par le DCM.

E) n-butyl-2 cyclohexyl-4 méthyl-4 imidazolin-2 one-5.

2 g de l'ester préparé à l'étape C) et 2,35 g de valérimidate d'éthyle sont mélangés dans 6 ml de xylène auxquels on ajoute 6 gouttes d'acide acétique ; on porte à reflux pendant 6 heures. Le milieu réactionnel est ensuite concentré sous vide et le résidu est chromatographié sur gel de silice fine en éluant par un mélange chloroforme/méthanol/acide acétique (95/9/3 ; v/v/v). Les fractions contenant le produit désiré sont rassemblées puis évaporées sous vide ; le résidu est repris par un mélange acétate d'éthyle/eau et on amène à pH 9 par addition d'une solution de soude. La phase organique est décantée, lavée à l'eau, puis par une solution saturée de chlorure de sodium, séchée sur sulfate de sodium, puis évaporée à sec. On obtient le produit attendu sous forme d'une huile épaisse qui se prend en masse pour donner un solide amorphe.

m = 1,56 g

- IR : (chloroforme)
  - 1720 $cm^{-1}$ C=O
  - 1640 $cm^{-1}$ C=N
- RMN
  - 1,13 ppm : s : 3 H : $CH_3$ en 4 de l'imidazolinone.

F) Acide p-tolyl phénylacétique.

Cet acide est préparé à partir de la p-tolylaldéhyde selon le procédé cité par M.E. Grundy et al. dans J. Chem. Soc., 1960, 372-376.

G) Ester *tert*-butylique de l'acide p-bromométhylphényl-2 phénylacétique.

Ce composé est préparé selon l'exemple 1, étapes B et C.

H) Ester *tert*-butylique de l'acide [((n-butyl-2 cyclohexyl-4 méthyl-4 oxo-5 imidazolin-2-yl-1) méthyl)-4 phényl]-2 phénylacétique.

Ce composé est préparé selon les méthodes habituelles.

- IR (chloroforme)
  - 1720-1725 $cm^{-1}$ C = O : imidazoline et ester
  - 1635 $cm^{-1}$ C = N : imidazoline

I) Trifluoroacétate de l'acide [((n-butyl-2 cyclohexyl-4 méthyl-4 oxo-5 imidazolin-2-yl-1) méthyl)-4 phényl]-2 phénylacétique.

L'ester *tert*-butylique obtenu à l'étape précédente est déprotégé par le TFA selon la méthode habituelle. Ce produit est obtenu sous forme d'une mousse.

- RMN :

  7,1-7,45 ppm : m : 9 H : Aromatiques
  5,1 ppm : s : 1 H : $C\underline{H}-CO_2H$
  4,7 ppm : s : 2 H : $N-C\underline{H_2}-C_6H_4-$
  2,4 ppm : t : 2H : $C\underline{H_2}-CH_2-CH_2-CH_3$
  0,9-1,9 ppm : m : 11 H : cyclohexyle
  + 4 H : $CH_3-C\underline{H_2}-C\underline{H_2}-CH_3$
  1,25 ppm : s : 3 H : $CH_3$ en 4 de l'imidazolinone
  0,8 ppm : t : 3 H : $CH_2-CH_2-CH_2-C\underline{H_3}$

EXEMPLE 53

n-butyl-2 spirocyclopentane-4 [(α-(tétrazol-5-yl) benzyloxy)-4 benzyl]-1 imidazoline-2 one-5.

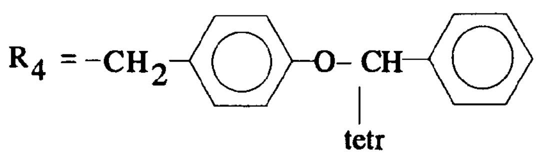

A) Chlorure de l'acide [((n-butyl-2 oxo-5 spirocyclopentane-4 imidazolin-2-yl-1) méthyl)-4 phénoxy]-2 phényl-2 acétique.

On mélange 163 mg de l'acide préparé à l'exemple 32 et 0,5 ml de chlorure de thionyle dans 5 ml de DCM et l'on agite à TA pendant 18 heures. On concentre à sec, reprend par du toluène puis concentre à nouveau et reprend par de l'éther. Après concentration du solvant, le résidu est utilisé tel quel à l'étape suivante.

B) [((n-butyl-2 oxo-5 spirocyclopentane-4 imidazolin-2-yl-1) méthyl)-4 phenoxy]-2 phényl-2 acétique.

On reprend le composé préparé précédemment dans 4 ml de THF et on introduit la solution dans un cylindre métallique ; on introduit 6 ml d'ammoniac gazeux préalablement liquéfié et on ferme le cylindre métallique. Après 18 heures à TA, on concentre, reprend par de la soude diluée et extrait à l'acétate d'éthyle. Après lavage à l'eau, on effectue une chromatographie sur colonne de silice en éluant par un mélange DCM/MeOH (97/3, v/v). On obtient 100 mg du produit attendu.

C) n-butyl-2 [((α-cyanobenzyloxy)-4 benzyl]-1 spirocyclopentane-4 imidazoline-2 one-5.

On porte à reflux pendant 4 heures un mélange de 120 mg du composé obtenu précédemment et 6 ml d'oxychlorure de phosphore. On concentre le milieu réactionnel, extrait à l'acétate d'éthyle puis lave à l'eau. Le produit obtenu est utilisé tel quel à l'étape suivante.

D) n-butyl-2 spirocyclopentane-4 [(α-(tétrazol-5-yl) benzyloxy)-4 benzyl]-1 imidazoline-2 one-5.

On porte à reflux pendant 96 heures, un mélange contenant 80 mg du produit préparé à l'étape précédente, 10 ml de toluène et 129 mg d'azide de tributylétain. Après refroidissement, on extrait par une solution de soude 1N, puis on lave à l'éther. La phase aqueuse est acidifiée par une solution d'acide chlrorhydrique 3N. On extrait à l'acétate d'éthyle, lave à l'eau, puis on chromatographie sur silice en éluant par un mélange DCM/MeOH/AcOH (97/3/0,5 ; v/v/v). On obtient 19 mg du produit attendu.

- Spectre de masse : $MH^+ = 459$
- RMN :

  0,72 ppm : t : 3 H : $C\underline{H_3}-CH_2-CH_2-CH_2-$
  1,2 ppm : sext : 2H : $CH_3-C\underline{H_2}-CH_2-CH_2-$

1,4 ppm : quint : 2H : $CH_3$-$CH_2$-C<u>H</u>$_2$-$CH_2$-
1,5-1,9 ppm : m : 8 H : cyclopentane
2,3 ppm : t : 2 H : $CH_3$-$CH_2$-$CH_2$-C<u>H</u>$_2$-
4,55 ppm : s : 2 H : N-C<u>H</u>$_2$-$C_6H_4$-
6,95 ppm : s : H : C<u>H</u>-tetr
7-7,6 ppm : m : 9 H : Aromatiques

## Revendications

**1.** Composé de formule :

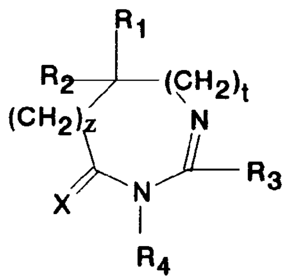

(I)

dans laquelle :

- $R_1$ et $R_2$ représentent chacun indépendamment un alkyle en $C_1$-$C_6$, un cycloalkyle en $C_3$-$C_7$ un phényle, un phénylalkyle dans lequel l'alkyle est en $C_1$-$C_3$, lesdits groupes alkyle, phényle et phénylalkyle étant non substitués ou substitués par un ou plusieurs atomes d'halogène ou par un groupe choisi parmi un perfluoroalkyle en $C_1$-$C_4$, un hydroxyle, un alcoxy en $C_1$-$C_4$ ;
- ou $R_1$ et $R_2$ ensemble forment un groupe de formule $=CR'_1R'_2$, dans laquelle $R'_1$ représente l'hydrogène, un alkyle en $C_1$-$C_4$ ou un phényle, et $R'_2$ représente un alkyle en $C_1$-$C_4$ ou un phényle;
- ou encore $R_1$ et $R_2$ liés ensemble représentent, soit un groupe de formule $-(CH_2)_n-$, soit un groupe de formule $-(CH_2)_pY(CH_2)_q-$, dans lequel Y est, soit un atome d'oxygène, soit un atome de soufre, soit un groupe CH substitué par un groupe alkyle en $C_1$-$C_4$ un phényle ou un phénylalkyle dans lequel l'alkyle est en $C_1$-$C_3$ soit un groupe N-$R_5$ dans lequel $R_5$ représente un hydrogène, un alkyle en $C_1$-$C_4$, un phénylalkyle dans lequel l'alkyle est en $C_1$-$C_3$, un alkylcarbonyle en $C_1$-$C_4$, un halogénoalkylcarbonyle en $C_1$-$C_4$, un polyhalogénalkylcarbonyle en $C_1$-$C_4$, un benzoyle, un alpha aminoacyle ou un groupe N-protecteur, ou $R_1$ et $R_2$ liés ensemble avec l'atome de carbone auquel ils sont liés constituent un indane ou un adamantane ;
- $R_3$ représente un hydrogène, un alkyle en $C_1$-$C_6$, non substitué ou substitué par un ou plusieurs atomes d'halogène ; un alcényle en $C_2$-$C_6$ ; un cycloalkyle en $C_3$-$C_7$ ; un phényle ; un phénylalkyle dans lequel l'alkyle est en $C_1$-$C_3$ ; un phénylalcényle dans lequel l'alcényle est en $C_2$-$C_3$, lesdits groupes phényles étant non substitués ou substitués une ou plusieurs fois par un atome d'halogène, un alkyle en $C_1$-$C_4$, un halogénoalkyle en $C_1$-$C_4$, un polyhalogénoalkyle en $C_1$-$C_4$, un hydroxyle ou un alcoxy en $C_1$-$C_4$;
- $R_4$ représente un groupement choisi parmi :

a) 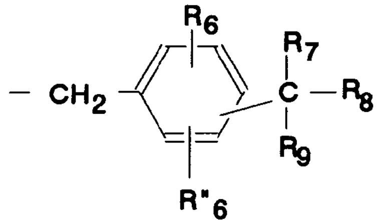 ,

b) 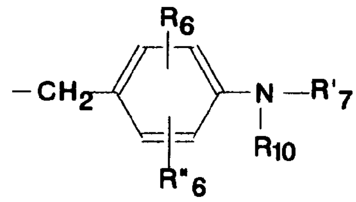

c) 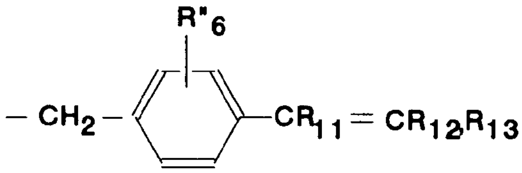 ,

d) 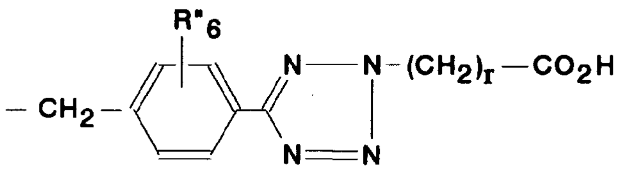 ,

e) 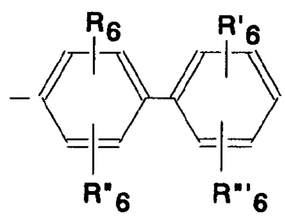 ,

f) 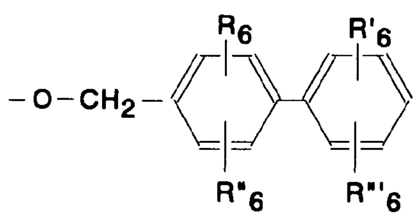 ,

g) 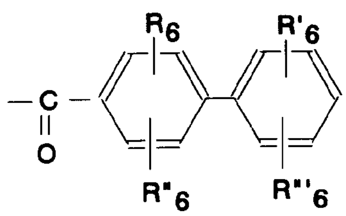 ,

h) 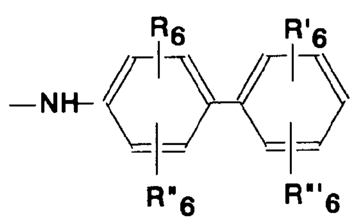 ,

j) 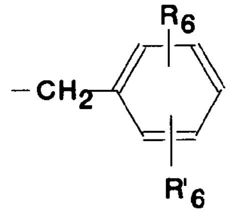

k) 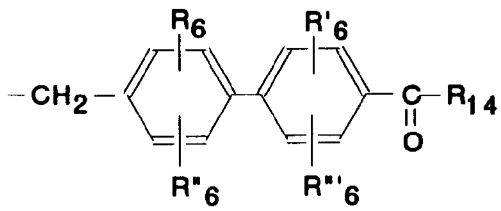

l) 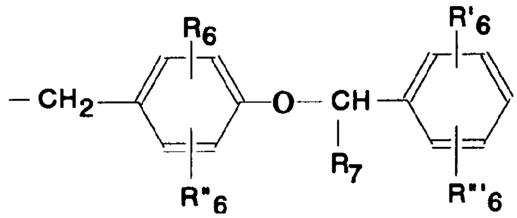 ,

- $R''_6$ et $R'''_6$ sont semblables ou différents et représentent chacun indépendamment l'hydrogène, un atome de chlore ou un groupe choisi parmi un alkyle en $C_1$-$C_6$, un alcoxy en $C_1$-$C_4$, un amino, un aminométhyle;
- $R_6$ et $R'_6$ sont semblables ou différents et représentent chacun indépendamment un atome d'hydrogène, un carboxy, un alcoxycarbonyle dans lequel l'alcoxy est en $C_1$-$C_4$, un cyano, un tétrazolyle -5, un méthyltétrazolyle-5 ou un 4H-oxo-5 oxadiazol-1,2,4 yle-3, ou un 4H-dioxo-3,5 oxadiazole-1, 2, 4 yle-2 à la condition que pour les groupes e, f, g, h, j, au moins un des substituants $R_6$ ou $R'_6$ soit différent de l'hydrogène ;
- $R_7$ représente un alkyle en $C_1$-$C_4$ ou un groupe choisi parmi :

i) $-(CH_2)_r-CO_2H$ ,

ii) 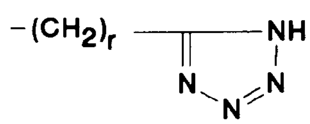 ,

iii) 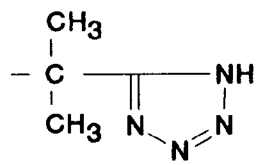 ,

iv) 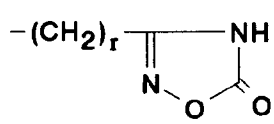 ,

v) 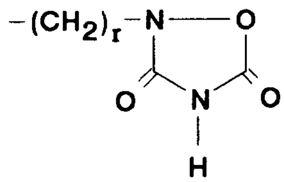

vi) 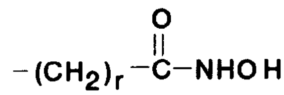

vii) $-(CH_2)_r-CN$

viii) $-OCOCH_3$

- R'$_7$ représente un alkyle en $C_1$-$C_4$ ou un groupe choisi parmi :

$-(CH_2)_s-CO_2H$ ,

$-(CH_2)_r-CN$

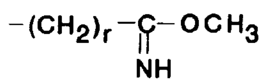

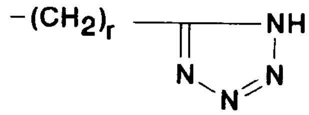 ,

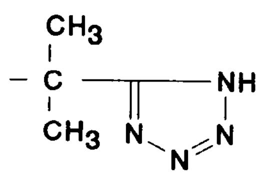 ,

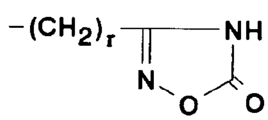

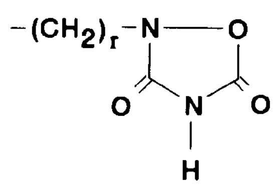

- $R_8$ est identique à $R_7$, ou bien $R_8$ représente l'hydrogène, un atome d'halogène, un alkyle en $C_1$-$C_4$, un cycloalkyle en $C_3$-$C_7$, un phényle substitué par R'$_6$, un benzyle dans lequel le phényle est substitué par R'$_6$, un hydroxyle, un alcoxy en $C_1$-$C_4$, un cyano, un groupe $OCOR_{15}$ dans lequel $R_{15}$ représente un alkyle en $C_1$-$C_4$, un halogénoalkyle en $C_1$-$C_4$ ou un phényle substitué par R'$_6$ ;

- $R_9$ est l'hydrogène ou $R_9$ est identique à $R_8$, avec la limitation que $R_9$ ne peut pas être identique à $R_7$ ;
- ou $R_8$ et $R_9$ ensemble avec l'atome de carbone auquel ils sont liés constituent un cycloalkyle en $C_3$-$C_7$ ou un groupe carbonyle ;
- $R_{10}$ représente l'hydrogène, un alkyle en $C_1$-$C_4$, un cycloalkyle en $C_3$-$C_7$, un phényle substitué par $R'_6$ ou un benzyle dont le phényle est substitué par $R'_6$ ;
- $R_{11}$ représente l'hydrogène, un alkyle en $C_1$-$C_6$, un phényle, un carboxy, un alcoxycarbonyle dans lequel l'alcoxy est en $C_1$-$C_4$, un tétrazolyle-5 ou un 4H-oxo-5 oxadiazol-1, 2, 4 yle-3 ou un 4H-dioxo-3,5 oxadiazole-1, 2, 4 yle-2 ;
- $R_{12}$ représente l'hydrogène, un alkyle en $C_1$-$C_6$, un cyano, un carboxy, un alcoxycarbonyle dans lequel l'alcoxy est en $C_1$-$C_4$, un tétrazolyle-5, un phényle substitué par $R'_6$ ou un benzyle dont le phényle est substitué par $R'_6$ ;
- $R_{13}$ représente l'hydrogène, un alkyle en $C_1$-$C_6$, un alcoxycarbonyle dans lequel l'alcoxy est en $C_1$-$C_4$, un phényle substitué par $R'_6$, un benzyle dont le phényle est substitué par $R'_6$, un cyano, un carboxy, un tétrazolyle-5, un 4H-oxo-5 oxadiazol-1, 2,4 yle-3, un 4H-dioxo-3,5-oxadiazole-1,2,4-yle-2, à la condition que $R_{11}$ et $R_{13}$ ne représentent pas simultanément l'un des groupes suivants : un carboxy, un tétrazolyle-5, un 4H-oxo-5 oxadiazol-1,2,4 yle-3 ou un 4H-dioxo-3,5 oxadiazole-1, 2, 4 yle-2 ; ou
- $R_{14}$ représente un carboxyalkyle en $C_1$-$C_4$, un phényle, un carboxyphényle ;
- $p + q = m$ ;
- n est un nombre entier compris entre 2 et 11 ;
- m est un nombre entier compris entre 2 et 5 ;
- r représente 0, 1 ou 2 ;
- s représente 1 ou 2 ;
- X représente un atome d'oxygène ou atome de soufre ;
- z et t sont nuls ou l'un est nul et l'autre représente 1 ;

avec la limitation que pour les valeurs a), b), k) et l) de $R_4$, $R_8$ représente l'hydrogène lorsque $R_7$, $R'_7$ ou $R_{14}$ contient soit un groupement carboxy, soit un groupement tétrazolyle-5, soit un groupement 4H-oxo-5 oxadiazol-1,2,4 yle-3 soit un groupement 4H-dioxo-3,5 oxadiazole-1, 2, 4 yle-2 ;

et ses sels.

**2.** Composé selon la revendication 1 dans lequel $R_1$ et $R_2$ liés ensemble constituent avec le carbone auquel ils sont liés un cyclopentane ou un cyclohexane.

**3.** Composé selon la revendication 1 dans lequel $R_1$ et $R_2$ représentent l'un un méthyle, l'autre un cyclohexyle.

**4.** Composé selon l'une quelconque des revendications 1 à 3 dans lequel $R_3$ représente un groupe alkyle linéaire en $C_1$-$C_6$.

**5.** Composé selon l'une quelconque des revendications 1 à 4 dans lequel X représente un atome d'oxygène.

**6.** Composé selon l'une quelconque des revendications 1 à 5 dans lequel $R_4$ représente un groupement a) de formule :

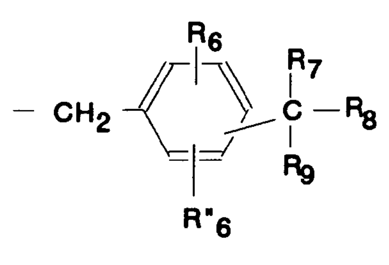

dans lequel $R_6$, $R''_6$, $R_7$, $R_8$ et $R_9$ sont tels que définis dans la revendication 1.

**7.** Composé selon la revendication 6 dans lequel $R_6$ et $R''_6$ représentent l'hydrogène et $R_7$ représente un groupe $-(CH_2)_r-COOH$ avec r=0,1 ou 2.

**8.** Procédé pour la préparation d'un composé de formule (I) selon la revendication 1, dans lequel $R_4$ à l'une des valeurs a), b), c), d), j), k) ou l), caractérisé en ce que :

a1) on fait réagir un dérivé hétérocyclique de formule :

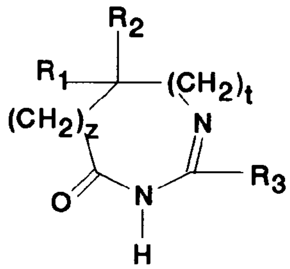

(2)

dans laquelle z, t, $R_1$ , $R_2$, et $R_3$ ont les significations indiquées ci-dessus pour (I) dans la revendication 1, sur un halogènure de formule :

Hal-$R'_4$ (3)

dans laquelle Hal représente un atome d'halogène, de préférence le brome, et $R'_4$ représente soit $R_4$, soit un groupement précurseur de $R_4$ ;

b1) éventuellement, le composé ainsi obtenu, de formule :

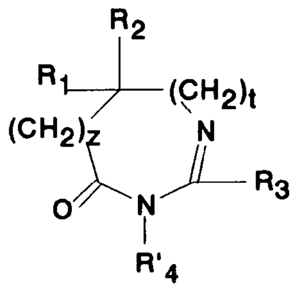

(4)

est traité par le réactif de Lawesson [bis(méthoxy-4 phényl)-2,4 dithia-1,3 diphosphétane-2,4 disulfure-2,4] ;

c1) le composé obtenu en a1) ou en b1), de formule :

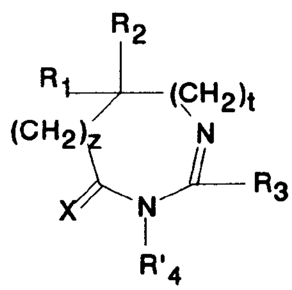

(5)

dans laquelle X représente un atome d'oxygène ou un atome de soufre, est éventuellement traité pour préparer le composé (I) par transformation du groupe $R'_4$ en $R_4$ ;

d1) le composé ainsi obtenu est ensuite éventuellement transformé en l'un de ses sels.

9. Procédé pour la préparation d'un composé selon la revendication 1, caractérisé en ce que

a2) on fait réagir un aminoacide de formule :

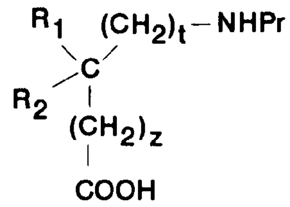

(7)

dans laquelle z, t, $R_1$ et $R_2$ ont les significations indiquées ci-dessus pour (I) dans la revendication 1 et dont la fonction amine est protégée par le groupe Pr, sur un dérivé de formule :

$H_2N\text{-}R'_4$ (8)

dans laquelle $R'_4$ représente soit $R_4$, soit un groupe précurseur de $R_4$ ;
b2) après déprotection de l'amine, le composé ainsi obtenu de formule :

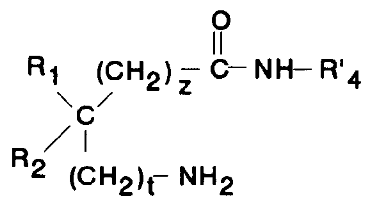

(9)

est traité par un ortho-ester d'alkyle de formule $R_3C(OR)_3$ (10) dans laquelle $R_3$ a la signification indiquée ci-dessus pour (I) dans la revendication 1 et R est un alkyle en $C_1$-$C_4$ ;
c2) éventuellement, le composé ainsi obtenu de formule :

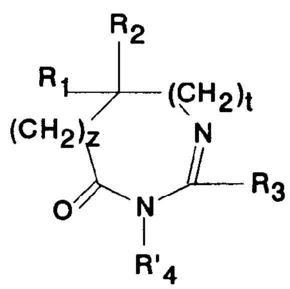

(4)

est traité par le réactif de Lawesson [bis(méthoxy-4 phényl)-2,4 dithia-1,3 diphosphétane-2,4 disulfure-2,4] ;
d2) le composé ainsi obtenu en b2) ou en c2) de formule :

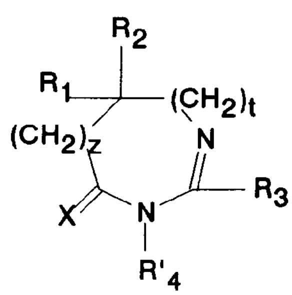

(5)

dans laquelle X représente un atome d'oxygène ou un atome de soufre, est ensuite éventuellement traité dans des conditions convenables pour préparer le composé (I) par transformation du groupe $R'_4$ en groupe $R_4$ ;
e2) le composé ainsi obtenu est ensuite éventuellement transformé en l'un de ses sels.

**10.** Procédé pour la préparation d'un composé de formule (I) selon la revendication 1, dans lequel $R_4$ repré-

sente le groupe g), caractérisé en ce que :

a1) on fait réagir un dérivé hétérocyclique de formule :

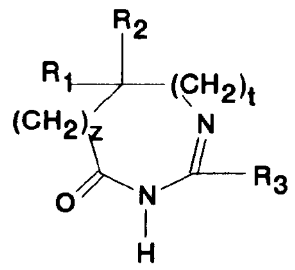

(2)

dans laquelle z, t, $R_1$ , $R_2$, et $R_3$ ont les significations indiquées ci-dessus pour (I) dans la revendication 1, sur un composé de formule :

$R'_4OH$ (3')

dans laquelle $R'_4$ représente soit $R_4$, soit un groupement précurseur de $R_4$ ;

b1) éventuellement, le composé ainsi obtenu, de formule :

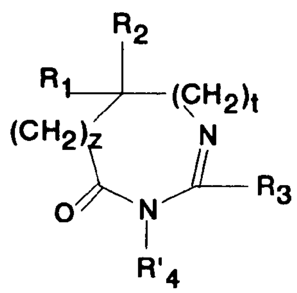

(4)

est traité par le réactif de Lawesson [bis(méthoxy-4 phényl)-2,4 dithia-1,3 diphosphétane-2,4 disulfure-2,4] ;

c1) le composé obtenu en a1) ou en b1), de formule :

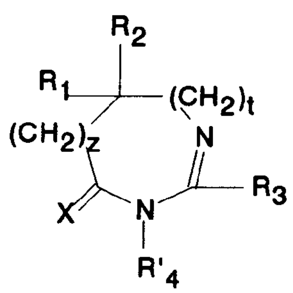

(5)

dans laquelle X représente un atome d'oxygène ou un atome de soufre, est éventuellement traité pour préparer le composé (I) par transformation du groupe $R'_4$ en $R_4$ ;

d1) le composé ainsi obtenu est ensuite éventuellement transformé en l'un de ses sels.

**11.** Composition pharmaceutique contenant à titre de principe actif un composé selon l'une quelconque des revendications 1 à 7.

**12.** Composition pharmaceutique contenant un composé selon l'une quelconque des revendications 1 à 7 en association avec un composé bêtabloquant.

**13.** Composition pharmaceutique contenant un composé selon l'une quelconque des revendications 1 à 7 en association avec un diurétique.

**14.** Composition pharmaceutique contenant un composé selon l'une quelconque des revendications 1 à 7 en association avec un antiinflammatoire non stéroïdien.

**15.** Composition pharmaceutique contenant un composé selon l'une quelconque des revendications 1 à 7 en association avec un antagoniste calcique.

**16.** Composition pharmaceutique contenant un composé selon l'une quelconque des revendications 1 à 7 en association avec un tranquillisant.

**Revendications pour l'Etat contractant suivant : ES.**

**1.** Procédé pour la préparation d'un composé de formule :

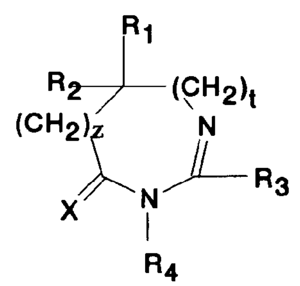

(I)

dans laquelle :

- $R_1$ et $R_2$ représentent chacun indépendamment un alkyle en $C_1$-$C_6$, un cycloalkyle en $C_3$-$C_7$ un phényle, un phénylalkyle dans lequel l'alkyle est en $C_1$-$C_3$, lesdits groupes alkyle, phényle et phénylalkyle étant non substitués ou substitués par un ou plusieurs atomes d'halogène ou par un groupe choisi parmi un perfluoroalkyle en $C_1$-$C_4$, un hydroxyle, un alcoxy en $C_1$-$C_4$ ;
- ou $R_1$ et $R_2$ ensemble forment un groupe de formule $=CR'_1R'_2$, dans laquelle $R'_1$ représente l'hydrogène, un alkyle en $C_1$-$C_4$ ou un phényle, et $R'_2$ représente un alkyle en $C_1$-$C_4$ ou un phényle;
- ou encore $R_1$ et $R_2$ liés ensemble représentent, soit un groupe de formule $-(CH_2)_n-$, soit un groupe de formule $-(CH_2)_pY(CH_2)_q-$, dans lequel Y est, soit un atome d'oxygène, soit un atome de soufre, soit un groupe CH substitué par un groupe alkyle en $C_1$-$C_4$ un phényle ou un phénylalkyle dans lequel l'alkyle est en $C_1$-$C_3$ soit un groupe N-$R_5$ dans lequel $R_5$ représente un hydrogène, un alkyle en $C_1$-$C_4$, un phénylalkyle dans lequel l'alkyle est en $C_1$-$C_3$, un alkylcarbonyle en $C_1$-$C_4$, un halogénoalkylcarbonyle en $C_1$-$C_4$, un polyhalogénalkylcarbonyle en $C_1$-$C_4$, un benzoyle, un alpha aminoacyle ou un groupe N-protecteur, ou $R_1$ et $R_2$ liés ensemble avec l'atome de carbone auquel ils sont liés constituent un indane ou un adamantane ;
- $R_3$ représente un hydrogène, un alkyle en $C_1$-$C_6$, non substitué ou substitué par un ou plusieurs atomes d'halogène ; un alcényle en $C_2$-$C_6$ ; un cycloalkyle en $C_3$-$C_7$ ; un phényle ; un phénylalkyle dans lequel l'alkyle est en $C_1$-$C_3$ ; un phénylalcényle dans lequel l'alcényle est en $C_2$-$C_3$, lesdits groupes phényles étant non substitués ou substitués une ou plusieurs fois par un atome d'halogène, un alkyle en $C_1$-$C_4$, un halogénoalkyle en $C_1$-$C_4$, un polyhalogénoalkyle en $C_1$-$C_4$, un hydroxyle ou un alcoxy en $C_1$-$C_4$ ;
- $R_4$ représente un groupement choisi parmi :

a) 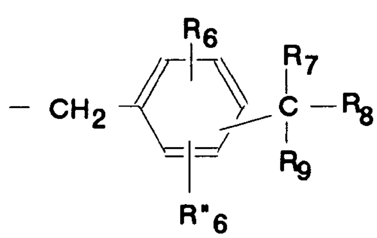 ,

b) 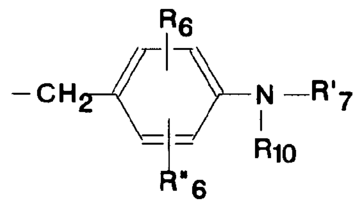

c) 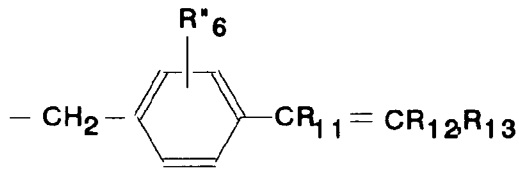 ,

d) 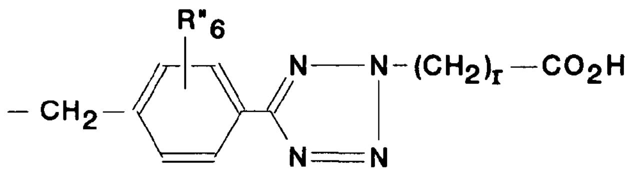 ,

e) 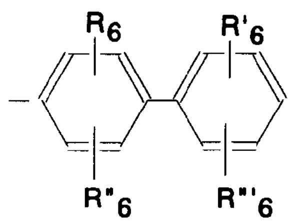 ,

f) 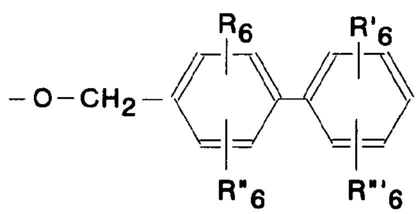 ,

g) 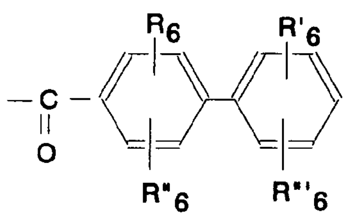 ,

h) 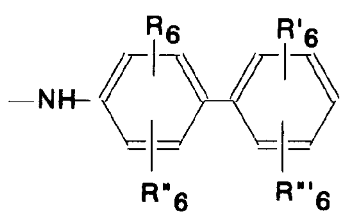 ,

j) 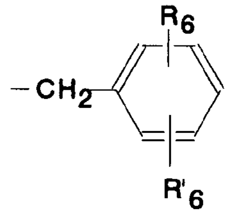

k) 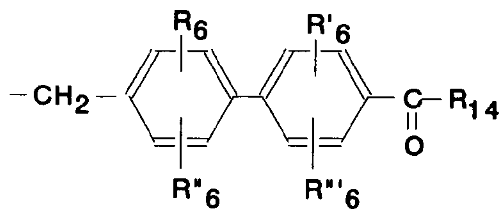

l) 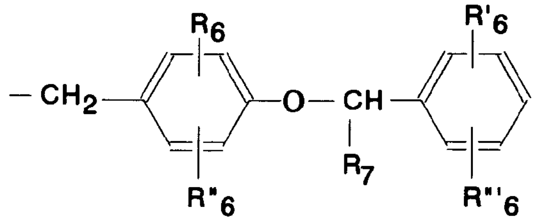 ,

- $R''_6$ et $R'''_6$ sont semblables ou différents et représentent chacun indépendamment l'hydrogène, un atome de chlore ou un groupe choisi parmi un alkyle en $C_1$-$C_6$, un alcoxy en $C_1$-$C_4$, un amino, un aminométhyle ;
- $R_6$ et $R'_6$ sont semblables ou différents et représentent chacun indépendamment un atome d'hydrogène, un carboxy, un alcoxycarbonyle dans lequel l'alcoxy est en $C_1$-$C_4$, un cyano, un tétrazolyle -5, un méthyltétrazolyle-5 ou un 4H-oxo-5 oxadiazol-1,2,4 yle-3, ou un 4H-dioxo-3,5 oxadiazole-1, 2, 4 yle-2 à la condition que pour les groupes e, f, g, h, j, au moins un des substituants $R_6$ ou $R'_6$ soit différent de l'hydrogène ;
- $R_7$ représente un alkyle en $C_1$-$C_4$ ou un groupe choisi parmi :

i) $-(CH_2)_r-CO_2H$,

ii) 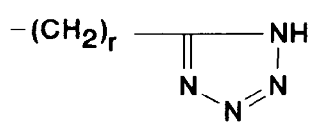 ,

iii) 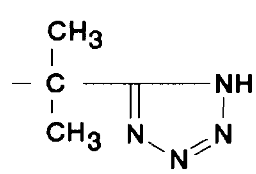 ,

iv) 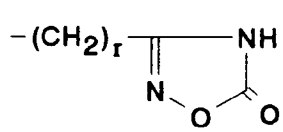 ,

v) 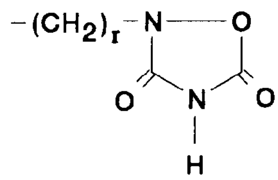

vi) 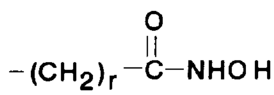

vii) $-(CH_2)_r-CN$

viii) $-OCOCH_3$

- R'$_7$ représente un alkyle en $C_1$-$C_4$ ou un groupe choisi parmi :

$-(CH_2)_s-CO_2H$ ,

$-(CH_2)_r-CN$ ,

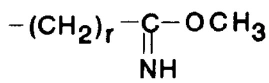

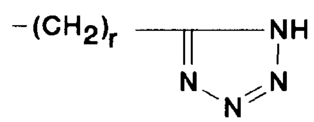 ,

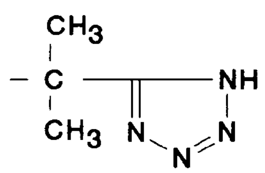 ,

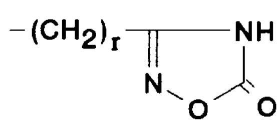

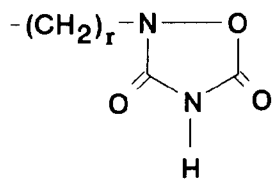

- $R_8$ est identique à $R_7$, ou bien $R_8$ représente l'hydrogène, un atome d'halogène, un alkyle en $C_1$-$C_4$, un cycloalkyle en $C_3$-$C_7$, un phényle substitué par R'$_6$, un benzyle dans lequel le phényle est substitué par R'$_6$, un hydroxyle, un alcoxy en $C_1$-$C_4$, un cyano, un groupe $OCOR_{15}$ dans lequel $R_{15}$ représente un alkyle en $C_1$-$C_4$, un halogénoalkyle en $C_1$-$C_4$ ou un phényle substitué par R'$_6$ ;
- $R_9$ est l'hydrogène ou $R_9$ est identique à $R_8$, avec la limitation que $R_9$ ne peut pas être identique à $R_7$ ;

- ou $R_8$ et $R_9$ ensemble avec l'atome de carbone auquel ils sont liés constituent un cycloalkyle en $C_3$-$C_7$ ou un groupe carbonyle ;
- $R_{10}$ représente l'hydrogène, un alkyle en $C_1$-$C_4$, un cycloalkyle en $C_3$-$C_7$, un phényle substitué par $R'_6$ ou un benzyle dont le phényle est substitué par $R'_6$ ;
- $R_{11}$ représente l'hydrogène, un alkyle en $C_1$-$C_6$, un phényle, un carboxy, un alcoxycarbonyle dans lequel l'alcoxy est en $C_1$-$C_4$, un tétrazolyle-5 ou un 4H-oxo-5 oxadiazol-1, 2, 4 yle-3 ou un 4H-dioxo-3,5 oxadiazole-1, 2, 4 yle-2 ;
- $R_{12}$ représente l'hydrogène, un alkyle en $C_1$-$C_6$, un cyano, un carboxy, un alcoxycarbonyle dans lequel l'alcoxy est en $C_1$-$C_4$, un tétrazolyle-5, un phényle substitué par $R'_6$ ou un benzyle dont le phényle est substitué par $R'_6$ ;
- $R_{13}$ représente l'hydrogène, un alkyle en $C_1$-$C_6$, un alcoxycarbonyle dans lequel l'alcoxy est en $C_1$-$C_4$, un phényle substitué par $R'_6$, un benzyle dont le phényle est substitué par $R'_6$, un cyano, un carboxy, un tétrazolyle-5, un 4H-oxo-5 oxadiazol-1, 2,4 yle-3, un 4H-dioxo-3,5-oxadiazole-1,2,4-yle-2, à la condition que $R_{11}$ et $R_{13}$ ne représentent pas simultanément l'un des groupes suivants : un carboxy, un tétrazolyle-5, un 4H-oxo-5 oxadiazol-1,2,4 yle-3 ou un 4H-dioxo-3,5 oxadiazole-1, 2, 4 yle-2 ; ou
- $R_{14}$ représente un carboxyalkyle en $C_1$-$C_4$, un phényle, un carboxyphényle ;
- p + q = m ;
- n est un nombre entier compris entre 2 et 11 ;
- m est un nombre entier compris entre 2 et 5 ;
- r représente 0, 1 ou 2 ;
- s représente 1 ou 2 ;
- X représente un atome d'oxygène ou atome de soufre ;
- z et t sont nuls ou l'un est nul et l'autre représente 1 ;

avec la limitation que pour les valeurs a), b), k) et l) de $R_4$, $R_6$ représente l'hydrogène lorsque $R_7$, $R'_7$ ou $R_{14}$ contient soit un groupement carboxy, soit un groupement tétrazolyle-5, soit un groupement 4H-oxo-5 oxadiazol-1,2,4 yle-3 soit un groupement 4H-dioxo-3,5 oxadiazole-1, 2, 4 yle-2 ;

et ses sels,

caractérisé en ce que :

a1) on fait réagir un aminoacide de formule :

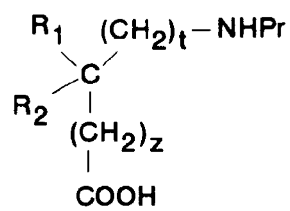

(7)

dans laquelle z, t, $R_1$ et $R_2$ ont les signification indiquées ci-dessus pour (I) et dont la fonction amine est protégée par le groupe Pr, sur un dérivé de formule :

$H_2N$-$R'_4$ (8)

dans laquelle $R'_4$ représente soit $R_4$ soit un groupe précurseur de $R_4$ ;

b1) après déprotection de l'amine, le composé ainsi obtenu de formule :

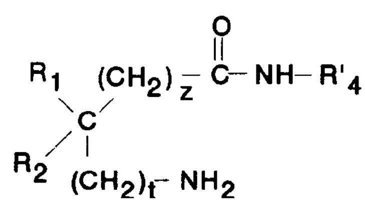

(9)

est traité par un ortho-ester d'alkyle de formule $R_3C(OR)_3$ (10) dans laquelle $R_3$ a la signification indiquée ci-dessus pour (I) et R est un alkyle en $C_1$-$C_4$ ;

c1) éventuellement, le composé ainsi obtenu de formule :

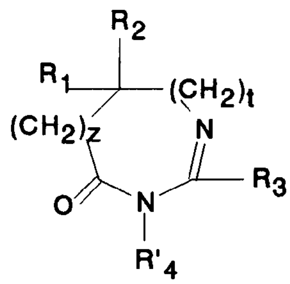

(4)

est traité par le réactif de Lawesson [bis(méthoxy-4 phényl)-2,4 dithia-1,3 diphosphétane-2,4 disulfure-2,4] ;
d1) le composé ainsi obtenu en b1) ou en c1) de formule :

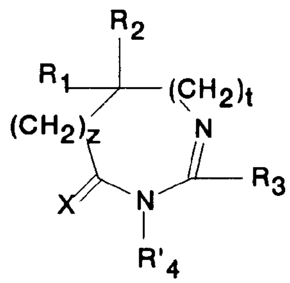

(5)

dans laquelle X représente un atome d'oxygène ou un atome de soufre, est ensuite éventuellement traité dans des conditions convenables pour préparer le composé (I) par transformation du groupe $R'_4$ en groupe $R_4$.
e1) Le composé obtenu en d1) est ensuite éventuellement transformé en l'un de ses sels.

**2.** Procédé pour la préparation d'un composé de formule (I) tel que défini dans la revendication 1, dans lequel $R_4$ a l'une des valeurs a), b), c), d), j), k) ou l), caractérisé en ce que :
a2) on fait réagir un dérivé hétérocyclique de formule :

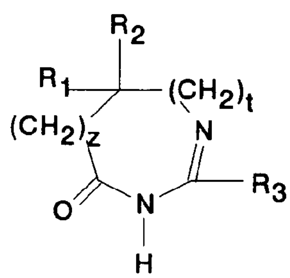

(2)

dans laquelle z, t, $R_1$ , $R_2$ , et $R_3$ ont les significations indiquées ci-dessus pour (I) dans la revendication 1, sur un halogénure de formule :

Hal-$R'_4$ (3)

dans laquelle Hal représente un atome d'halogène, de préférence le brome, et $R'_4$ représente soit $R_4$, soit un groupement précurseur de $R_4$ ;
b2) éventuellement, le composé ainsi obtenu, de formule :

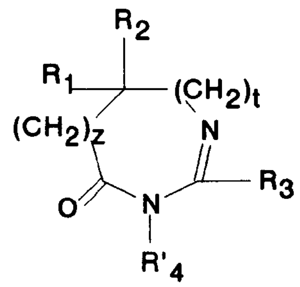

(4)

est traité par le réactif de Lawesson [bis(méthoxy-4 phényl)-2,4 dithia-1,3 diphosphétane-2,4 disulfure-2,4] ;

c2) le composé obtenu en a2) ou en b2), de formule :

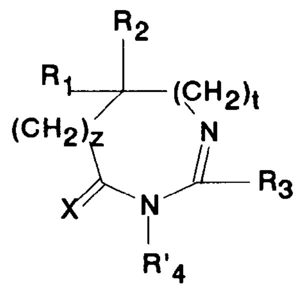

(5)

dans laquelle X représente un atome d'oxygène ou un atome de soufre, est éventuellement traité pour préparer le composé (I) par transformation du groupe $R'_4$ en $R_4$.

d2) Le composé ainsi obtenu en c2) est ensuite éventuellement transformé en l'un de ses sels.

**3.** Procédé pour la préparation d'un composé de formule (I) tel que défini dans la revendication 1, dans lequel $R_4$ représente le groupe g), caractérisé en ce que :

a2) on fait réagir un dérivé hétérocyclique de formule :

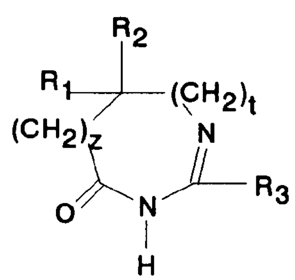

(2)

dans laquelle z, t, $R_1$ , $R_2$ , et $R_3$ ont les significations indiquées ci-dessus pour (I) dans la revendication 1, sur un composé de formule :

$R'_4OH$ (3')

dans laquelle $R'_4$ représente soit $R_4$, soit un groupement précurseur de $R_4$ ;

b2) éventuellement, le composé ainsi obtenu, de formule :

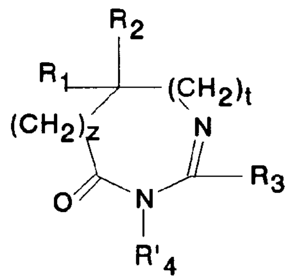

(4)

est traité par le réactif de Lawesson [bis(méthoxy-4 phényl)-2,4 dithia-1,3 diphosphétane-2,4 disulfure-2,4] ;

c2) le composé obtenu en a2) ou en b2), de formule :

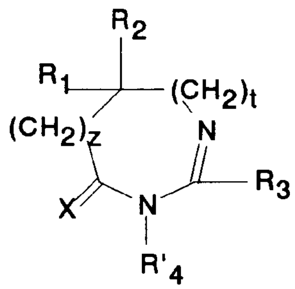

(5)

dans laquelle X représente un atome d'oxygène ou un atome de soufre, est éventuellement traité pour préparer le composé (I) par transformation du groupe $R'_4$ en $R_4$.

d2) Le composé ainsi obtenu en c2) est ensuite éventuellement transformé en l'un de ses sels.

**4.** Procédé selon l'une quelconque des revendications 1 à 3, caractérisé en ce que l'on utilise les composés (7) ou (2) dans lesquels $R_1$ et $R_2$ liés ensemble constituent avec le carbone auquel ils sont liés un cyclopentane ou un cyclohexane.

**5.** Procédé selon l'une quelconque des revendications 1 à 3, caractérisé en ce que l'on utilise les composés (7) ou (2) dans lesquels $R_1$ et $R_2$ représentent l'un un méthyle, l'autre un cyclohexyle.

**6.** Procédé selon l'une quelconque des revendications 1 à 3, caractérisé en ce que l'on utilise les composés (10) ou (2) dans lesquels $R_3$ représente un groupe alkyle linéaire en $C_1$-$C_6$.

**7.** Procédé selon l'une quelconque des revendications 1 à 3, caractérisé en ce qu'il comprend les étapes $a_1$), $b_1$), $d_1$) et $e_1$) ou les étapes $a_2$), $c_2$) et $d_2$), pour préparer un composé de formule (I) dans lequel X = O.

**8.** Procédé selon l'une quelconque des revendications 1 à 3, caractérisé en ce que l'on utilise les composés (8), (3) ou (3') dans lesquels $R'_4$ représente un groupement a) de formule :

a) 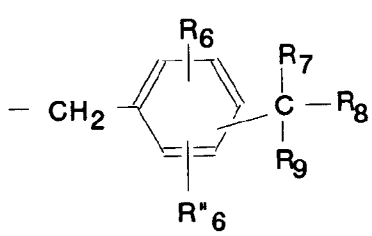 ,

dans lequel $R_6$, $R''_6$, $R_7$, $R_8$ et $R_9$ ont les significations indiquées pour (I) dans la revendication 1.

**9.** Procédé selon la revendication 8, caractérisé en ce que $R_6$ et $R''_6$ représentent l'hydrogène et $R_7$ représente un groupe $-(CH_2)_r-$ COOH avec r = 0, 1 ou 2.

**Revendications pour l'Etat contractant suivant : GR.**

1. Composé de formule :

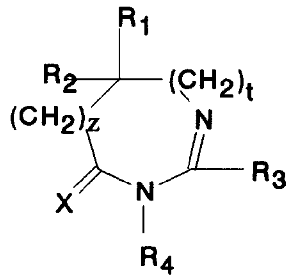

(I)

dans laquelle :

- $R_1$ et $R_2$ représentent chacun indépendamment un alkyle en $C_1$-$C_6$, un cycloalkyle en $C_3$-$C_7$ un phényle, un phénylalkyle dans lequel l'alkyle est en $C_1$-$C_3$, lesdits groupes alkyle, phényle et phénylalkyle étant non substitués ou substitués par un ou plusieurs atomes d'halogène ou par un groupe choisi parmi un perfluoroalkyle en $C_1$-$C_4$, un hydroxyle, un alcoxy en $C_1$-$C_4$ ;
- ou $R_1$ et $R_2$ ensemble forment un groupe de formule $=CR'_1R'_2$, dans laquelle $R'_1$ représente l'hydrogène, un alkyle en $C_1$-$C_4$ ou un phényle, et $R'_2$ représente un alkyle en $C_1$-$C_4$ ou un phényle;
- ou encore $R_1$ et $R_2$ liés ensemble représentent, soit un groupe de formule $-(CH_2)_n-$, soit un groupe de formule $-(CH_2)_pY(CH_2)_q-$, dans lequel Y est, soit un atome d'oxygène, soit un atome de soufre, soit un groupe CH substitué par un groupe alkyle en $C_1$-$C_4$ un phényle ou un phénylalkyle dans lequel l'alkyle est en $C_1$-$C_3$ soit un groupe $N$-$R_5$ dans lequel $R_5$ représente un hydrogène, un alkyle en $C_1$-$C_4$, un phénylalkyle dans lequel l'alkyle est en $C_1$-$C_3$, un alkylcarbonyle en $C_1$-$C_4$, un halogénoalkylcarbonyle en $C_1$-$C_4$, un polyhalogénalkylcarbonyle en $C_1$-$C_4$, un benzoyle, un alpha aminoacyle ou un groupe N-protecteur, ou $R_1$ et $R_2$ liés ensemble avec l'atome de carbone auquel ils sont liés constituent un indane ou un adamantane ;
- $R_3$ représente un hydrogène, un alkyle en $C_1$-$C_6$, non substitué ou substitué par un ou plusieurs atomes d'halogène ; un alcényle en $C_2$-$C_6$ ; un cycloalkyle en $C_3$-$C_7$ ; un phényle ; un phénylalkyle dans lequel l'alkyle est en $C_1$-$C_3$ ; un phénylalcényle dans lequel l'alcényle est en $C_2$-$C_3$, lesdits groupes phényles étant non substitués ou substitués une ou plusieurs fois par un atome d'halogène, un alkyle en $C_1$-$C_4$, un halogénoalkyle en $C_1$-$C_4$, un polyhalogénoalkyle en $C_1$-$C_4$, un hydroxyle ou un alcoxy en $C_1$-$C_4$ ;
- $R_4$ représente un groupement choisi parmi :

a) 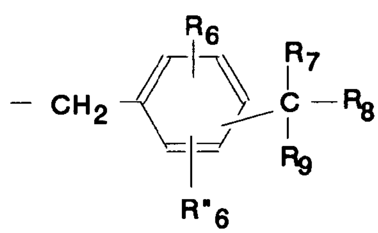 ,

b) 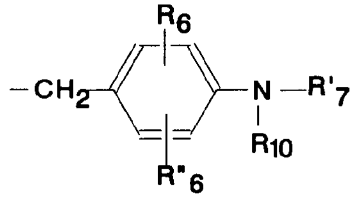

c) 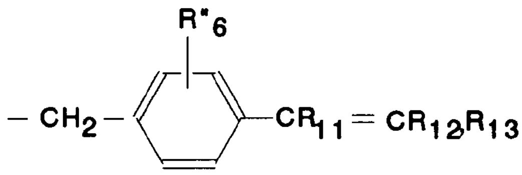 ,

d) 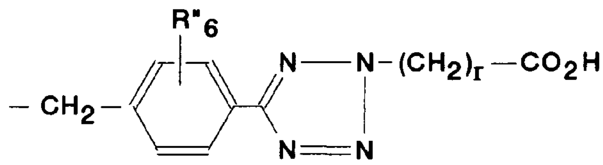 ,

e) 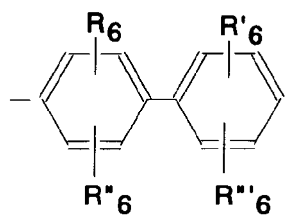 ,

f) 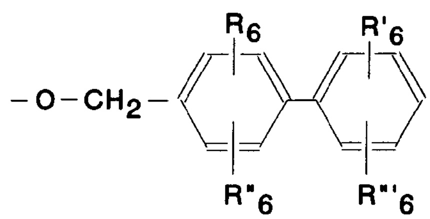 ,

g) 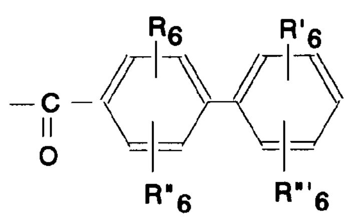 ,

h) 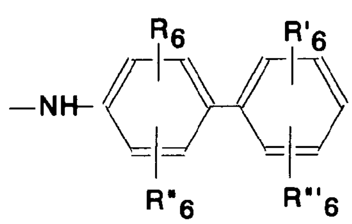 ,

j) 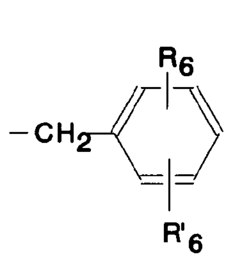

k) 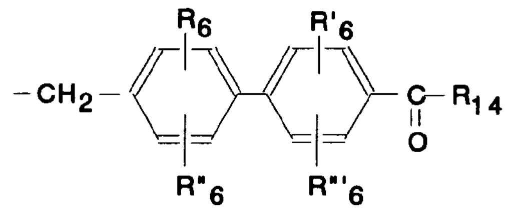

l) 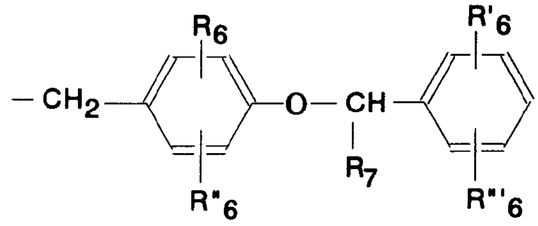 ,

- $R''_6$ et $R'''_6$ sont semblables ou différents et représentent chacun indépendamment l'hydrogène, un atome de chlore ou un groupe choisi parmi un alkyle en $C_1$-$C_6$, un alcoxy en $C_1$-$C_4$, un amino, un aminométhyle ;
- $R_6$ et $R'_6$ sont semblables ou différents et représentent chacun indépendamment un atome d'hydrogène, un carboxy, un alcoxycarbonyle dans lequel l'alcoxy est en $C_1$-$C_4$, un cyano, un tétrazolyle -5, un méthyltétrazolyle-5 ou un 4H-oxo-5 oxadiazol-1,2,4 yle-3, ou un 4H-dioxo-3,5 oxadiazol-1, 2, 4 yle-2 à la condition que pour les groupes e, f, g, h, j, au moins un des substituants $R_6$ ou $R'_6$ soit différent de l'hydrogène ;
- $R_7$ représente un alkyle en $C_1$-$C_4$ ou un groupe choisi parmi :

i) $-(CH_2)_r-CO_2H$

ii) 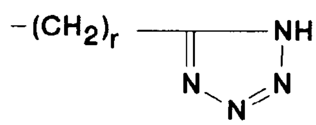 ,

iii) 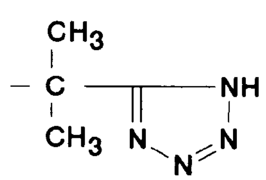 ,

iv) 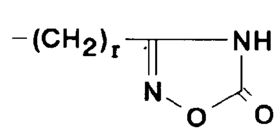 ,

v) 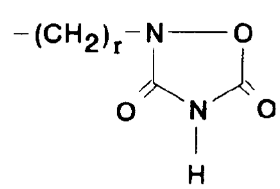

vi) 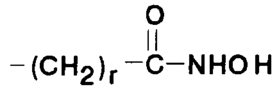

vii) $-(CH_2)_r-CN$

viii) $-OCOCH_3$

- $R'_7$ représente un alkyle en $C_1$-$C_4$ ou un groupe choisi parmi :

$-(CH_2)_s-CO_2H$ ,

$-(CH_2)_r-CN$ ,

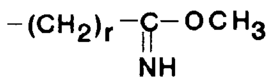

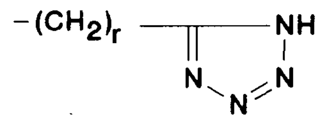
,

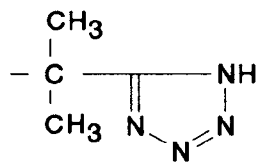
,

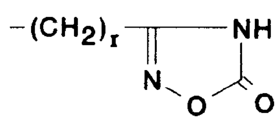

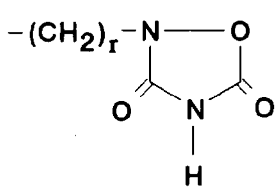

- $R_8$ est identique à $R_7$, ou bien $R_8$ représente l'hydrogène, un atome d'halogène, un alkyle en $C_1$-$C_4$, un cycloalkyle en $C_3$-$C_7$, un phényle substitué par $R'_6$, un benzyle dans lequel le phényle est substitué par $R'_6$, un hydroxyle, un alcoxy en $C_1$-$C_4$, un cyano, un groupe $OCOR_{15}$ dans lequel $R_{15}$ représente un alkyle en $C_1$-$C_4$, un halogénoalkyle en $C_1$-$C_4$ ou un phényle substitué par $R'_6$ ;
- $R_9$ est l'hydrogène ou $R_9$ est identique à $R_8$, avec la limitation que $R_9$ ne peut pas être identique à $R_7$ ;
- ou $R_8$ et $R_9$ ensemble avec l'atome de carbone auquel ils sont liés constituent un cycloalkyle en $C_3$-

$C_7$ ou un groupe carbonyle ;
- $R_{10}$ représente l'hydrogène, un alkyle en $C_1$-$C_4$, un cycloalkyle en $C_3$-$C_7$, un phényle substitué par $R'_6$ ou un benzyle dont le phényle est substitué par $R'_6$ ;
- $R_{11}$ représente l'hydrogène, un alkyle en $C_1$-$C_6$, un phényle, un carboxy, un alcoxycarbonyle dans lequel l'alcoxy est en $C_1$-$C_4$, un tétrazolyle-5 ou un 4H-oxo-5 oxadiazol-1, 2, 4 yle-3 ou un 4H-dioxo-3,5 oxadiazole-1, 2, 4 yle-2 ;
- $R_{12}$ représente l'hydrogène, un alkyle en $C_1$-$C_6$, un cyano, un carboxy, un alcoxycarbonyle dans lequel l'alcoxy est en $C_1$-$C_4$, un tétrazolyle-5, un phényle substitué par $R'_6$ ou un benzyle dont le phényle est substitué par $R'_6$ ;
- $R_{13}$ représente l'hydrogène, un alkyle en $C_1$-$C_6$, un alcoxycarbonyle dans lequel l'alcoxy est en $C_1$-$C_4$, un phényle substitué par $R'_6$, un benzyle dont le phényle est substitué par $R'_6$, un cyano, un carboxy, un tétrazolyle-5, un 4H-oxo-5 oxadiazol-1, 2,4 yle-3, un 4H-dioxo-3,5-oxadiazole-1,2,4-yle-2, à la condition que $R_{11}$ et $R_{13}$ ne représentent pas simultanément l'un des groupes suivants : un carboxy, un tétrazolyle-5, un 4H-oxo-5 oxadiazol-1,2,4 yle-3 ou un 4H-dioxo-3,5 oxadiazole-1, 2, 4 yle-2 ; ou
- $R_{14}$ représente un carboxyalkyle en $C_1$-$C_4$, un phényle, un carboxyphényle ;
- $p + q = m$ ;
- n est un nombre entier compris entre 2 et 11 ;
- m est un nombre entier compris entre 2 et 5 ;
- r représente 0, 1 ou 2 ;
- s représente 1 ou 2 ;
- X représente un atome d'oxygène ou atome de soufre ;
- z et t sont nuls ou l'un est nul et l'autre représente 1 ;
avec la limitation que pour les valeurs a), b), k) et l) de $R_4$, $R_6$ représente l'hydrogène lorsque $R_7$, $R'_7$ ou $R_{14}$ contient soit un groupement carboxy, soit un groupement tétrazolyle-5, soit un groupement 4H-oxo-5 oxadiazol-1,2,4 yle-3 soit un groupement 4H-dioxo-3,5 oxadiazole-1, 2, 4 yle-2 ;
et ses sels.

2. Composé selon la revendication 1 dans lequel $R_1$ et $R_2$ liés ensemble constituent avec le carbone auquel ils sont liés un cyclopentane ou un cyclohexane.

3. Composé selon la revendication 1 dans lequel $R_1$ et $R_2$ représentent l'un un méthyle, l'autre un cyclohexyle.

4. Composé selon l'une quelconque des revendications 1 à 3 dans lequel $R_3$ représente un groupe alkyle linéaire en $C_1$-$C_6$.

5. Composé selon l'une quelconque des revendications 1 à 4 dans lequel X représente un atome d'oxygène.

6. Composé selon l'une quelconque des revendications 1 à 5 dans lequel $R_4$ représente un groupement a) de formule :

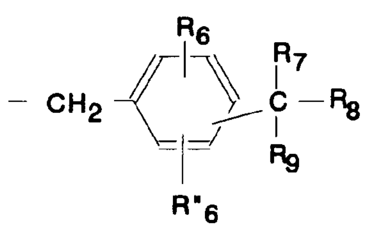

dans lequel $R_6$, $R''_6$, $R_7$, $R_6$ et $R_9$ sont tels que définis dans la revendication 1.

7. Composé selon la revendication 6 dans lequel $R_6$ et $R''_6$ représentent l'hydrogène et $R_7$ représente un groupe $-(CH_2)_r$-COOH avec r=0,1 ou 2.

8. Procédé pour la préparation d'un composé de formule (I) selon la revendication 1, dans lequel $R_4$ à l'une des valeurs a), b), c), d), j), k) ou l), caractérisé en ce que :
a1) on fait réagir un dérivé hétérocyclique de formule :

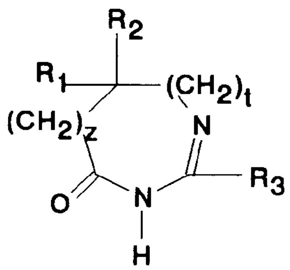

(2)

dans laquelle z, t, $R_1$ , $R_2$ , et $R_3$ ont les significations indiquées ci-dessus pour (I) dans la revendication 1, sur un halogènure de formule :

Hal-$R'_4$ (3)

dans laquelle Hal représente un atome d'halogène, de préférence le brome, et $R'_4$ représente soit $R_4$, soit un groupement précurseur de $R_4$ ;

b1) éventuellement, le composé ainsi obtenu, de formule :

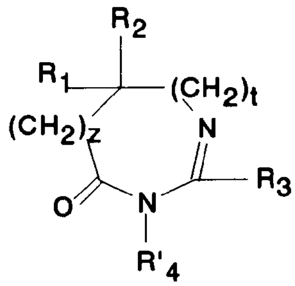

(4)

est traité par le réactif de Lawesson [bis(méthoxy-4 phényl)-2,4 dithia-1,3 diphosphétane-2,4 disulfure-2,4] ;

c1) le composé obtenu en a1) ou en b1), de formule :

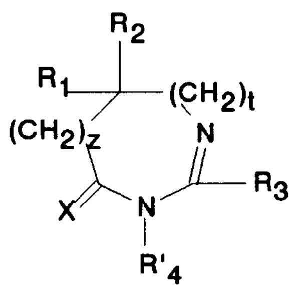

(5)

dans laquelle X représente un atome d'oxygène ou un atome de soufre, est éventuellement traité pour préparer le composé (I) par transformation du groupe $R'_4$ en $R_4$ ;

d1) le composé ainsi obtenu est ensuite éventuellement transformé en l'un de ses sels.

**9.** Procédé pour la préparation d'un composé selon la revendication 1, caractérisé en ce que

a2) on fait réagir un aminoacide de formule :

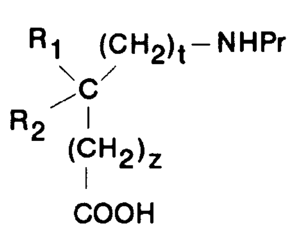

(7)

dans laquelle z, t, $R_1$ et $R_2$ ont les significations indiquées ci-dessus pour (I) dans la revendication 1 et dont la fonction amine est protégée par le groupe Pr, sur un dérivé de formule :

$H_2N\text{-}R'_4$ (8)

dans laquelle $R'_4$ représente soit $R_4$ soit un groupe précurseur de $R_4$ ;

b2) après déprotection de l'amine, le composé ainsi obtenu de formule :

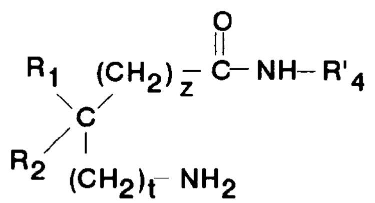

(9)

est traité par un ortho-ester d'alkyle de formule $R_3C(OR)_3$ (10) dans laquelle $R_3$ a la signification indiquée ci-dessus pour (I) dans la revendication 1 et R est un alkyle en $C_1$-$C_4$ ;

c2) éventuellement, le composé ainsi obtenu de formule :

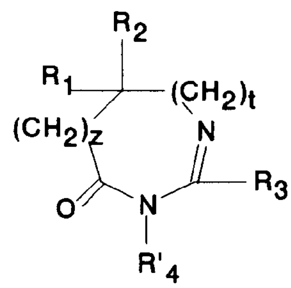

(4)

est traité par le réactif de Lawesson [bis(méthoxy-4 phényl)-2,4 dithia-1,3 diphosphétane-2,4 disulfure-2,4] ;

d2) le composé ainsi obtenu en b2) ou en c2) de formule :

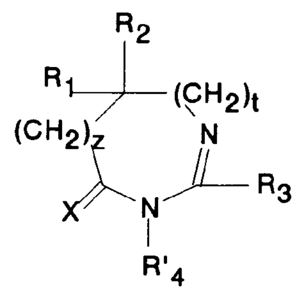

(5)

dans laquelle X représente un atome d'oxygène ou un atome de soufre, est ensuite éventuellement traité dans des conditions convenables pour préparer le composé (I) par transformation du groupe $R'_4$ en groupe $R_4$ ;

e2) le composé ainsi obtenu est ensuite éventuellement transformé en l'un de ses sels.

**10.** Procédé pour la préparation d'un composé de formule (I) selon la revendication 1, dans lequel $R_4$ représente le groupe g), caractérisé en ce que :

a1) on fait réagir un dérivé hétérocyclique de formule :

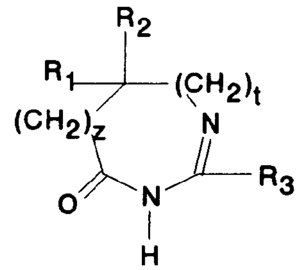

(2)

dans laquelle z, t, $R_1$ , $R_2$ , et $R_3$ ont les significations indiquées ci-dessus pour (I) dans la revendication 1, sur un composé de formule :

$R'_4OH$ (3')

dans laquelle $R'_4$ représente soit $R_4$, soit un groupement précurseur de $R_4$ ;
b1) éventuellement, le composé ainsi obtenu, de formule :

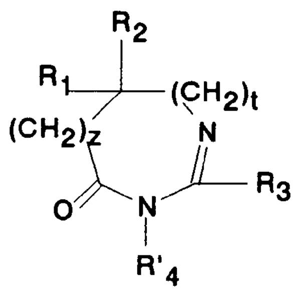

(4)

est traité par le réactif de Lawesson [bis(méthoxy-4 phényl)-2,4 dithia-1,3 diphosphétane-2,4 disulfure-2,4] ;
c1) le composé obtenu en a1) ou en b1), de formule :

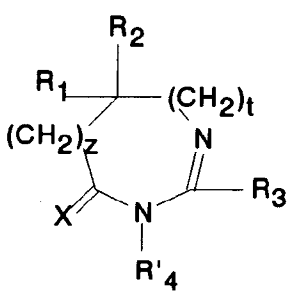

(5)

dans laquelle X représente un atome d'oxygène ou un atome de soufre, est éventuellement traité pour préparer le composé (I) par transformation du groupe $R'_4$ en $R_4$ ;
d1) le composé ainsi obtenu est ensuite éventuellement transformé en l'un de ses sels.

**11.** Procédé pour la préparation d'une composition pharmaceutique, caractérisé en ce qu'il consiste à mélanger un composé selon l'une quelconque des revendications 1 à 7 avec un véhicule pharmaceutiquement acceptable.

**12.** Procédé pour la préparation d'une composition pharmaceutique, caractérisé en ce qu'il consiste à associer un composé selon l'une quelconque des revendications 1 à 7 avec un composé bêtabloquant.

**13.** Procédé pour la préparation d'une composition pharmaceutique, caractérisé en ce qu'il consiste à associer un composé selon l'une quelconque des revendications 1 à 7 avec un diurétique.

**14.** Procédé pour la préparation d'une composition pharmaceutique, caractérisé en ce qu'il consiste à associer un composé selon l'une quelconque des revendications 1 à 7 avec un anti-inflammatoire non stéroïdien.

**15.** Procédé pour la préparation d'une composition pharmaceutique, caractérisé en ce qu'il consiste à associer un composé selon l'une quelconque des revendications 1 à 7 avec un antagoniste calcique.

**16.** Procédé pour la préparation d'une composition pharmaceutique, caractérisé en ce qu'il consiste à associer un composé selon l'une quelconque des revendications 1 à 7 avec un tranquilisant.

Office européen des brevets

# RAPPORT PARTIEL DE RECHERCHE EUROPEENNE

qui selon la règle 45 de la Convention sur le brevet européen est consideré, aux fins de la procédure ultérieure comme le rapport de la recherche européenne

Numero de la demande

EP 92 40 1715

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 880 804 (E.I. DU PONT DE NEMOURS AND CO.)<br>--- | | C 07 D 235/02<br>C 07 D 233/70<br>C 07 D 403/12<br>A 61 K 31/415<br>C 07 D 403/10<br>C 07 D 413/12 |
| A | EP-A-0 380 959 (E.I. DU PONT DE NEMOURS AND CO.)<br>--- | | |
| A | EP-A-0 144 749 (HOECHST AKTIENGESELLSCHAFT)<br>--- | | |
| A | EP-A-0 144 748 (HOECHST AKTIENGESELLSCHAFT)<br>--- | | |
| A | EP-A-0 226 947 (DELAMERCK GmbH & CO. KG)<br>--- | | |
| A | EP-A-0 253 310 (E.I. DU PONT DE NEMOURS AND CO.)<br>--- | | |
| A | EP-A-0 291 969 (E.I. DU PONT DE NEMOURS AND CO.)<br>--- -/- | | DOMAINES TECHNIQUES RECHERCHES (Int. Cl.5)<br>C 07 D<br>A 61 K |

## RECHERCHE INCOMPLETE

La division de la recherche estime que la présente demande de brevet européen n'est pas conforme aux dispositions de la Convention sur le brevet européen au point qu'une recherche significative sur l'état de la technique ne peut être effectuée au regard d'une partie des revendications.

Revendications ayant fait l'objet de recherches complètes:

Revendications ayant fait l'objet de recherches incomplètes: 1-16

Revendications n'ayant pas fait l'objet de recherches:

Raison pour la limitation de la recherche:

Voir annex -C-

| Lieu de la recherche | Date d'achèvement de la recherche | Examinateur |
|---|---|---|
| LA HAYE | 30-07-1992 | DE BUYSER I.A.F. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0406)

Page 2

Office européen des brevets

# RAPPORT PARTIEL DE RECHERCHE EUROPEENNE

Numero de la demande
EP 92 40 1715

| DOCUMENTS CONSIDERES COMME PERTINENTS | | | CLASSEMENT DE LA DEMANDE (Int. Cl.5) |
|---|---|---|---|
| Catégorie | Citation du document avec indication, en cas de besoin, des parties pertinentes | Revendication concernée | |
| D,A | EP-A-0 324 377 (E.I. DU PONT DE NEMOURS AND CO.)<br>----- | | |
| | | | DOMAINES TECHNIQUES RECHERCHES (Int. Cl.5) |

EPO FORM 1503 03.82 (P0411)

EP 92 40 1715 -C-

La rédaction des revendications n'est pas claire ni concise (Art.83-84 OEB), et représente une telle masse énorme de produits, qu'une recherche complète n'est pas possible pour des raisons d'économie (voir Directives relatives à l'examen pratiqué à l'OEB, Partie B, Chapitre III,2). De cette façon la recherche a été fondé sur le concept inventif de la demande telle qu'exemplifié par les composés, qui sont bien caractérisés par des éléments physiques ou chimiques, c.a.d. les composés des exemples.